# EUROPEAN PATENT APPLICATION

(11) **EP 4 778 937 A1**
(43) Date of publication of application: **22.07.2026**
(21) Application number: 24884963.0
(22) Date of filing: 01.11.2024
(51) Int. Cl.: C07K 14/705, C12N 15/62, C12N 5/10, C12N 15/85, A61K 35/17, A61K 39/00

(54) **SPECIFIC TIGIT PEPTIDE FRAGMENT**

(30) Priority: 02.11.2023 CN 202311451861; 02.11.2023 CN 202311450946
(71) Applicant: Chongqing Precision Biotech Co., Ltd., Chongqing 400038 (CN); Chongqing Precision Biological Industrial Technology Research Institute Co., Ltd., Chongqing 400039 (CN)
(72) Inventor: DAI, Depeng, Chongqing 400038 (CN); HONG, Juan, Chongqing 400038 (CN); ZHAO, Yongchun, Chongqing 400038 (CN); ZHAO, Wenxu, Chongqing 400038 (CN); SHEN, Junjie, Chongqing 400038 (CN); XU, Yanmin, Chongqing 400038 (CN); QI, Yanan, Chongqing 400038 (CN); HUANG, Xia, Chongqing 400038 (CN); QIN, Lei, Chongqing 400038 (CN); CHEN, Jun, Chongqing 400038 (CN)
(74) Representative: Fabry, Bernd
(86) International application number: PCT/CN2024/129324
(87) International publication number: WO 2025/092966

(57) **Abstract**

Provided are a TIGIT-derived polypeptide or a mutant thereof, and a fusion protein, engineered receptor or engineered cell containing the polypeptide or the mutant, wherein the binding capacity of the TIGIT-derived polypeptide or mutant thereof to CD112 is reduced.

## Description

### TECHNICAL FIELD

The present application relates to the field of biomedicine, in particular to the modification and application of TIGIT extracellular region polypeptides.

### BACKGROUND ART

CAR-T is currently one of the most promising immunotherapeutic methods, and has achieved remarkable success particularly against B-ALL and lymphoma. However, its application in solid tumors remains less effective, mainly attributed to antigen heterogeneity of solid tumors, immunosuppressive microenvironment, etc.

CD155 is the fifth member of the Nectin-like molecule family and functions as a poliovirus receptor. Therefore, CD155 is also known as necl-5 or PVR (poliovirus receptor). As an immunoglobulin-like adhesion molecule, CD155 participates in cell motility and natural killer cell- and T cell-mediated immunity. It is barely expressed or weakly expressed in almost all normal human tissues, but is usually overexpressed in human malignant tumors. Overexpression of CD155 promotes tumor cell invasion and migration, and is associated with tumor progression and poor prognosis, thus making it an advantageous target for cell therapy of solid tumors, such as CAR-T and CAR-NK.

CD155 is a ligand for the costimulatory receptor CD226 and the co-inhibitory receptors TIGIT and CD96 on natural killer cells and T cells. Common design approaches in the art involve selecting the ScFv fragment of an anti-CD155 antibody as an extracellular antigen binding domain, or selecting a receptor of CD155, such as TIGIT or CD96, as an extracellular antigen binding domain of a CAR structure. TIGIT, namely T cell immunoglobulin and ITIM domain protein (T cell immunoreceptor with Ig and ITIM domains), is a co-inhibitory receptor on T cells and NK cells, and is mainly expressed on activated T cells, NK cells, Treg cells, and helper T cells. In addition to CD155, ligands for TIGIT also include CD112. CD112, also known as nectin-2 or PVR-related protein 2 (PVRL2), is a member of the Nectin family and is primarily localized at the adherens junctions of epithelial cells. Although CD112 is also expressed in tumors, it is widely expressed in various cells, including epithelial cells, endothelial cells, neurons, and fibroblasts, posing safety risks due to off-target effects when used in the CAR structure design. Therefore, to enable the application of TIGIT in the field of cell therapy, it is necessary to improve the antigen specificity of TIGIT.

### SUMMARY

The present application relates to a TIGIT extracellular region polypeptide derived from an extracellular portion of a TIGIT protein, whose binding ability to CD155 is significantly higher than that to CD112, and the difference between its CD155-binding ability and CD112-binding ability is significantly greater than that of an extracellular portion of a wild-type TIGIT protein (whose amino acid sequence is as set forth in SEQ ID NO:1). The present application further relates to a fusion protein and an engineered receptor comprising the TIGIT extracellular region polypeptide, an engineered cell comprising the engineered receptor, and use of the engineered TIGIT extracellular region polypeptide, fusion protein, engineered receptor or engineered cell.

Specifically, the present application relates to:
1. A TIGIT extracellular region polypeptide, comprising a mutation at position 48 relative to a reference sequence, wherein the reference sequence is an amino acid sequence as set forth in SEQ ID NO:1, and wherein amino acid position numbering is based on the reference sequence.
2. The TIGIT extracellular region polypeptide according to item 1, comprising at least amino acids corresponding to positions 33 to 93 of the reference sequence. In some embodiments, the polypeptide comprises deletion of one or more amino acids at amino acid positions corresponding to positions 33 to 93 of the reference sequence. In some embodiments, the polypeptide comprises one or more additional amino acids at any two amino acid positions among the amino acid positions corresponding to positions 33 to 93 of the reference sequence. In some embodiments, the polypeptide does not comprise other amino acids at C-terminus and/or N-terminus side of the amino acids corresponding to positions 33 to 93 of the reference sequence, for example, amino acids corresponding to positions numbered less than 33 of the reference sequence and/or amino acids corresponding to positions numbered greater than 93 of the reference sequence. In some embodiments, the polypeptide further comprises other amino acids at the C-terminus and/or N-terminus side of the amino acids corresponding to positions 33 to 93 of the reference sequence, for example, amino acids corresponding to positions numbered less than 33 of the reference sequence and/or amino acids corresponding to positions numbered greater than 93 of the reference sequence.
3. The TIGIT extracellular region polypeptide according to item 1 or 2, wherein the mutation is any one selected from the group consisting of:
   C48G, C48A, C48V, C48L, C48I, C48P, C48F, C48W, C48M, C48Y, C48S, C48T, C48N, C48Q, C48D, C48E, C48K, C48R, C48H, and deletion at position C48.
4. The TIGIT extracellular region polypeptide according to any one of items 1 to 3, further comprising one or more amino acid positions selected from the group consisting of:
   2M, 2W, 2T, 9T, 9S, 12I, 12N, 14A, 14V, 20I, 20T, 21I, 21F, 22L, 22F, 34T, 34S, 37N, 37D, 37E, 39E, 39G, 39K, 39V, 42D, 42G, 44L, 44F, 61K, 61R, 70L, 70Q, 70P, 71G, 71D, 76S, 76P, 79V, 79E, 80N, 80Y, 86F, 86S, 86L, 101I, 101T, 102S, 102F, 106L, 106Q, 110V, 110Y, 110E, 110A, 113H, and 113Y.
5. The TIGIT extracellular region polypeptide according to item 4, further comprising a combination of amino acid positions of any one of the following 1) to 12):
   1) 22F, 37D, 39G;
   2) 37D;
   3) 20T, 37D, 39K, 44F;
   4) 22L, 37E, 71D, 102S;
   5) 20T, 37D, 42G, 70Q;
   6) 21F, 34S, 37E, 39K, 70P, 80Y, 101T, 110A;
   7) 9S, 37D, 39V, 61R, 101T;
   8) 20T, 37D, 39K;
   9) 37D, 86S, 113Y;
   10) 2W, 37D, 56F;
   11) 12N, 14V, 21F, 37D, 39K, 70Q; or
   12) 2T, 37D, 39K, 86L.
6. The TIGIT extracellular region polypeptide according to item 1, comprising any amino acid sequence selected from the following, or comprising a conservative substitution variant of any amino acid sequence selected from the following, or in some embodiments, the polypeptide comprising an amino acid sequence having at least 80% (for example, 81%, 82%, 83%, 84%, 85%, 90%, 91%, 92%, 93%, 94%, 95%, 96%, 97%, 98%, 98.5%, 99%, 99.5%) identity to any amino acid sequence selected from the following:
   SEQ ID NOs:5-17, 19, and 22, and SEQ ID NOs:24-26.
7. A fusion protein comprising the TIGIT extracellular region polypeptide according to any one of items 1 to 6.
8. The fusion protein according to item 7, further comprising optionally one or more polypeptides that bind to a tumor antigen and/or an immune checkpoint protein, wherein optionally, the polypeptide that binds to the tumor antigen and/or the immune checkpoint protein is an antibody, ligand or receptor of the tumor antigen and/or the immune checkpoint protein, and optionally the antibody against the tumor antigen and/or the immune checkpoint protein is a single-chain antibody (scFv), Fab, F(ab')2, Fab', Fv, Fd, dAb or diabody.
9. An engineered receptor, comprising an antigen binding domain, wherein the antigen binding domain comprises the TIGIT extracellular region polypeptide according to any one of items 1 to 6, or the fusion protein according to item 7 or 8.
10. The engineered receptor according to item 9, further comprising a signal transduction domain, wherein the signal transduction domain comprises a primary signal transduction domain and/or a costimulatory domain.
11. The engineered receptor according to item 9 or 10, which is a chimeric antigen receptor (CAR), a T cell receptor (TCR), a T cell antigen coupler (TAC) or a fusion protein.
12. The engineered receptor according to any one of items 9 to 11, wherein the costimulatory domain comprises a signal transduction domain of one or more molecules selected from the following:
   CD27, CD28, 4-1BB, OX40, CD30, CD40, CD2, LFA-1, LIGHT, NKG2C, B7-H3, PD-1, ICOS, CDS, ICAM-1, GITR, BAFFR, LIGHTR, SLAMF7, CD7, NKp80 (KLRF1), CD160, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CDI11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1, SLAMF4, CD84, CD96, CEACAM1, CRTAM, CD229, CD160, PSGL1, CD100, CD69, SLAMF6, SLAMF1, SLAMF8, CD162, LTBR, LAT, GADS, SLP-76, PAG/Cbp, NKp44, NKp30, NKp46, NKG2D, and a ligand that specifically binds to CD83.
13. The engineered receptor according to any one of items 9 to 12, wherein the primary signal transduction domain comprises a signal transduction domain of one or more molecules selected from the following: CD3ζ, CD3γ, CD3δ, CD3ε, CD5, CD22, FcRγ, FcRβ, FcεRIγ, FcεRIβ, FcγRIIa, CD79α, CD79β, CD66d, DAP10, and DAP12.
14. The engineered receptor according to item 13, further comprising a transmembrane domain between the antigen binding domain and the signal transduction domain, wherein the transmembrane domain comprises a transmembrane domain of any one or more molecules selected from the following: ICOS, CD4, CD8α, CD28, CD3ζ, and TIGIT.
15. The engineered receptor according to item 14, wherein the antigen binding domain and the transmembrane domain are linked via a hinge region, preferably the hinge region is a hinge region of TIGIT, CD7, IgG, IgD, CD8α or CD28, or a combination thereof.
16. The engineered receptor according to any one of items 9 to 15, from N-terminus to C-terminus, comprising or consisting of in sequence: the TIGIT extracellular region polypeptide, a transmembrane domain, and a costimulatory domain, wherein a hinge region is further comprised or not comprised between the TIGIT extracellular region polypeptide and the transmembrane domain, wherein
   the costimulatory domain comprises or is a signal transduction domain of one or more molecules selected from the following:
   CD27, CD28, 4-1BB, OX40, CD30, CD40, CD2, LFA-1, LIGHT, NKG2C, B7-H3, PD-1, ICOS, CDS, ICAM-1, GITR, BAFFR, LIGHTR, SLAMF7, CD7, NKp80 (KLRF1), CD160, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CDI11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1, SLAMF4, CD84, CD96, CEACAM1, CRTAM, CD229, CD160, PSGL1, CD100, CD69, SLAMF6, SLAMF1, SLAMF8, CD162, LTBR, LAT, GADS, SLP-76, PAG/Cbp, NKp44, NKp30, NKp46, NKG2D, and a ligand that specifically binds to CD83;
   the transmembrane domain comprises or is a transmembrane domain of any one or more molecules selected from the following: ICOS, CD4, CD8α, CD28, CD3ζ, and TIGIT; and
   the hinge region comprises or is a hinge region of TIGIT, CD7, IgG, IgD, CD8α or CD28, or a combination thereof.
   In some embodiments, the engineered receptor comprises, from N-terminus to C-terminus: the TIGIT extracellular region polypeptide, a transmembrane domain, and a costimulatory domain; or the engineered receptor comprises, from N-terminus to C-terminus: the TIGIT extracellular region polypeptide, a hinge region, a transmembrane domain, and a costimulatory domain.
17. The engineered receptor according to item 16, from N-terminus to C-terminus, comprising or consisting of in sequence: the TIGIT extracellular region polypeptide, a CD28 transmembrane domain, and a CD28 signal transduction domain.
18. The engineered receptor according to any one of items 9 to 15, from N-terminus to C-terminus, comprising or consisting of in sequence any one of the following 1) to 10):
   1) the TIGIT extracellular region polypeptide, a CD28 transmembrane domain, a CD28 signal transduction domain, and a CD3ζ signal transduction domain;
   2) the TIGIT extracellular region polypeptide, a CD8 hinge region, a CD28 transmembrane domain, a CD28 signal transduction domain, and a CD3ζ signal transduction domain;
   3) the TIGIT extracellular region polypeptide, a CD8 hinge region, a CD28 transmembrane domain, a 4-1BB signal transduction domain, and a CD3ζ signal transduction domain;
   4) the TIGIT extracellular region polypeptide, a G4h hinge region, a CD28 transmembrane domain, a CD28 signal transduction domain, and a CD3ζ signal transduction domain;
   5) the TIGIT extracellular region polypeptide, an ICOS transmembrane domain, an ICOS signal transduction domain, and a CD3ζ signal transduction domain;
   6) the TIGIT extracellular region polypeptide, a CD8 transmembrane domain, a CD134 signal transduction domain, and a CD3ζ signal transduction domain;
   7) the TIGIT extracellular region polypeptide, a CD28 transmembrane domain, a CD28 signal transduction domain, a 4-1BB signal transduction domain, and a CD3ζ signal transduction domain;
   8) the TIGIT extracellular region polypeptide, a CD7 hinge region, a CD28 transmembrane domain, a CD28 signal transduction domain, and a CD3ζ signal transduction domain;
   9) the TIGIT extracellular region polypeptide, a G4h hinge region, a CD28 transmembrane domain, a CD28 signal transduction domain, a 4-1BB signal transduction domain, and a CD3ζ signal transduction domain; and
   10) the TIGIT extracellular region polypeptide, a CD8 hinge region, a CD8 transmembrane domain, a 4-1BB signal transduction domain, and a CD3ζ signal transduction domain.
19. An engineered nucleic acid molecule, encoding the TIGIT extracellular region polypeptide according to any one of items 1 to 6, the fusion protein according to item 7 or 8, or the engineered receptor according to any one of items 9 to 18. In some embodiments, the engineered nucleic acid molecule is DNA, RNA (e.g., mRNA), or a DNA-RNA hybrid molecule.
   In some embodiments, the engineered nucleic acid molecule is chemically modified. For example, in some embodiments, one or more thymidines of the engineered nucleic acid molecule are replaced with uridine. In some embodiments, one or more uridines of the engineered nucleic acid molecule are replaced with thymidine. In some embodiments, one or more guanosines of the engineered nucleic acid molecule are replaced with inosine. In some embodiments, the chemical modification is such that one or more nucleotides of the nucleic acid molecule are replaced with their corresponding nucleotide derivatives. For example, in some embodiments, one or more uridines of the engineered nucleic acid molecule are each replaced with one or more selected from the group consisting of: 5-(carboxyhydroxymethyl)uridine (chm5u), 5-carboxymethylaminomethyluridine (cmnm5u), 5-carboxymethylaminomethyl-2-thiouridine (cmnm5s2u), dihydrouridine (dhu), 2'-O-methylpseudouridine (fm), 1-methylpseudouridine (mlf), 3-(3-amino-3-carboxy-propyl)uridine ((acp3)u), uridine-5-oxyacetic acid (o5u), uridine-5-oxyacetic acid methyl ester (mv), 5-methoxycarbonylmethyluridine (mcm5u), 5-methoxycarbonylmethyl-2-thiouridine (mcm5s2u), 5-methoxyuridine (mo5u), 5-methyl-2-thiouridine (s2t), 2-thiouridine (s2u), 4-thiouridine (s4u), 5-methyluridine (m5u), 2'-O-methyl-5-methyluridine (tm), 2'-O-methyluridine (um), 5-methylaminomethyluridine (mam5u), 5-methylaminomethyl-2-thiouridine (mam5s2u), pseudouridine (p), and 5-methoxycarbonylmethyl-2-thioguanosine (mcm5s2u). In some embodiments, one or more guanosines of the engineered nucleic acid molecule are each replaced with one or more selected from the group consisting of: wybutoxosine (osyw), wybutosine (yw), 1-methylinosine (mli), 2'-O-methylguanosine (gm), 1-methylguanosine (m1g), 2,2-dimethylguanosine (m22g), 2-methylguanosine (m2g), 7-methylguanosine (m7g), β,D-galactosylqueuosine (gal q), queuosine (q), and β,D-mannosylqueuosine (man q). In some embodiments, one or more adenosines of the engineered nucleic acid molecule are each replaced with one or more selected from the group consisting of: N6-isopentenyladenosine (i6a), 1-methyladenosine (m1a), 2-methyladenosine (m2a), N6-methyladenosine (m6a), 2-methylthio-N6-isopentenyladenosine (ms2i6a), N-((9-β-D-ribofuranosyl-2-methylthiopurine-6-yl)carbamoyl)threonine (ms2t6a), N-((9-β-D-ribofuranosylpurin-6-yl)N-methylcarbamoyl)threonine (mt6a), N-((9-β-D-ribofuranosylpurin-6-yl)-carbamoyl)threonine (t6a), queuosine (q), and D-mannosylqueuosine (man q). In some embodiments, one or more cytidines of the engineered nucleic acid molecule are each replaced with one or more selected from the group consisting of: 4-acetylcytidine (ac4c), 2'-O-methylcytidine (cm), 3-methylcytosine (m3c), N4-methylcytidine (m4c), 5-methylcytidine (m5c), β, and 2-thiocytidine (s2c). In some embodiments, the chemical modification includes 2'-O-methylation modification on the ribose of a nucleotide, 3' phosphorothioate bond modification between nucleotides, or both. In some embodiments, the modification is 2'-O-methylation modification on the ribose of the first three nucleotides at the 5' end, 2'-O-methylation modification on the ribose of the last three nucleotides at the 3' end, internucleotide 3' phosphorothioate modification of the first three nucleotides at the 5' end, and internucleotide 3' phosphorothioate modification of the last three nucleotides at the 3' end.
20. An engineered cell, comprising the TIGIT extracellular region polypeptide according to any one of items 1 to 6, the fusion protein according to item 7 or 8, the engineered receptor according to any one of items 9 to 18, and/or the engineered nucleic acid molecule according to item 19.
21. The engineered cell according to item 20, comprising two or more engineered receptors that bind to the same target molecule or different target molecules.
22. The engineered cell according to item 21, wherein one of the target molecules is CD155, and the other target molecules are one, two or three selected from PSCA, CD123, and CEA. In some embodiments, the target molecules are CD155 and PSCA. In some embodiments, the target molecules are CD155 and CD123. In some embodiments, the target molecules are CD155 and CEA. In some embodiments, the engineered receptor that binds to the target molecule CD155 is the engineered receptor according to any one of claims 16 to 18, and the engineered receptor that binds to one, two or three target molecules selected from PSCA, CD123, and CEA is a CAR targeting PSCA, CD123 or CEA, respectively.
23. The engineered cell according to item 22, wherein in some embodiments, the engineered cell comprises: an engineered receptor targeting CD155 and a CAR whose target molecule is one, two or three selected from PSCA, CD123, and CEA. Herein, in some embodiments, the engineered receptor targeting CD155 comprises: an antigen binding domain, a transmembrane domain, and an intracellular signaling domain, where the intracellular signaling domain consists only of a costimulatory domain. In some embodiments, the engineered cell comprises: an engineered receptor targeting CD155 and a CAR targeting PSCA, wherein the engineered receptor targeting CD155 comprises, from N-terminus to C-terminus, an antigen binding domain, a transmembrane domain, and a costimulatory domain, wherein the antigen binding domain comprises the TIGIT extracellular region polypeptide according to any one of items 1 to 6. In some embodiments, the engineered cell comprises: an engineered receptor targeting CD155 and a CAR targeting PSCA, wherein the engineered receptor targeting CD155 comprises, from N-terminus to C-terminus, an antigen binding domain, a hinge region, a transmembrane domain, and a costimulatory domain, wherein the antigen binding domain is the TIGIT extracellular region polypeptide according to any one of items 1 to 6. In some embodiments, the engineered cell comprises: an engineered receptor targeting CD155 and a CAR targeting CD123, wherein the engineered receptor targeting CD155 comprises, from N-terminus to C-terminus, an antigen binding domain, a transmembrane domain, and a costimulatory domain, wherein the antigen binding domain is the TIGIT extracellular region polypeptide according to any one of items 1 to 6. In some embodiments, the engineered cell comprises: an engineered receptor targeting CD155 and a CAR targeting CD123, wherein the engineered receptor targeting CD155 comprises, from N-terminus to C-terminus, an antigen binding domain, a hinge region, a transmembrane domain, and a costimulatory domain, wherein the antigen binding domain is the TIGIT extracellular region polypeptide according to any one of items 1 to 6. In some embodiments, the engineered cell comprises: an engineered receptor targeting CD155 and a CAR targeting CEA, wherein the engineered receptor targeting CD155 comprises, from N-terminus to C-terminus, an antigen binding domain, a transmembrane domain, and a costimulatory domain, wherein the antigen binding domain is the TIGIT extracellular region polypeptide according to any one of items 1 to 6. In some embodiments, the engineered cell comprises: an engineered receptor targeting CD155 and a CAR targeting CEA, wherein the engineered receptor targeting CD155 comprises, from N-terminus to C-terminus, an antigen binding domain, a hinge region, a transmembrane domain, and a costimulatory domain, wherein the antigen binding domain is the TIGIT extracellular region polypeptide according to any one of items 1 to 6.

The engineered cell according to item 22, wherein in some embodiments, the engineered cell comprises: an engineered receptor targeting CD155 and a CAR whose target molecule is one, two or three selected from PSCA, CD123, and CEA, wherein the engineered receptor targeting CD155 may comprise: an antigen binding domain, a hinge structure, a transmembrane domain, and an intracellular signaling domain, or an antigen binding domain, a transmembrane domain, and an intracellular signaling domain, wherein the intracellular signaling domain may comprise only a primary signal transduction domain such as a CD3ζ signal transduction domain, or may be a CAR structure comprising at least one costimulatory domain and a primary signal transduction domain.
In some embodiments, the antigen binding domain of the engineered receptor that binds to CD155 comprises an amino acid sequence as set forth in any one of SEQ ID NOs:2-26 or a conservative substitution variant thereof;
the antigen binding domain of the CAR that binds to the target molecule PSCA comprises an amino acid sequence as set forth in SEQ ID NO:27 or 28, or a conservative substitution variant thereof, or an amino acid sequence having at least 80%, 85%, 90%, 91%, 92%, 93%, 94%, 95%, 96%, 97%, 98% or 99% identity thereto;
the antigen binding domain of the CAR that binds to the target molecule CEA comprises an amino acid sequence as set forth in SEQ ID NO:29, or a conservative substitution variant thereof, or an amino acid sequence having at least 80%, 85%, 90%, 91%, 92%, 93%, 94%, 95%, 96%, 97%, 98% or 99% identity thereto; and
the antigen binding domain of the CAR that binds to the target molecule CD123 comprises an amino acid sequence as set forth in SEQ ID NO:30, or a conservative substitution variant thereof, or an amino acid sequence having at least 80%, 85%, 90%, 91%, 92%, 93%, 94%, 95%, 96%, 97%, 98% or 99% identity thereto.

24. The engineered cell according to any one of items 20 to 23, which is derived from a T cell, NK cell, macrophage, DC cell, B cell, or a precursor cell thereof. In some embodiments, the engineered cell is derived from a CAR-T, CAR-NK, CAR-macrophage, or CAR-DC. In some embodiments, the engineered cell is derived from a TCR-T cell. In some embodiments, the engineered cell is a TAC-T cell.

25. Use of the TIGIT extracellular region polypeptide according to any one of items 1 to 6, the fusion protein according to item 7 or 8, the engineered receptor according to any one of items 9 to 18, the nucleic acid molecule according to item 19, or the engineered cell according to any one of items 20 to 24 in the manufacture of a medicament for treating cancer.

26. The use according to item 25, wherein the cancer is one or more selected from the group consisting of:
bladder cancer, blood cancer, bone cancer, bone marrow cancer, brain/nervous system cancer, breast cancer, colorectal cancer, esophageal cancer, gastrointestinal cancer, head cancer, kidney cancer, liver cancer, lung cancer, nasopharyngeal cancer, neck cancer, ovarian cancer, pancreatic cancer, prostate cancer, skin cancer, gastric cancer, testicular cancer, tongue cancer, and uterine cancer. In some embodiments, the cancer is breast cancer, pancreatic cancer, bladder cancer and/or human acute myeloid leukemia.

In addition, the present application further provides a method for treating cancer, comprising administering to a subject in need thereof a therapeutically effective amount of the TIGIT extracellular region polypeptide according to any one of items 1 to 6, the fusion protein according to item 7 or 8, the engineered receptor according to any one of items 9 to 18, the nucleic acid molecule according to item 19, or the engineered cell according to any one of items 20 to 25. In some embodiments of the method for treating cancer, the cancer is one or more selected from the following:
bladder cancer, blood cancer, bone cancer, bone marrow cancer, brain/nervous system cancer, breast cancer, colorectal cancer, esophageal cancer, gastrointestinal cancer, head cancer, kidney cancer, liver cancer, lung cancer, nasopharyngeal cancer, neck cancer, ovarian cancer, pancreatic cancer, prostate cancer, skin cancer, gastric cancer, testicular cancer, tongue cancer, and uterine cancer. In some embodiments, the cancer is breast cancer, pancreatic cancer, bladder cancer and/or human acute myeloid leukemia. In some embodiments, the subject or patient is a human patient.

28. A method for prolonging an *in vivo* persistence period of CAR-T cells, comprising expressing on a membrane of the CAR-T cells the TIGIT extracellular region polypeptide according to any one of items 1 to 6, the fusion protein according to item 7 or 8, or the engineered receptor according to any one of items 9 to 18.

29. A method for enhancing an *in vivo* expansion ability of CAR-T cells, comprising expressing on a membrane of the CAR-T cells the TIGIT extracellular region polypeptide according to any one of items 1 to 6, the fusion protein according to item 7 or 8, or the engineered receptor according to any one of items 9 to 18.

30. A method for enhancing an *in vivo* killing ability of CAR-T against target cells, comprising expressing on a membrane of the CAR-T cells the TIGIT extracellular region polypeptide according to any one of items 1 to 6, the fusion protein according to item 7 or 8, or the engineered receptor according to any one of items 9 to 18.

In some embodiments, the *in vivo* refers to within a cancer patient or subject. In some embodiments, the *in vivo* refers to within a patient or subject with abnormal expression of CD155 in certain tissues and organs. In some embodiments, the cancer is one or more selected from the following:
bladder cancer, blood cancer, bone cancer, bone marrow cancer, brain/nervous system cancer, breast cancer, colorectal cancer, esophageal cancer, gastrointestinal cancer, head cancer, kidney cancer, liver cancer, lung cancer, nasopharyngeal cancer, neck cancer, ovarian cancer, pancreatic cancer, prostate cancer, skin cancer, gastric cancer, testicular cancer, tongue cancer, and uterine cancer.

Specifically, the present application relates to:
1. A TIGIT extracellular region polypeptide, comprising mutations at the following positions relative to a reference sequence:
   1) position 48, position 57, and position 86; or
   2) position 48, position 71, and position 88;
      wherein the reference sequence is an amino acid sequence as set forth in SEQ ID NO:1, and wherein amino acid position numbering is based on the reference sequence.
2. The TIGIT extracellular region polypeptide according to item 1, comprising the following mutation combination relative to the reference sequence:
   1) C48W, S57P, and F86S; or
   2) C48W, G71D, and I88V.

In some embodiments, the TIGIT extracellular region polypeptide comprises only the following mutations relative to amino acids at positions 33 to 93 of the reference sequence:
1) C48W, S57P, and F86S; or
2) C48W, G71D, and I88V.

3. The TIGIT extracellular region polypeptide according to claim 2, comprising a combination of amino acid positions of any one of 1) to 4):
1) 34T, 39E, 48W, 57P, 61K, 70L, 71G, 80N, 86S, and 88I;
2) 34T, 39E, 48W, 57S, 61K, 70L, 71D, 80N, 86F, and 88V;
3) 9T, 20I, 21I, 34T, 39E, 48W, 57P, 61K, 70L, 71G, 80N, 86S, 88I, 101I, and 110V; or
4) 9T, 20I, 21I, 34T, 39E, 48W, 57S, 61K, 70L, 71D, 80N, 86F, 88V, 101I, and 110V.

4. The TIGIT extracellular region polypeptide according to any one of items 1 to 3, comprising at least amino acids corresponding to positions 33 to 93 of the reference sequence. In some embodiments, the polypeptide comprises deletion of one or more amino acids at amino acid positions corresponding to positions 33 to 93 of the reference sequence. In some embodiments, the polypeptide comprises one or more additional amino acids at any two amino acid positions among the amino acid positions corresponding to positions 33 to 93 of the reference sequence. In some embodiments, the polypeptide does not comprise other amino acids at the C-terminus and/or N-terminus side of the amino acids corresponding to positions 33 to 93 of the reference sequence, for example, amino acids corresponding to positions numbered less than 33 of the reference sequence and/or amino acids corresponding to positions numbered greater than 93 of the reference sequence. In some embodiments, the polypeptide further comprises other amino acids at the C-terminus and/or N-terminus side of the amino acids corresponding to positions 33 to 93 of the reference sequence, for example, amino acids corresponding to positions numbered less than 33 of the reference sequence and/or amino acids corresponding to positions numbered greater than 93 of the reference sequence.

5. The TIGIT extracellular region polypeptide according to any one of items 1 to 5, comprising any amino acid sequence selected from the following, or a conservative substitution variant of any amino acid sequence selected from the following, or in some embodiments, the polypeptide comprising an amino acid sequence having at least 80% (for example, 81%, 82%, 83%, 84%, 85%, 90%, 91%, 92%, 93%, 94%, 95%, 96%, 97%, 98%, 98.5%, 99%, 99.5%) identity to any amino acid sequence selected from the following:
SEQ ID NOs:2-8.

6. A fusion protein, comprising the TIGIT extracellular region polypeptide according to any one of items 1 to 5.

7. The fusion protein according to item 6, further comprising one or more polypeptides that bind to a tumor antigen and/or an immune checkpoint protein;
wherein optionally, the polypeptide that binds to the tumor antigen and/or the immune checkpoint protein is an antibody, ligand or receptor of the tumor antigen and/or the immune checkpoint protein; and
optionally the antibody against the tumor antigen and/or the immune checkpoint protein is a single-chain antibody (scFv), Fab, F(ab')2, Fab', Fv, Fd, dAb or diabody.

8. An engineered receptor, comprising an antigen binding domain, wherein the antigen binding domain comprises the TIGIT extracellular region polypeptide according to any one of items 1 to 5, or the fusion protein according to item 6 or 7.

9. The engineered receptor according to item 8, further comprising a signal transduction domain, wherein the signal transduction domain comprises a primary signal transduction domain and/or a costimulatory domain.

10. The engineered receptor according to item 8 or 9, which is a chimeric antigen receptor (CAR), a T cell receptor (TCR) or a T cell antigen coupler (TAC).

11. The engineered receptor according to any one of items 9 or 10, wherein the costimulatory domain comprises a signal transduction domain of one or more molecules selected from the group consisting of:
CD27, CD28, 4-1BB, OX40, CD30, CD40, CD2, LFA-1, LIGHT, NKG2C, B7-H3, PD-1, ICOS, CDS, ICAM-1, GITR, BAFFR, LIGHTR, SLAMF7, CD7, NKp80 (KLRF1), CD160, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CDI11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1, SLAMF4, CD84, CD96, CEACAM1, CRTAM, CD229, CD160, PSGL1, CD100, CD69, SLAMF6, SLAMF1, SLAMF8, CD162, LTBR, LAT, GADS, SLP-76, PAG/Cbp, NKp44, NKp30, NKp46, NKG2D, and a ligand that specifically binds to CD83.

12. The engineered receptor according to any one of items 9 to 11, wherein the primary signal transduction domain comprises a signal transduction domain of one or more molecules selected from the group consisting of: CD3ζ, CD3γ, CD3δ, CD3ε, CD5, CD22, FcRγ, FcRβ, FcεRIγ, FcεRIβ, FcγRIIa, CD79α, CD79β, CD66d, DAP10, and DAP12.

13. The engineered receptor according to item 12, further comprising a transmembrane domain between the antigen binding domain and the signal transduction domain, wherein the transmembrane domain comprises a transmembrane domain of any one or more molecules selected from the group consisting of: ICOS, CD4, CD8α, CD28, CD3ζ, and TIGIT.

14. The engineered receptor according to item 13, wherein the antigen binding domain and the transmembrane domain are linked via a hinge region, preferably the hinge region is a hinge region of TIGIT, CD7, IgG, IgD, CD8α or CD28, or a combination thereof.

15. The engineered receptor according to any one of claims 9 to 14, from N-terminus to C-terminus, comprising or consisting of in sequence: the TIGIT extracellular region polypeptide, a transmembrane domain, and a costimulatory domain, wherein a hinge region is further comprised or not comprised between the TIGIT extracellular region polypeptide and the transmembrane domain, wherein
the costimulatory domain comprises or is a signal transduction domain of one or more molecules selected from the group consisting of:
CD27, CD28, 4-1BB, OX40, CD30, CD40, CD2, LFA-1, LIGHT, NKG2C, B7-H3, PD-1, ICOS, CDS, ICAM-1, GITR, BAFFR, LIGHTR, SLAMF7, CD7, NKp80 (KLRF1), CD160, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CDI11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1, SLAMF4, CD84, CD96, CEACAM1, CRTAM, CD229, CD160, PSGL1, CD100, CD69, SLAMF6, SLAMF1, SLAMF8, CD162, LTBR, LAT, GADS, SLP-76, PAG/Cbp, NKp44, NKp30, NKp46, NKG2D, and a ligand that specifically binds to CD83;
the transmembrane domain comprises or is a transmembrane domain of any one or more molecules selected from the group consisting of: ICOS, CD4, CD8α, CD28, CD3ζ, and TIGIT; and
the hinge region comprises or is a hinge region of TIGIT, CD7, IgG, IgD, CD8α or CD28, or a combination thereof.

In some embodiments, the engineered receptor comprises, from N-terminus to C-terminus: the TIGIT extracellular region polypeptide, a transmembrane domain, and a costimulatory domain; or the engineered receptor comprises, from N-terminus to C-terminus: the TIGIT extracellular region polypeptide, a hinge region, a transmembrane domain, and a costimulatory domain.

16. The engineered receptor according to item 15, from N-terminus to C-terminus, comprising or consisting of in sequence: the TIGIT extracellular region polypeptide, a CD28 transmembrane domain, and a CD28 signal transduction domain.

17. The engineered receptor according to any one of items 8 to 14, from N-terminus to C-terminus, comprising or consisting of in sequence any one of 1) to 4):
1) the TIGIT extracellular region polypeptide, a CD28 transmembrane domain, a CD28 signal transduction domain, and a CD3ζ signal transduction domain;
2) the TIGIT extracellular region polypeptide, a CD8 hinge region, a CD8 transmembrane domain, a 4-1BB signal transduction domain, and a CD3ζ signal transduction domain;
3) the TIGIT extracellular region polypeptide, a CD7 hinge region, a CD28 transmembrane domain, a CD28 signal transduction domain, and a CD3ζ signal transduction domain; and
4) the TIGIT extracellular region polypeptide, a G4h hinge region, a CD28 transmembrane domain, a CD28 signal transduction domain-4-1BB signal transduction domain, and a CD3ζ signal transduction domain.

18. An engineered nucleic acid molecule, encoding the TIGIT extracellular region polypeptide according to any one of items 1 to 5, the fusion protein according to item 6 or 7, or the engineered receptor according to any one of items 8 to 17. In some embodiments, the engineered nucleic acid molecule is DNA, RNA (e.g., mRNA), or a DNA-RNA hybrid molecule.

In some embodiments, the engineered nucleic acid molecule is chemically modified. For example, in some embodiments, one or more thymidines of the engineered nucleic acid molecule are replaced with uridine. In some embodiments, one or more uridines of the engineered nucleic acid molecule are replaced with thymidine. In some embodiments, one or more guanosines of the engineered nucleic acid molecule are replaced with inosine. In some embodiments, the chemical modification is such that one or more nucleotides of the nucleic acid molecule are replaced with their corresponding nucleotide derivatives. For example, in some embodiments, one or more uridines of the engineered nucleic acid molecule are each replaced with one or more selected from the group consisting of: 5-(carboxyhydroxymethyl)uridine (chm5u), 5-carboxymethylaminomethyluridine (cmnm5u), 5-carboxymethylaminomethyl-2-thiouridine (cmnm5s2u), dihydrouridine (dhu), 2'-O-methylpseudouridine (fm), 1-methylpseudouridine (m1f), 3-(3-amino-3-carboxy-propyl)uridine ((acp3)u), uridine-5-oxyacetic acid (o5u), uridine-5-oxyacetic acid methyl ester (mv), 5-methoxycarbonylmethyluridine (mcm5u), 5-methoxycarbonylmethyl-2-thiouridine (mcm5s2u), 5-methoxyuridine (mo5u), 5-methyl-2-thiouridine (s2t), 2-thiouridine (s2u), 4-thiouridine (s4u), 5-methyluridine (m5u), 2'-O-methyl-5-methyluridine (tm), 2'-O-methyluridine (um), 5-methylaminomethyluridine (mam5u), 5-methylaminomethyl-2-thiouridine (mam5s2u), pseudouridine (p), and 5-methoxycarbonylmethyl-2-thioguanosine (mcm5s2u). In some embodiments, one or more guanosines of the engineered nucleic acid molecule are each replaced with one or more selected from the group consisting of: wybutoxosine (osyw), wybutosine (yw), 1-methylinosine (mli), 2'-O-methylguanosine (gm), 1-methylguanosine (m1g), 2,2-dimethylguanosine (m22g), 2-methylguanosine (m2g), 7-methylguanosine (m7g), β,D-galactosylqueuosine (gal q), queuosine (q), and β,D-mannosylqueuosine (man q). In some embodiments, one or more adenosines of the engineered nucleic acid molecule are each replaced with one or more selected from the group consisting of: N6-isopentenyladenosine (i6a), 1-methyladenosine (m1a), 2-methyladenosine (m2a), N6-methyladenosine (m6a), 2-methylthio-N6-isopentenyladenosine (ms2i6a), N-((9-β-D-ribofuranosyl-2-methylthiopurine-6-yl)carbamoyl)threonine (ms2t6a), N-((9-β-D-ribofuranosylpurin-6-yl)N-methylcarbamoyl)threonine (mt6a), N-((9-β-D-ribofuranosylpurin-6-yl)-carbamoyl)threonine (t6a), queuosine (q), and D-mannosylqueuosine (man q). In some embodiments, one or more cytidines of the engineered nucleic acid molecule are each replaced with one or more selected from the group consisting of: 4-acetylcytidine (ac4c), 2'-O-methylcytidine (cm), 3-methylcytosine (m3c), N4-methylcytidine (m4c), 5-methylcytidine (m5c), β, and 2-thiocytidine (s2c). In some embodiments, the chemical modification includes 2'-O-methylation modification on the ribose of a nucleotide, 3' phosphorothioate bond modification between nucleotides, or both. In some embodiments, the modification is 2'-O-methylation modification on the ribose of the first three nucleotides at the 5' end, 2'-O-methylation modification on the ribose of the last three nucleotides at the 3' end, internucleotide 3' phosphorothioate modification of the first three nucleotides at the 5' end, and internucleotide 3' phosphorothioate modification of the last three nucleotides at the 3' end.

19. An engineered cell, comprising the TIGIT extracellular region polypeptide according to any one of items 1 to 5, the fusion protein according to item 6 or 7, the engineered receptor according to any one of items 8 to 17, and/or the engineered nucleic acid molecule according to item 18.

20. The engineered cell according to item 19, comprising two or more engineered receptors that bind to the same target molecule or different target molecules.

21. The engineered cell according to item 20, wherein one of the target molecules is CD155, and the other target molecule(s) is/are one, two or three selected from PSCA, CD123, and CEA. In some embodiments, the target molecules are CD155 and PSCA. In some embodiments, the target molecules are CD155 and CD123. In some embodiments, the target molecules are CD155 and CEA. In some embodiments, the engineered receptor that binds to the target molecule CD155 is the engineered receptor according to any one of claims 15 to 17, and the engineered receptor that binds to one, two or three target molecules selected from PSCA, CD123, and CEA is a CAR targeting PSCA, CD123 or CEA, respectively.

22. The engineered cell according to item 21, wherein in some embodiments, the engineered cell comprises: an engineered receptor targeting CD155 and a CAR whose target molecule is one, two or three selected from PSCA, CD123, and CEA. Herein, in some embodiments, the engineered receptor targeting CD155 comprises: an antigen binding domain, a transmembrane domain, and an intracellular signaling domain, wherein the intracellular signaling domain consists only of a costimulatory domain. In some embodiments, the engineered cell comprises: an engineered receptor targeting CD155 and a CAR targeting PSCA, wherein the engineered receptor targeting CD155 comprises, from N-terminus to C-terminus, an antigen binding domain, a transmembrane domain, and a costimulatory domain, wherein the antigen binding domain comprises the TIGIT extracellular region polypeptide according to any one of items 1 to 5. In some embodiments, the engineered cell comprises: an engineered receptor targeting CD155 and a CAR targeting PSCA, wherein the engineered receptor targeting CD155 comprises, from N-terminus to C-terminus, an antigen binding domain, a hinge region, a transmembrane domain, and a costimulatory domain, wherein the antigen binding domain is the TIGIT extracellular region polypeptide according to any one of items 1 to 5. In some embodiments, the engineered cell comprises: an engineered receptor targeting CD155 and a CAR targeting CD123, wherein the engineered receptor targeting CD155 comprises, from N-terminus to C-terminus, an antigen binding domain, a transmembrane domain, and a costimulatory domain, wherein the antigen binding domain is the TIGIT extracellular region polypeptide according to any one of items 1 to 5. In some embodiments, the engineered cell comprises: an engineered receptor targeting CD155 and a CAR targeting CD123, wherein the engineered receptor targeting CD155 comprises, from N-terminus to C-terminus, an antigen binding domain, a hinge region, a transmembrane domain, and a costimulatory domain, wherein the antigen binding domain is the TIGIT extracellular region polypeptide according to any one of items 1 to 5. In some embodiments, the engineered cell comprises: an engineered receptor targeting CD155 and a CAR targeting CEA, wherein the engineered receptor targeting CD155 comprises, from N-terminus to C-terminus, an antigen binding domain, a transmembrane domain, and a costimulatory domain, wherein the antigen binding domain is the TIGIT extracellular region polypeptide according to any one of items 1 to 5. In some embodiments, the engineered cell comprises: an engineered receptor targeting CD155 and a CAR targeting CEA, wherein the engineered receptor targeting CD155 comprises, from N-terminus to C-terminus, an antigen binding domain, a hinge region, a transmembrane domain, and a costimulatory domain, wherein the antigen binding domain is the TIGIT extracellular region polypeptide according to any one of items 1 to 5.

The engineered immune cell according to item 21, wherein in some embodiments, the engineered cell comprises: an engineered receptor targeting CD155 and a CAR whose target molecule is one, two or three selected from PSCA, CD123, and CEA, wherein the engineered receptor targeting CD155 comprises: an antigen binding domain, a hinge structure, a transmembrane domain, and an intracellular signaling domain, or an antigen binding domain, a transmembrane domain, and an intracellular signaling domain, wherein the intracellular signaling domain may comprise only a primary signal transduction domain, such as a CD3ζ signal transduction domain, or may be a CAR structure comprising at least one costimulatory domain and a primary signal transduction domain.

23. The engineered cell according to item 22, wherein the antigen binding domain of the engineered receptor that binds to CD155 comprises an amino acid sequence as set forth in any one of SEQ ID NOs:2-8 or a conservative substitution variant thereof;
the antigen binding domain of the CAR that binds to the target molecule PSCA comprises an amino acid sequence as set forth in SEQ ID NO:9 or 10, or a conservative substitution variant thereof, or an amino acid sequence having at least 80%, 85%, 90%, 91%, 92%, 93%, 94%, 95%, 96%, 97%, 98% or 99% identity thereto;
the antigen binding domain of the CAR that binds to the target molecule CEA comprises an amino acid sequence as set forth in SEQ ID NO:11, or a conservative substitution variant thereof, or an amino acid sequence having at least 80%, 85%, 90%, 91%, 92%, 93%, 94%, 95%, 96%, 97%, 98% or 99% identity thereto; and
the antigen binding domain of the CAR that binds to the target molecule CD123 comprises an amino acid sequence as set forth in SEQ ID NO:12, or a conservative substitution variant thereof, or an amino acid sequence having at least 80%, 85%, 90%, 91%, 92%, 93%, 94%, 95%, 96%, 97%, 98% or 99% identity thereto.

24. The engineered cell according to any one of items 19 to 23, which is derived from a T cell, NK cell, macrophage, DC cell, B cell, or a precursor cell thereof. In some embodiments, the engineered cell is CAR-T, CAR-NK, CAR-macrophage, or CAR-DC. In some embodiments, the engineered cell is a TCR-T cell. In some embodiments, the engineered cell is a TAC-T cell.

25. Use of the TIGIT extracellular region polypeptide according to any one of items 1 to 5, the fusion protein according to item 6 or 7, the engineered receptor according to any one of items 8 to 17, the nucleic acid molecule according to item 18, or the engineered cell according to any one of items 19 to 24 in the manufacture of a medicament for treating cancer.

26. The use according to item 25, wherein the cancer is one or more selected from the group consisting of:
bladder cancer, blood cancer, bone cancer, bone marrow cancer, brain/nervous system cancer, breast cancer, colorectal cancer, esophageal cancer, gastrointestinal cancer, head cancer, kidney cancer, liver cancer, lung cancer, nasopharyngeal cancer, neck cancer, ovarian cancer, pancreatic cancer, prostate cancer, skin cancer, gastric cancer, testicular cancer, tongue cancer, and uterine cancer. In some embodiments, the cancer is breast cancer, pancreatic cancer, bladder cancer and/or human acute myeloid leukemia.

In addition, the present application further provides a method for treating cancer, comprising administering to a subject in need thereof a therapeutically effective amount of the TIGIT extracellular region polypeptide according to any one of items 1 to 5, the fusion protein according to item 6 or 7, the engineered receptor according to any one of items 8 to 17, the nucleic acid molecule according to item 18, or the engineered immune cell according to any one of items 19 to 20. In some embodiments of the method for treating cancer, the cancer is one or more selected from the group consisting of:
bladder cancer, blood cancer, bone cancer, bone marrow cancer, brain/nervous system cancer, breast cancer, colorectal cancer, esophageal cancer, gastrointestinal cancer, head cancer, kidney cancer, liver cancer, lung cancer, nasopharyngeal cancer, neck cancer, ovarian cancer, pancreatic cancer, prostate cancer, skin cancer, gastric cancer, testicular cancer, tongue cancer, and uterine cancer. In some embodiments, the cancer is breast cancer, pancreatic cancer, bladder cancer and/or human acute myeloid leukemia. In some embodiments, the subject or patient is a human patient.

27. A method for prolonging an *in vivo* persistence period of CAR-T cells, comprising expressing on a membrane of the CAR-T cells the TIGIT extracellular region polypeptide according to any one of items 1 to 5, the fusion protein according to item 6 or 7, or the engineered receptor according to any one of items 8 to 17.

28. A method for enhancing *in vivo* expansion ability of CAR-T cells, comprising expressing on a membrane of the CAR-T cells the TIGIT extracellular region polypeptide according to any one of items 1 to 5, the fusion protein according to item 6 or 7, or the engineered receptor according to any one of items 8 to 17.

29. A method for enhancing *in vivo* killing ability of CAR-T against target cells, comprising expressing on a membrane of the CAR-T cells the TIGIT extracellular region polypeptide according to any one of items 1 to 5, the fusion protein according to item 6 or 7, or the engineered receptor according to any one of items 8 to 17.

In some embodiments, the *in vivo* refers to within a cancer patient or subject. In some embodiments, the *in vivo* refers to within a patient or subject with abnormal expression of CD155 in certain tissues and organs. In some embodiments, the cancer is one or more selected from the group consisting of:
bladder cancer, blood cancer, bone cancer, bone marrow cancer, brain/nervous system cancer, breast cancer, colorectal cancer, esophageal cancer, gastrointestinal cancer, head cancer, kidney cancer, liver cancer, lung cancer, nasopharyngeal cancer, neck cancer, ovarian cancer, pancreatic cancer, prostate cancer, skin cancer, gastric cancer, testicular cancer, tongue cancer, and uterine cancer.

2A self-cleaving peptide is a commonly used multi-gene expression strategy that enables co-expression of multiple genes at the translation level. This peptide was first discovered in foot-and-mouth disease virus (FMDV) in 1991, with an average length of 18-22 amino acids. To date, multiple distinct 2A peptides have been identified, derived from foot-and-mouth disease virus 2A (F2A), porcine teschovirus-1 2A (P2A), thosea asigna virus 2A (T2A), and equine rhinitis A virus 2A (E2A), respectively. Their main working principle is that during translation, when the ribosome recognizes the 2A peptide, it skips the synthesis of the glycyl-prolyl peptide bond therein and slips, thereby directly generating two independent proteins. The purpose of using the 2A peptide in the present application is to obtain the engineered immune cell expressing the engineered receptor of the present application, ultimately validate the function of the engineered immune cell through experiments in examples, and further validate the unpredictable effect of the engineered receptor of the present application on the engineered immune cell. In addition to the above 2A self-cleaving peptide, IRES (internal ribosome entry site, IRES) can also be used. IRES can recruit ribosomes to translate mRNA, allowing for independent expression of multiple proteins. The coding genes (also referred to as ORFs) for target molecules such as the engineered receptor of the present application, at least one chimeric antigen receptor (CAR), and other fusion proteins are separated by the internal ribosome entry sites (IRES), and a single mRNA transcript will produce multiple proteins. The above-mentioned 2A peptide, IRES, and other small molecule sequences with similar functions can be referred to as linkers. In addition to using the above linkers to achieve multi-gene expression, the above-mentioned engineered cell can also be obtained by introducing different genes separately into a target cell via transduction, or by introducing constructed vectors expressing different genes into a target gene via simultaneous transduction. Whether using linkers or separately constructing expression vectors of multiple genes for introduction into the target cells via transduction, the purpose of obtaining the engineered cell expressing the TIGIT extracellular region polypeptide can be finally achieved, and the function and unpredictable effects of the engineered cell are primarily based on the protein molecules it expresses and the final engineered cell itself.

The polypeptide, fusion protein, CAR, and engineered cell provided in the present application can recognize CD155 more specifically in the presence of CD112 and are less interfered by CD112. Since CD112 is widely expressed on normal cells of mammals, the CAR and engineered cell provided in the present application can exhibit higher *in vivo* safety. In functional validation of the CAR provided in the present application in the fields of CAR-T and CAR-NK, it indeed achieves a more specific and high-proportion killing effect on the CD155-expressing target cells.

### BRIEF DESCRIPTION OF THE DRAWINGS

FIG. 1 shows flow cytometry staining of CD112 in MDA-MB-231-Luc-GFP, where the horizontal axis represents an experimental group and a control group, and the vertical axis represents mean fluorescence intensity (MFI) value of cells stained with anti-CD112 antibody in the experimental group and the control group. Results indicate that the MFI of CD112 in MDA-MB-231-Luc-GFP is approximately 4-fold higher than that in the control group.
FIG. 2A shows specificity assay for five TIGIT mutant peptides.
FIG. 2B shows specificity assay for two TIGIT mutant peptides.
FIG. 2C shows specificity assay for seven TIGIT mutant peptides.
FIG. 2D shows specificity assay for nine TIGIT mutant peptides.
FIG. 2E shows specificity assay for three TIGIT mutant peptides.
FIG. 3 shows assay of binding between the screened TIGIT mutant peptides and CD112.
FIG. 4 shows assay of binding between the screened TIGIT mutant peptides and CD155.
FIG. 5 shows functional validation of the screened TIGIT mutant peptides against CD155-negative cells.
FIG. 6 shows *in vivo* efficacy evaluation of MDA-MB-231 tumorigenesis models.
FIG. 7A shows killing of seven CARs with different structures against MDA-MB-231-Luc-GFP.
FIG. 7B shows killing of five CARs with different structures against HPAC-Luc-GFP.
FIG. 7C shows killing of two CARs with different structures against HT1376-Luc-GFP.
FIG. 8A shows CEA expression in DLD1-CEA-Luc-GFP.
FIG. 8B shows CEA expression in DLD1-Luc-GFP.
FIG. 8C shows CD155 expression in DLD1-CEA-Luc-GFP.
FIG. 9A shows killing of CAR37, targeting both CEA and CD155, against DLD-1-CEA-Luc-GFP and DLD-1-Luc-GFP cells.
FIG. 9B shows *in vitro* killing of CAR38, targeting both CD123 and CD155, against Molm-13-Luc-GFP.
FIG. 9C shows *in vitro* killing of CAR-T cells targeting both PSCA and CD155, with four different CAR structures, against three cell models.
FIG. 10 shows killing of CAR42 and CAR45 against two cell models.
FIG. 11 shows of IFN-γ cytokine secretion during *in vitro* killing of CAR42 and CAR45 against two cell models.
FIG. 12 shows *in vivo* efficacy of CAR42 and CAR45 in DLD-1-CEA-Luc-GFP intraperitoneal tumor-bearing models.
FIG. 13 shows blood copy number in the intraperitoneal tumor evaluation models.
FIG. 14 shows validation of influence of TIGIT mutant peptides on *in vivo* effectiveness of CARs.
FIG. 15A shows validation of effectiveness of CD155-targeting multi-target CAR-T against acute myeloid leukemia.
FIG. 15B shows validation of effectiveness of CD155-targeting multi-target CAR-T against pancreatic cancer and bladder cancer.
FIGS. 16A and 16B show validation of *in vivo* effectiveness of CAR-T cells constructed with different TIGIT mutant peptides, wherein FIG. 16A shows mouse images of *in vivo* tumor killing by CAR-T cells expressing CARs that use different TIGIT mutant peptides as an extracellular recognition region, in immunodeficient mouse models of acute lymphoblastic leukemia; and FIG. 16B shows fluorescence statistical curves.
FIG. 17 shows *in vitro* killing of CAR53 against three target cells.
FIG. 18 shows validation of effectiveness of CAR-T with mutated TIGIT peptide as an extracellular recognition domain against pancreatic cancer.
FIG. 19 shows validation of effectiveness of CAR-T with mutated TIGIT peptide as an extracellular recognition domain against bladder cancer.
FIG. 20 shows killing of malignant tumors of different indications by CAR-T cells with different CAR structures.
FIG. 21 shows *in vitro* killing of target cells of three different indications by CAR53 and CAR61.
FIG. 22 shows *in vitro* effectiveness data of CAR-T cells expressing an engineered receptor with a TIGIT mutant peptide as an antigen binding domain.
FIG. 23 shows *in vivo* effectiveness of CAR-T cells expressing an engineered receptor with a TIGIT mutant peptide as an antigen binding domain against colorectal cancer tumors in tumor-bearing mice with colorectal cancer indications.
FIG. 24 shows *in vivo* tumor fluorescence curves of CAR-T cells expressing an engineered receptor with a TIGIT mutant peptide as an antigen binding domain against colorectal cancer tumors in tumor-bearing mice with colorectal cancer indications.
FIG. 25 shows *in vivo* copy number in mice following tumor killing by CAR-T cells expressing an engineered receptor with a TIGIT mutant peptide as an antigen binding domain, in tumor-bearing mice with colorectal cancer indications.
FIG. 26 shows *in vitro* killing of Molm-13-Luc-GFP by CAR60.

### DETAILED DESCRIPTION OF EMBODIMENTS

The present application relates to a TIGIT mutant peptide derived from an extracellular portion of a TIGIT protein. For example, it comprises a mutation at position 48 relative to a reference sequence, its ability to bind to CD155 is significantly higher than its ability to bind to CD112, and the difference between its CD155-binding ability to and its CD112-binding ability is significantly greater than that of an extracellular portion of the wild-type TIGIT protein (whose amino acid sequence is as set forth in SEQ ID NO:1). For example, in some embodiments, the TIGIT mutant peptide has a CD155-binding ability comparable to or stronger than that of the extracellular portion of the wild-type TIGIT protein, but its CD112-binding ability is significantly reducedCD112-binding ability. In some embodiments, the TIGIT mutant peptide has a stronger CD155-binding ability and stronger CD112-binding ability than the extracellular portion of the wild-type TIGIT protein, but compared with the extracellular portion of the wild-type TIGIT protein, the enhancement in the CD155-binding ability of the TIGIT mutant peptide is much greater than the enhancement in its CD112-binding ability. In some embodiments, the TIGIT mutant peptide has a weaker CD155-binding ability and weaker CD112-binding ability than the extracellular portion of the wild-type TIGIT protein, but compared with the extracellular portion of the wild-type TIGIT protein, the reduction in the CD155-binding ability of the TIGIT mutant peptide is much smaller than the reduction in its CD112-binding ability. In some embodiments, the TIGIT mutant peptide has a stronger CD155-binding ability and relatively unchanged CD112-binding ability than the extracellular portion of the wild-type TIGIT protein. In some embodiments, the TIGIT mutant peptide has a stronger CD155-binding ability and weaker CD112-binding ability than the extracellular portion of the wild-type TIGIT protein. The present application further relates to an engineered polypeptide derived from an extracellular portion of a TIGIT protein. For example, it comprises mutations at positions 48, 57, and 86, or mutations at positions 48, 71, and 88 relative to a reference sequence. Compared with the extracellular portion of the wild-type TIGIT protein (whose amino acid sequence is as set forth in SEQ ID NO:1), its binding ability to CD155 is significantly improved. Moreover, an engineered receptor using the engineered polypeptide as an antigen binding domain (or a ligand-binding domain) can result in a stronger target cell killing ability of the immune effector cell than an engineered receptor using the extracellular portion of the wild-type TIGIT protein as an antigen binding domain (or a ligand-binding domain). Herein, the target cell is a cell that highly expresses CD155. In some embodiments, the target cell is a tumor cell, such as a bladder cancer cell, a prostate cancer cell, a pancreatic cancer cell or the like. In addition, the present application further relates to a fusion protein or an engineered receptor comprising the TIGIT mutant peptide, for example, a fusion protein, CAR, engineered TCR, TAC, etc. comprising an antibody or an antigen-recognizing fragment of the antibody; an engineered cell comprising the fusion protein or engineered receptor, such as T cell, NK cell, macrophage, DC cell, B cell, or a progenitor cell thereof; and use of the aforementioned TIGIT mutant peptide, engineered receptor, fusion protein, or engineered cell.

### Definitions

It should be understood that the present application is not limited to the aspects described herein, and certainly may vary. It should also be understood that, as used herein, the terms are used only to describe particular aspects and are not intended to be limiting, as the scope of the present application is limited only by the appended claims.

Unless otherwise defined, all technical and scientific terms used herein have the same meaning as commonly understood by those ordinarily skilled in the art to which the described technology belongs. All technical and patent publications cited herein are incorporated herein by reference in their entirety. Unless otherwise indicated, those skilled in the art would employ conventional techniques within the technical scope of the art, including tissue culture, immunology, molecular biology, microbiology, cell biology, and recombinant DNA.

The term "TIGIT" is an abbreviation for T cell immunoreceptor with Ig and ITIM domains, also known as WUCAM, Vstm3 or VSIG9. It consists of an extracellular region of an extracellular immunoglobulin variable domain (IgV), a type I transmembrane domain, and an intracellular domain possessing a canonical immunoreceptor tyrosine-based inhibitory motif (ITIM) and an immunoglobulin tyrosine tail (ITT)-like motif. TIGIT is a member of the poliovirus receptor/nectin family, a subset of the immunoglobulin superfamily. TIGIT is an immunoreceptor inhibitory checkpoint involved in tumor immune surveillance. TIGIT competes with the immune activator receptor CD226 (DNAM-1) for the same set of ligands: CD155 (PVR or poliovirus receptor) and CD112 (nectin-2 or PVRL2). An exemplary TIGIT is human TIGIT, encoded by the gene with gene ID 201633 in the NCBI database. Herein, the term "TIGIT extracellular region" refers to an amino acid sequence corresponding to positions 1 to 120 of a reference sequence SEQ ID NO:1 in a TIGIT protein or variant thereof, which can be determined by sequence alignment with the reference sequence. For example, by introducing gaps or other methods, any TIGIT protein or variant thereof can be aligned to have identical residues at as many positions as possible with the reference sequence. After alignment, the portion composed of amino acids at positions corresponding to position 1 and position 120 of the amino acid sequence set forth in SEQ ID NO:1 and all amino acid positions therebetween in the TIGIT protein or variant thereof, linked in the order of their positions in the TIGIT protein or variant thereof, may be referred to as the "TIGIT extracellular region." For example, for the TIGIT protein with GenBank accession No. ACD74757.1, its extracellular region corresponds to positions 22 to 141.

As used herein, the "TIGIT extracellular region polypeptide" may refer to any peptide fragment within the TIGIT extracellular region, any truncation of the TIGIT extracellular region, or the full-length TIGIT extracellular region.

As used herein, the "reference sequence" specifically refers to the amino acid sequence as set forth in SEQ ID NO:1, which is used to define positions of amino acids in the present application. Unless otherwise indicated, the numbering of all amino acid positions in the present application is based on the reference sequence. As used in the present application, a mutation "relative to a reference sequence" refers to that, as determined by sequence alignment, at a defined amino acid position, there is an amino acid different from the amino acid at that position in the reference sequence (substitution), there is more than one amino acid at that amino acid position (addition), and/or there is a gap at that amino acid position (deletion). For example, the TIGIT extracellular region polypeptide "comprising a mutation at position 48 relative to the reference sequence" means that the amino acid corresponding to position 48 of SEQ ID NO:1 in the TIGIT extracellular region polypeptide is not 48C of SEQ ID NO:1 (but is, for example, 48W), or it comprises two or more amino acids at position 48, or comprises no amino acid at position 48. In the present application, "the amino acid position numbering is based on the reference sequence" in a polypeptide, protein, or amino acid sequence means that after aligning the polypeptide, protein, or amino acid sequence with the reference sequence to have identical residues at as many positions as possible, by means such as introducing a gap into or deleting an amino acid from the polypeptide, protein, or amino acid sequence, the amino acids of the reference sequence are sequentially numbered starting from 1, and amino acids of the polypeptide, protein, or amino acid sequence that correspond to those in the reference sequence by alignment are assigned the same position numbers.

"CD155," also known as "PVR," i.e., poliovirus receptor, is also referred to as Necl5 and Tage4. CD155 is a cell surface adhesion molecule that is dramatically overexpressed in several human malignant tumors, while its expression is relatively low or absent in most healthy tissues. Consistent with the biological properties of PVR, its overexpression promotes invasion, migration, and proliferation of tumor cells, and is associated with poor prognosis and enhanced tumor progression.

"CD112," also known as "PVRL2," i.e., nectin-2, is a single-pass type I membrane protein with two Ig-like C2-type domains and one Ig-like V-type domain. It is a component of the plasma membrane of adherens junctions.

As used herein, the percentage of "identity," such as 85%, 90%, 91%, 92%, 93%, 94%, 95%, 96%, 97%, 98%, 98.5%, 99%, 99.5% identity, refers to the degree of similarity determined by sequence alignment between amino acid sequences or between nucleotide sequences, which is 85%, 90%, 91%, 92%, 93%, 94%, 95%, 96%, 97%, 98%, 98.5%, 99%, 99.5%. For example, after aligning two sequences to have identical residues at as many positions as possible by means such as introducing a gap, the percentage of identity is the determined proportion of the number of positions with identical bases or amino acid residues relative to the total number of positions. The percentage of "identity" can be determined using software programs known in the art. Preferably, alignment is performed using default parameters. One preferred alignment program is BLAST. Preferred programs include BLASTN and BLASTP. Details of these programs can be found at the following internet address: ncbi.nlm.nih.gov/cgi-bin/BLAST.

As used herein, a "variant" differs from a reference amino acid sequence by at least one amino acid, for example, by at least one amino acid addition, insertion, deletion, or substitution. For example, the amino acid substitution may be a conservative amino acid substitution, namely the substitution of an original corresponding amino acid with an amino acid having similar properties. "Conservative substitution" may refer to a polar-to-polar amino acid substitution, such as glycine (G, Gly), serine (S, Ser), threonine (T, Thr), tyrosine (Y, Tyr), cysteine (C, Cys), asparagine (N, Asn), and glutamine (Q, Gln); a nonpolar-to-nonpolar amino acid substitution, such as alanine (A, Ala), valine (V, Val), tryptophan (W, Trp), leucine (L, Leu), proline (P, Pro), methionine (M, Met), and phenylalanine (F, Phe); an acidic-to-acidic amino acid substitution, such as aspartic acid (D, Asp), glutamic acid (E, Glu); a basic-to-basic amino acid substitution, such as arginine (R, Arg), histidine (H, His), lysine (K, Lys); a charged amino acid-to-charged amino acid substitution, such as aspartic acid (D, Asp), glutamic acid (E, Glu), histidine (H, His), lysine (K, Lys), and arginine (R, Arg); a hydrophobic-to-hydrophobic amino acid substitution, such as alanine (A, Ala), leucine (L, Leu), isoleucine (I, Ile), valine (V, Val), proline (P, Pro), phenylalanine (F, Phe), tryptophan (W, Trp), and methionine (M, Met). In some other embodiments, the variant may also comprise non-conservative substitutions. In some embodiments, a "variant" of the amino acid sequence may have at least about 90%, 95%, 96%, 97%, 98%, or 99% sequence identity to the amino acid sequence. Compared with the amino acid sequence, a "variant" of the amino acid sequence may have an activity of at least 40%, 45%, 50%, 55%, 60%, 65%, 70%, 75%, 80%, 85%, 90%, 91%, 92%, 93%, 94%, 95%, 96%, 97%, 98%, 99%, or 100%, or a range constituted by any two of the preceding values. As used herein, a "conservative substitution variant" of a certain protein, polypeptide, or amino acid sequence refers to one in which one or more amino acid residues are subjected to amino acid substitution without altering the overall conformation and function of the protein or enzyme. This includes, but is not limited to, substitution of an amino acid in the amino acid sequence of a parent protein in the manner described in the preceding "conservative substitution". Accordingly, the similarity between two proteins or amino acid sequences with similar functions may vary. For example, a similarity (identity) ranges from 70% to 99% based on the MEGALIGN algorithm. "Conservative substitution variants" also encompasses polypeptides or enzymes determined by the BLAST or FASTA algorithm to have amino acid identity of 60% or more, preferably 75% or more, more preferably 85% or more, and most preferably 90% or more, and that have the same or substantially similar properties or functions as the natural or parent protein or enzyme.

As used herein, "amino acid" refers to any monomeric unit that can be incorporated into a peptide, polypeptide, or protein. As used herein, the term "amino acid" includes the following 20 natural or genetically encoded α-amino acids: alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V). In some embodiments, when referring to any of the above natural amino acids, the present application also encompasses non-natural amino acids or amino acid analogs derived or modified therefrom. As used herein, "amino acid" also includes non-natural amino acids, modified amino acids (e.g., having a modified side chain and/or backbone), and amino acid analogs. For further illustration, an amino acid is generally an organic acid comprising a substituted or unsubstituted amino group, a substituted or unsubstituted carboxyl group, and one or more side chains or groups, or an analog of any of these groups. Exemplary side chains include, for example, mercapto, seleno, sulfonyl, alkyl, aryl, acyl, keto, azido, hydroxyl, hydrazino, cyano, halo, hydrazide, alkenyl, alkynyl, ether, borate, boronate, phospho, phosphonyl, phosphine, heterocycle, enone, imine, aldehyde, ester, thioacid, hydroxylamine, or any combination of these groups. Other representative amino acids include, but are not limited to, amino acids comprising photo-sensitive crosslinkers, metal-binding amino acids, spin-labeled amino acids, fluorescent amino acids, metal-containing amino acids, amino acids with novel functional groups, amino acids that interact covalently or non-covalently with other molecules, photocaged and/or photo-isomerizable amino acids, radioactive amino acids, amino acids comprising biotin or biotin analogs, glycosylated amino acids, other carbohydrate-modified amino acids, amino acids comprising polyethylene glycol or polyether, heavy atom-substituted amino acids, chemically cleavable and/or photo-cleavable amino acids, amino acids comprising carbon-linked sugars, redox-active amino acids, amino acids comprising aminothioic acids, and amino acids comprising one or more toxic moieties. Amino acids described in the present application include, but are not limited to: 20 natural amino acids and 2-aminoadipic acid (Aad), 3-aminoadipic acid (bAad), beta-alanine or beta-aminopropanoic acid (bAla), 2-aminobutyric acid (Abu), 4-aminobutyric acid or piperidic acid (4Abu), 6-aminocaproic acid (Acp), 2-aminoheptanoic acid (Ahe), 2-aminoisobutyric acid (Aib), 3-aminoisobutyric acid (bAib), 2-aminopimelic acid (Apm), 2,4-diaminobutyric acid (Dbu), methamphetamine (Des), 2,2'-diaminopimelic acid (Dpm), 2,3-diaminopropionic acid (Dpr), ethylglycine (EtGly), N-ethylasparagine (EtAsn), hydroxylysine (Hyl), allo-hydroxylysine (aHyl), 3-hydroxyproline (3Hyp), 4-hydroxyproline (4Hyp), isodesmosine (Ide), allo-isoleucine (aIle), N-methylglycine or sarcosine (MeGly), N-methylisoleucine (MeIle), 6-N-methyllysine (MeLys), N-methylvaline (MeVal), norvaline (Nva), norleucine (Nle), and ornithine (Orm). Thus, in some embodiments, after the mutation, the amino acid mutation at the position comprises a substitution mutation to any one of the above 20 natural amino acids and the above non-natural amino acids. In some embodiments, the amino acid mutation comprises a substitution mutation to any amino acid selected from the group consisting of: G, A, V, L, I, P, F, Y, W, S, T, C, M, N, Q, D, E, K, R, H, Aad, bAad, bAla, Abu, 4Abu, Acp, Ahe, Aib, bAib, Apm, Dbu, Des, Dpm, Dpr, EtGly, EtAsn, Hyl, aHyl, 3Hyp, 4Hyp, Ide, aIle, MeGly, Melle, MeLys, MeVal, Nva, Nle, and Orm.

In the context of the present application, the terms "DNA" and "RNA" refer to single-stranded or double-stranded DNA or RNA molecules. Unless otherwise indicated, the terms "DNA" and "DNA molecule" refer to double-stranded DNA molecules composed of A, C, G, and/or T nucleotides, while the terms "RNA" and "RNA molecule" refer to single-stranded RNA molecules composed of A, C, G, and/or U nucleotides. As used herein, the A, C, G, T, and U nucleotides refer to nucleotides comprising adenine, guanine, cytosine, thymine, and uracil as their respective nitrogenous bases.

RNA molecules include coding RNA or non-coding RNA (ncRNA), such as pre-mRNA, mature mRNA, or long noncoding RNA (lncRNA).

As used herein, a "DNA-RNA hybrid molecule" refers to a molecule comprising a polynucleotide sequence composed of deoxyribonucleotides and ribonucleotides. The DNA-RNA hybrid molecule may be obtained by:
replacing one or more deoxyribonucleotides in DNA with ribonucleotides;
replacing one or more ribonucleotides in RNA with deoxyribonucleotides; or
de novo synthesis using deoxyribonucleotides and ribonucleotides as starting materials via biological or chemical synthesis. It should be noted that the manner of obtaining the DNA-RNA hybrid molecule is not limited to the above methods, and any DNA-RNA hybrid molecule obtained by any method falls within the scope of the "DNA-RNA hybrid molecule" as defined in the present application.

As used herein, if two nucleic acid molecules are described as having "identical genetic information," it means that the two nucleic acid molecules are complementary to each other, or comprise identical base sequences, or that after converting one or more thymines in the base sequence of one of the two nucleic acid molecules to uracils, a nucleic acid molecule having a base sequence identical to that of the other nucleic acid molecule can be obtained. Accordingly, any two of DNA, RNA, and DNA-RNA hybrid molecules may have identical genetic information. Herein, the term "base sequence" refers to the order of bases in a polynucleotide molecule. Those skilled in the art should understand that, unless otherwise indicated, a base sequence or polynucleotide sequence described in the present application, when used to describe a DNA sequence, may use "T" to represent thymine; however, when the base sequence or polynucleotide sequence is used to describe RNA (e.g., mRNA), "T" will be substituted by "U" (uracil). Thus, any DNA disclosed herein by a specific sequence identification number (SEQ ID NO) also discloses the RNA (e.g., mRNA or poly(A) tail) sequence that is complementary or corresponding to the DNA, wherein each "T" in the DNA sequence is replaced by "U."

As used herein, "encoding" means that i) a DNA sequence contains genetic information that can be transcribed into an RNA molecule, and/or ii) an RNA molecule contains genetic information that can be translated into an amino acid sequence. Thus, as used herein, a "coding sequence" may refer to a ribonucleotide (RNA) sequence in a pre-mRNA or mature mRNA that can be translated into a protein or a fragment thereof, or may refer to a complementary sequence of a deoxyribonucleic acid (DNA) sequence that serves as a template for transcribing the pre-mRNA or mature mRNA or a fragment thereof. In addition, the "coding sequence" of the present application may further comprise a polynucleotide sequence encoding a protein, a functional nucleic acid, or a fragment thereof, such as miRNA, shRNA, dsRNA, guide RNA, Poly(A) tail, 5'UTR, 3'UTR, etc. Herein, a DNA molecule containing genetic information that can be transcribed into an RNA molecule is referred to as a "coding nucleic acid" of the RNA molecule; an RNA molecule containing genetic information that can be translated into an amino acid sequence is referred to as a "coding nucleic acid" of the amino acid sequence.

Unless otherwise specified, "peptide," "polypeptide," and "protein" are used interchangeably in the present application and refer to any naturally active substance formed by covalent linkage of two or more amino acids via peptide bonds, which may or may not contain secondary or tertiary structures of a protein molecule.

As used herein, the term "contact" is used in its plain ordinary meaning and refers to a process of allowing at least two different substances to become sufficiently close to react, interact, or make physical contact. It is to be understood that the resulting reaction product may be generated directly from the reaction between the added reagents or from an intermediate of one or more added reagents, which intermediate may be produced in the reaction mixture. The term "contact" may include allowing two substances to react, interact or make physical contact, wherein the two substances may be, for example, an engineered receptor (or engineered nucleic acid molecule) and a cell as provided herein. In embodiments, contacting includes, for example, allowing the engineered nucleic acid molecule or engineered peptide in the present application to enter cells.

As used herein, *"in vivo* persistence period of CAR-T cells" refers to the duration during which CAR-T cells persist in a patient or subject, i.e., the period from administration of the CAR-T cells *in vivo* until their eventual disappearance from the body. It can be calculated or measured by comparing copy number of CARs. For example, after a specified period of *in vivo* use of the CAR-T, an increase in the copy number thereof can be used to indicate a prolonged *in vivo* persistence period of the CAR-T cells.

Unless otherwise defined herein, all technical and scientific terms used herein have the same meanings as commonly understood by those ordinarily skilled in the art to which the present application pertains.

### TIGIT Extracellular Region Polypeptide

In one aspect, the present application relates to a TIGIT extracellular region polypeptide (also referred to as a TIGIT mutant peptide unless otherwise indicated), which comprises a mutation at position 48 relative to a reference sequence, wherein the reference sequence is an amino acid sequence as set forth in SEQ ID NO:1, and wherein amino acid position numbering is based on the reference sequence.

In some embodiments, the TIGIT extracellular region polypeptide comprises amino acids corresponding to positions 33 to 93 of the reference sequence, i.e., it comprises amino acids corresponding to positions 33 and 93 of the reference sequence in any TIGIT extracellular region amino acid sequence, and all amino acids between positions 33 and 93 in the any TIGIT extracellular region amino acid sequence. It should be understood that it does not require that all amino acids between positions 33 and 93 correspond one-to-one to all amino acids of positions 33 to 93 of the reference sequence, but rather allows for deletion or addition of amino acids at any of the amino acid positions corresponding to positions 33 to 93 of the reference sequence. Furthermore, it is to be understood that when the amino acid sequence of the any TIGIT extracellular region does not have an amino acid corresponding to position 33 of the reference sequence, the amino acids corresponding to positions 33 to 93 of the reference sequence start from the amino acid position greater than position 33 of the reference sequence and closest to position 33 of the reference sequence; and when the amino acid sequence of the any TIGIT extracellular region does not have an amino acid corresponding to position 93 of the reference sequence, the amino acids corresponding to positions 33 to 93 of the reference sequence end at an amino acid position less than position 93 of the reference sequence and closest to position 93 of the reference sequence. In some embodiments, the engineered TIGIT extracellular region polypeptide may further comprise one or more contiguous amino acid sequences extending toward C-terminus from position 33 of the any TIGIT extracellular region amino acid sequence, and/or one or more contiguous amino acid sequences extending toward N-terminus from position 93 of the any TIGIT extracellular region amino acid sequence. In some embodiments, the engineered TIGIT extracellular region polypeptide may further comprise one or more contiguous amino acid sequences extending toward the N-terminus from position 33 of the any TIGIT extracellular region amino acid sequence, and/or one or more contiguous amino acid sequences extending toward the C-terminus from position 93 of the any TIGIT extracellular region amino acid sequence. In some embodiments, the TIGIT extracellular region polypeptide only comprises amino acids corresponding to positions 33 to 93 of the reference sequence.

In some embodiments, the mutation at position 48 comprised in the TIGIT extracellular region polypeptide relative to the reference sequence is any one selected from the group consisting of:
C48G, C48A, C48V, C48L, C48I, C48P, C48F, C48W, C48M, C48Y, C48S, C48T, C48N, C48Q, C48D, C48E, C48K, C48R, C48H, and deletion at position 48. Herein, the deletion at position 48 means that the TIGIT extracellular region polypeptide has no amino acid at position 48 relative to the reference sequence. In some embodiments, the TIGIT extracellular region polypeptide comprises a non-natural amino acid substitution at position 48. Thus, in some embodiments, position 48 in the TIGIT extracellular region relative to the reference sequence is Aad, bAad, bAla, Abu, 4Abu, Acp, Ahe, Aib, bAib, Apm, Dbu, Des, Dpm, Dpr, EtGly, EtAsn, Hyl, aHyl, 3Hyp, 4Hyp, Ide, aIle, MeGly, MeIle, MeLys, MeVal, Nva, Nle, or Orn.

In some embodiments, the TIGIT extracellular region polypeptide comprises: amino acids corresponding to positions 33 to 93 of the reference sequence, the mutation at position 48 relative to the reference sequence as described above, and one or more amino acid positions selected from the group consisting of:
2M, 2W, 2T, 9T, 9S, 12I, 12N, 14A, 14V, 20I, 20T, 21I, 21F, 22L, 22F, 34T, 34S, 37N, 37D, 37E, 39E, 39G, 39K, 39V, 42D, 42G, 44L, 44F, 61K, 61R, 70L, 70Q, 70P, 71G, 71D, 76S, 76P, 79V, 79E, 80N, 80Y, 86F, 86S, 86L, 101I, 101T, 102S, 102F, 106L, 106Q, 110V, 110Y, 110E, 110A, 113H, and 113Y.

In some embodiments, the TIGIT extracellular region polypeptide comprises: amino acids corresponding to positions 33 to 93 of the reference sequence, the mutation at position 48 relative to the reference sequence as described above, and the following amino acid positions:
1) 22F, 37D, 39G;
2) 37D;
3) 20T, 37D, 39K, 44F;
4) 22L, 37E, 71D, 102S;
5) 20T, 37D, 42G, 70Q;
6) 21F, 34S, 37E, 39K, 70P, 80Y, 101T, 110A;
7) 9S, 37D, 39V, 61R, 101T;
8) 20T, 37D, 39K;
9) 37D, 86S, 113Y;
10) 2W, 37D, 56F;
11) 12N, 14V, 21F, 37D, 39K, 70Q; or
12) 2T, 37D, 39K, 86L.

In some embodiments, any letter referring to a natural amino acid described above may also be used to refer to a non-natural amino acid or amino acid analog formed by modification or derivation of the natural amino acid.

In some embodiments, the TIGIT extracellular region polypeptide comprises any amino acid sequence selected from the following or a conservative substitution variant of any amino acid sequence selected from the following:
SEQ ID NOs:5-17, 19, and 22, and SEQ ID NOs:24-26.

In some embodiments, the amino acid sequence of the TIGIT extracellular region polypeptide is any amino acid sequence selected from the following or a conservative substitution variant of any amino acid sequence selected from the following:
SEQ ID NOs:5-17, 19, and 22, and SEQ ID NOs:24-26.

In some embodiments, the TIGIT extracellular region polypeptide comprises a mutation relative to the reference sequence at one or more positions selected from the following:
position 9, position 20, position 21, position 34, position 39, position 48, position 57, position 61, position 70, position 71, position 80, position 86, position 88, position 101, and position 110.

In some embodiments, the TIGIT extracellular region polypeptide comprises mutations relative to the reference sequence at the following positions:
1) position 48, position 57, and position 86; or
2) position 48, position 71, and position 88.

In some embodiments:
the mutation at position 48 is any one selected from the group consisting of:
C48G, C48A, C48V, C48L, C48I, C48P, C48F, C48W, C48M, C48Y, C48S, C48T, C48N, C48Q, C48D, C48E, C48K, C48R, C48H, and deletion at position 48;
the mutation at position 57 is any one selected from the group consisting of: S57G, S57A, S57V, S57L, S57I, S57P, S57F, S57W, S57M, S57Y, S57C, S57T, S57N, S57Q, S57D, S57E, S57K, S57R, S57H, and deletion at position 57;
the mutation at position 71 is any one selected from the group consisting of: G71S, G71A, G71V, G71L, G71I, G71P, G71F, G71W, G71M, G71Y, G71C, G71T, G71N, G71Q, G71D, G71E, G71K, G71R, G71H, and deletion at position 71;
the mutation at position 86 is any one selected from the group consisting of: F86G, F86A, F86V, F86L, F86I, F86P, F86S, F86W, F86M, F86Y, F86C, F86T, F86N, F86Q, F86D, F86E, F86K, F86R, F86H, and deletion at position 86; and/or
the mutation at position 88 is any one selected from the group consisting of: I88G, 188A, 188V, 188L, I88S, 188P, 188F, 188W, I88M, I88Y, I88C, I88T, I88N, I88Q, I88D, I88E, I88K, I88R, I88H, and deletion at position 88.

Herein, the deletions at positions 48, 57, 71, 86, and 88 mean that the TIGIT extracellular region polypeptide has no amino acid at positions 48, 57, 71, 86, and 88 relative to the reference sequence, respectively. In some embodiments, the TIGIT extracellular region polypeptide comprises a non-natural amino acid substitution at one or more of positions 9, 20, 21, 34, 39, 48, 57, 61, 70, 71, 80, 86, 88, 101, and 110. Thus, in some embodiments, one or more of positions 9, 20, 21, 34, 39, 48, 57, 61, 70, 71, 80, 86, 88, 101, and 110 in the TIGIT extracellular region relative to the reference sequence are Aad, bAad, bAla, Abu, 4Abu, Acp, Ahe, Aib, bAib, Apm, Dbu, Des, Dpm, Dpr, EtGly, EtAsn, Hyl, aHyl, 3Hyp, 4Hyp, Ide, aIle, MeGly, MeIle, MeLys, MeVal, Nva, Nle, or Orn.

In some embodiments, the TIGIT extracellular region polypeptide comprises at least the following mutations relative to the reference sequence:
1) C48W, S57P, and F86S; or
2) C48W, G71D, and I88V.

In some embodiments, the TIGIT extracellular region polypeptide comprises the following amino acid positions:
1) 34T, 39E, 48W, 57P, 61K, 70L, 71G, 80N, 86S, and 88I;
2) 34T, 39E, 48W, 57S, 61K, 70L, 71D, 80N, 86F, and 88V;
3) 9T, 20I, 211, 34T, 39E, 48W, 57P, 61K, 70L, 71G, 80N, 86S, 88I, 101I, and 110V; or
4) 9T, 20I, 21I, 34T, 39E, 48W, 57S, 61K, 70L, 71D, 80N, 86F, 88V, 101I, and 110V.

In some embodiments, any letter referring to a natural amino acid described above may also be used to refer to a non-natural amino acid or amino acid analog formed by modification or derivation of the natural amino acid.

In some embodiments, the TIGIT extracellular region polypeptide comprises any amino acid sequence selected from the following or a conservative substitution variant of any amino acid sequence selected from the following:
SEQ ID NOs:47-53.

In some embodiments, the amino acid sequence of the TIGIT extracellular region polypeptide is any amino acid sequence selected from the following or comprises a conservative substitution variant of any amino acid sequence selected from the following:
SEQ ID NOs:47-53.

In some embodiments, the TIGIT extracellular region polypeptide is chemically modified, for example, one or more amino acids thereof are substituted with non-natural amino acids or amino acid analogs.

### Fusion Protein

In one aspect, the present application further provides a fusion protein comprising the aforementioned TIGIT extracellular region polypeptide, wherein the fusion protein may be a protein complex formed by combining the above TIGIT extracellular region polypeptide with one or more additional proteins, polypeptides or protein functional domains in any manner. In some embodiments, the combining is such that the TIGIT extracellular region polypeptide is linked to the one or more additional proteins, polypeptides or protein functional domains directly and/or indirectly. In some embodiments, the C-terminus of the TIGIT extracellular region polypeptide in the fusion protein is linked to the N-terminus of the additional protein, polypeptide or protein functional domain. In some embodiments, the N-terminus of the TIGIT extracellular region polypeptide in the fusion protein is linked to the C-terminus of the additional protein, polypeptide or protein functional domain. In some embodiments, the TIGIT extracellular region polypeptide and the multiple proteins, polypeptides, or protein functional domains in the fusion protein are linked in tandem with each other. In some embodiments, the C-terminus and/or N-terminus of the TIGIT extracellular region polypeptide in the fusion protein is linked to at least two or more additional proteins, polypeptides, or protein functional domains, and the two or more additional proteins, polypeptides, or protein functional domains are not linked in tandem. In some embodiments, the one or more proteins are a homologous protein of the TIGIT extracellular region polypeptide or a part of the homologous protein. In some embodiments, the one or more proteins are heterologous proteins of the TIGIT extracellular region polypeptide, or parts of the heterologous proteins. The term "direct" linkage or "directly linked" refers to linkage achieved only by chemical bonds, i.e., two proteins or polypeptides are not linked via other molecules. The chemical bonds may be non-covalent bonds (e.g., ionic bonds, hydrogen bonds, hydrophobic bonds or van der Waals bonds) or covalent bonds (e.g., peptide bonds). The term "indirect" linkage or "indirectly linked" refers to linkage via a linker, wherein two proteins or polypeptides linked to each other using a linker are respectively linked to one end of the linker via covalent or non-covalent bonds. The "linker" may be a peptide linker (i.e., a peptide chain, e.g., consisting of 1 to 50 amino acids or derivatives thereof) or a non-peptide linker. Moreover, the linker may be cleavable (i.e., capable of being hydrolyzed by enzymes in an organism, e.g., a mammalian organism) or non-cleavable. Exemplary non-peptide linkers include, but are not limited to, polyethylene glycol, polypropylene glycol, copolymers of ethylene glycol and propylene glycol, polyoxyethylene polyols, polyvinyl alcohol, polysaccharides, dextrans, polyvinyl ethers, biodegradable polymers, polymeric lipids, chitin and hyaluronic acid, or derivatives thereof, or combinations thereof.

In some embodiments, the one or more additional proteins, polypeptides or protein functional domains comprise a recognition polypeptide that specifically binds to one or more other proteins, and the one or more other proteins may be tumor antigens, for example, one or more selected from the group consisting of: prostate stem cell antigen (PSCA), carcinoembryonic antigen (CEA), CD123, thyroid stimulating hormone receptor (TSHR); CD171; CS-1; C-type lectin-like molecule-1; ganglioside GD3; Tn antigen; CD19; CD20; CD22; CD30; CD70; CD123; CD138; CD33; CD44; CD44v7/8; CD38; CD44v6; B7H3 (CD276), B7H6; KIT (CD117); interleukin 13 receptor subunit alpha (IL-13Rα); interleukin 11 receptor alpha (IL-11Rα); prostate specific membrane antigen (PSMA); NY-ESO-1; HIV-1 Gag; MART-1; gp100; tyrosinase; mesothelin; EpCAM; protease serine 21 (PRSS21); vascular endothelial growth factor receptor; Lewis(Y) antigen; CD24; platelet derived growth factor receptor beta (PDGFR-β); stage-specific embryonic antigen-4 (SSEA-4); cell surface associated mucin 1 (MUC1), MUC6; epidermal growth factor receptor family and mutants thereof (EGFR, EGFR2, ERBB3, ERBB4, EGFRvIII); neural cell adhesion molecule (NCAM); carbonic anhydrase IX (CAIX); LMP2; ephrin type-A receptor 2 (EphA2); fucosyl GM1; sialyl Lewis adhesion molecule (sLe); ganglioside GM3 (aNeu5Ac(2-3)bDGalp(1-4)bDGlcp(1-1)Cer); TGS5; high molecular weight melanoma-associated antigen (HMWMAA); O-acetyl-GD2 ganglioside (OAcGD2); folate receptor; tumor endothelial marker 1 (TEM1/CD248); tumor endothelial marker 7-related (TEM7R); Claudin 6, Claudin 18.2, Claudin 18.1; ASGPR1; CDH16; 5T4; 8H9; αvβ6 integrin; B cell maturation antigen (BCMA); CA9; kappa light chain; CSPG4; EGP2, EGP40; FAP; FAR; FBP; embryonic AchR; HLA-A1, HLA-A2; MAGEA1, MAGE3; KDR; MCSP; NKG2D ligand; PSC1; ROR1; Sp17; SURVIVIN; TAG72; TEM1; fibronectin; tenascin; carcinoembryonic variant of tumor necrosis region; G protein-coupled receptor class C group 5 member D (GPRC5D); chromosome X open reading frame 61 (CXORF61); CD97; CD179a; anaplastic lymphoma kinase (ALK); polysialic acid; placenta-specific 1 (PLAC1); hexasaccharide of globoH glycoceramide (GloboH); mammary gland differentiation antigen (NY-BR-1); uroplakin 2 (UPK2); hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex locus K9 (LY6K); olfactory receptor 51E2 (OR51E2); TCR gamma alternate reading frame protein (TARP); Wilms tumor protein (WT1); ETS translocation-variant gene 6 (ETV6-AML); sperm protein 17 (SPA17); X antigen family member 1A (XAGE1); angiopoietin-binding cell surface receptor 2 (Tie2); melanoma cancer testis antigen-1 (MAD-CT-1); melanoma cancer testis antigen-2 (MAD-CT-2); Fos-related antigen 1; p53 mutant; human telomerase reverse transcriptase (hTERT); sarcoma translocation breakpoints; melanoma inhibitor of apoptosis (ML-IAP); ERG (transmembrane protease serine 2 (TMPRSS2) ETS fusion gene); N-acetyl glucosaminyl-transferase V (NA17); paired box protein Pax-3 (PAX3); androgen receptor; cyclin B1; V-myc avian myelocytomatosis viral oncogene neuroblastoma derived homolog (MYCN); Ras homolog family member C (RhoC); cytochrome P450 1B1 (CYP1B1); CCCTC-binding factor (zinc finger protein)-like (BORIS); squamous cell carcinoma antigen recognized by T cells 3 (SART3); paired box protein Pax-5 (PAX5); proacrosin binding protein sp32 (OYTES1); lymphocyte-specific protein tyrosine kinase (LCK); A kinase anchor protein 4 (AKAP-4); synovial sarcoma, X breakpoint 2 (SSX2); CD79a; CD79b; CD72; leukocyte-associated immunoglobulin-like receptor 1 (LAIR1); Fc fragment of IgA receptor (FCAR); leukocyte immunoglobulin-like receptor subfamily member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); glypican-3 (GPC3); Fc receptor-like 5 (FCRL5); immunoglobulin lambda-like polypeptide 1 (IGLL1), and CD155.

In some embodiments, the one or more additional proteins, polypeptides or protein functional domains comprise a recognition polypeptide that specifically binds to one or more other proteins, wherein the one or more other proteins may be immune checkpoint proteins, for example, one or more selected from the group consisting of:
2B4, 4-1BB, 4-1BB ligand, B7-1, B7-2, B7H2, B7H3, B7H4, B7H6, BTLA, CD155, CD160, CD19, CD200, CD27, CD27 ligand, CD28, CD40, CD40 ligand, CD47, CD48, CTLA-4, DNAM-1, Galectin-9, GITR, GITR ligand, HVEM, ICOS, ICOS ligand, IDO1, KIR, 3DL3, LAG-3, OX40, OX40 ligand, PD-L1, PD-1, PD-L2, LAG3, PGK, SIRPα, TIM-3, TIGIT, and VSIG8.

In some embodiments, the one or more additional proteins, polypeptides or protein functional domains comprise a recognition polypeptide that specifically binds to one or more other proteins, wherein the one or more other proteins include any one or more immune checkpoint proteins mentioned above, and any one or more tumor antigens mentioned above.

In some embodiments, the recognition polypeptide that specifically binds to one or more other proteins is an antibody or an antigen-binding fragment of the antibody, or a ligand or receptor that binds to the tumor antigen and/or immune checkpoint protein, or a fragment thereof.

In some embodiments, the one or more additional proteins, polypeptides or protein functional domains may be a hinge structure linking the TIGIT extracellular region polypeptide and a transmembrane structure, for example, one or more selected from the group consisting of: a hinge region of TIGIT, CD7, IgG, IgD, CD8α, or CD28, or a combination thereof. In some embodiments, the one or more additional proteins, polypeptides or protein functional domains may be a transmembrane structure linking the TIGIT extracellular region polypeptide and an intracellular signaling domain, for example, one or more selected from the group consisting of: ICOS, CD4, CD8α, CD28, CD3ζ, and TIGIT. In some embodiments, the one or more additional proteins, polypeptides or protein functional domains may further comprise an intracellular signaling domain, wherein the intracellular signaling domain is a costimulatory signaling molecule, comprising a signal transduction domain of one or more molecules selected from the group consisting of:
CD27, CD28, 4-1BB, OX40, CD30, CD40, CD2, LFA-1, LIGHT, NKG2C, B7-H3, PD-1, ICOS, CDS, ICAM-1, GITR, BAFFR, LIGHTR, SLAMF7, CD7, NKp80 (KLRF1), CD160, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1, SLAMF4, CD84, CD96, CEACAM1, CRTAM, CD229, CD160, PSGL1, CD100, CD69, SLAMF6, SLAMF1, SLAMF8, CD162, LTBR, LAT, GADS, SLP-76, PAG/Cbp, NKp44, NKp30, NKp46, NKG2D, and a ligand that specifically binds to CD83.

Further, in some embodiments, the fusion protein may be a TIGIT fusion protein comprising the TIGIT extracellular region polypeptide, a transmembrane structure, and an intracellular costimulatory domain.

### Engineered Receptor

The present application further provides an engineered receptor comprising an antigen binding domain, wherein the antigen binding domain comprises the aforementioned engineered TIGIT extracellular region polypeptide or the aforementioned fusion protein. In some embodiments, the engineered receptor further comprises a signal transduction domain. In some embodiments, the signal transduction domain comprises a primary signal transduction domain. In some embodiments, the signal transduction domain comprises a costimulatory domain or a secondary signal transduction domain, but does not comprise a primary signal transduction domain. In some embodiments, the signal transduction domain comprises a primary signal transduction domain and a costimulatory domain.

In some embodiments, the engineered receptor comprises or consists of, from N-terminus to C-terminus: an antigen binding domain-a transmembrane domain-a costimulatory domain (or a secondary signal transduction domain). In some embodiments, the engineered receptor comprises or does not comprise a hinge region, wherein the hinge region links the antigen binding domain and the transmembrane domain or the signal transduction domain. In some embodiments, the hinge is derived from 7h (CD7 hinge region)/G4h/8h (CD8 hinge region). In some embodiments, the transmembrane domain is derived from a transmembrane domain of ICOS, CD4, CD8α, CD28, CD3ζ, or TIGIT, or a combination thereof. In some embodiments, the costimulatory domain or secondary signal transduction domain comprises one or more of the following, or consists of one or more of the following: CD27, CD28, 4-1BB, OX40, CD30, CD40, CD2, LFA-1, LIGHT, NKG2C, B7-H3, PD-1, ICOS, CDS, ICAM-1, GITR, BAFFR, LIGHTR, SLAMF7, CD7, NKp80 (KLRF1), CD160, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1, SLAMF4, CD84, CD96, CEACAM1, CRTAM, CD229, CD160, PSGL1, CD100, CD69, SLAMF6, SLAMF1, SLAMF8, CD162, LTBR, LAT, GADS, SLP-76, PAG/Cbp, NKp44, NKp30, NKp46, NKG2D, and a signal transduction domain of a ligand that specifically binds to CD83.

In some embodiments, the engineered receptor may comprise an extracellular antigen binding domain, with or without an additional hinge structure, a transmembrane domain, and an intracellular signal transduction domain, wherein the extracellular antigen binding domain is any amino acid sequence selected from the following or comprises a conservative substitution variant of any amino acid sequence selected from the following: SEQ ID NOs:5-17, 19, and 22, SEQ ID NOs:24-26, and SEQ ID NOs:47-53. Specifically, in some embodiments, the structure of the engineered receptor is TIGIT extracellular region polypeptide-28TM-28. In some embodiments, the TIGIT extracellular region polypeptide is the aforementioned engineered TIGIT extracellular region polypeptide. In some embodiments, the TIGIT extracellular region polypeptide may be any amino acid sequence selected from the following or comprises a conservative substitution variant of any amino acid sequence selected from the following: SEQ ID NOs:5-17, 19, and 22, SEQ ID NOs:24-26, and SEQ ID NOs:47-53; 28TM is a transmembrane domain derived from human CD28 (e.g., the amino acid sequence set forth in SEQ ID NO:36); and 28 is an intracellular signal transduction domain derived from human CD28 (e.g., the amino acid sequence set forth in SEQ ID NO:37). In some embodiments, the structure of the engineered receptor is TIGIT extracellular region polypeptide-28TM-28, wherein the TIGIT extracellular region polypeptide may be the polypeptide set forth in SEQ ID NOs:7, 48, and 51-53, 28TM is a transmembrane domain derived from human CD28, and 28 is an intracellular signal transduction domain derived from the human CD28. In some embodiments, the structure of the engineered receptor is TIGIT extracellular region polypeptide-28TM-28z, wherein the TIGIT extracellular region polypeptide may be any amino acid sequence selected from the following or comprises a conservative substitution variant of any amino acid sequence selected from the following: SEQ ID NOs:5-17, 19, and 22, SEQ ID NOs:24-26, and SEQ ID NOs:47-53; 28TM is a transmembrane domain derived from human CD28 (e.g., the amino acid sequence set forth in SEQ ID NO:36); 28 is a signal transduction domain derived from the human CD28 (e.g., the amino acid sequence set forth in SEQ ID NO:37), and z is a signal transduction domain derived from human CD3ζ (e.g., the amino acid sequence set forth in any one of SEQ ID NOs:41-43).

In some embodiments, the engineered receptor is: a chimeric antigen receptor (CAR), a T cell receptor (TCR), a T cell antigen coupler (TAC), or the aforementioned fusion protein. In some embodiments, the fusion protein comprises an extracellular antigen binding domain and an intracellular domain, wherein the extracellular antigen binding domain comprises the aforementioned engineered TIGIT extracellular region polypeptide, and the intracellular domain is selected from intracellular domains of any one or more membrane proteins. In some embodiments, the fusion protein comprises an extracellular antigen binding domain and an intracellular domain, wherein the extracellular antigen binding domain comprises the aforementioned engineered TIGIT extracellular region polypeptide, and the intracellular domain comprises a T cell receptor costimulatory domain. In some embodiments, the fusion protein comprises an extracellular antigen binding domain and an intracellular domain, wherein the extracellular antigen binding domain comprises the aforementioned engineered TIGIT extracellular region polypeptide, and the intracellular domain comprises one or more T cell receptor costimulatory domains, wherein the T cell receptor costimulatory domain is derived from intracellular domains of the following proteins: CARD11, CD2, CD4, CD7, CD19, CD27, CD28, CD30, CD40, CD160, ICAM-1, OX40, 4-1BB, SELPLG, LIGHT, HVEM, B7-H3, ICOS, PD-1, SLAMF7, LFA-1, NKG2C, CDS, GITR, BAFFR, NKp80, IPO-3, SLAMF8, LTBR, LAT, GADS, SLP-76, PAG/Cbp, NKp44, NKp30, NKp46, NKG2D, CD83, SLAMF1, CTLA-4, LAG-3, PD-L2, PD-L1, DAP10, TRIM, ZAP70, and a ligand that specifically binds to CD83.

As used herein, the term "CAR," i.e., chimeric antigen receptor, comprises: i) an antigen binding domain that specifically recognizes one or more target antigens (e.g., tumor antigens) or target epitopes (e.g., tumor epitopes); ii) a transmembrane domain; and iii) an intracellular signal transduction domain. Herein, the "intracellular signal transduction domain" comprises a primary signal transduction domain and/or a costimulatory domain. In some embodiments, the antigen binding domain is one or more selected from the group consisting of: an extracellular antigen binding domain of a receptor or ligand, single-domain antibody (sdAb), single-chain Fv (scFv), and Fab. In some embodiments, the transmembrane domain is derived from any one molecule selected from the group consisting of: TCRα, TCRβ, TCRγ, TCRδ, CD3ζ, CD3ε, CD3γ, CD3δ, CD4, CD5, CD6, CD7, CD8α, CD9, CD16, CD22, CD27, CD28, CD33, CD37, CD45, CD47, CD52, CD64, CD80, CD86, CD134, 4-1BB, CD152, CD154, CISH, and PD-1. In some embodiments, the transmembrane domain is derived from CD8α. In some embodiments, the intracellular signal transduction domain comprises a primary signal transduction domain derived from any one molecule selected from the group consisting of: CD3ζ, CD3γ, CD3ε, CD3δ, FcRγ, FcRβ, CD5, CD22, CD79a, CD79b, CD66d, FcγRIIa, DAP10, and DAP12. In some embodiments, the primary signal transduction domain is derived from CD3ζ. As used herein, the "primary signal transduction domain" typically comprises immune-receptor tyrosine-based activation motifs (ITAMs), with a basic composition of YXXL/V, wherein Y is tyrosine, L/V refers to leucine or valine, and X can be any amino acid. Upon binding of the receptor to a corresponding ligand, tyrosine within the ITAM linked thereto can be phosphorylated under the action of a class of protein tyrosine kinases (PTKs) associated with the cell membrane, thereby recruiting other free intracellular protein kinases or adaptor proteins, and propagating activation signals into cells.

In some embodiments, the intracellular signal transduction domain further comprises a costimulatory domain derived from one or more costimulatory receptor molecules selected from the group consisting of: CARD11, CD2, CD4, CD7, CD19, CD27, CD28, CD30, CD40, CD160, ICAM-1, OX40, 4-1BB, SELPLG, LIGHT, HVEM, B7-H3, ICOS, PD-1, SLAMF7, LFA-1, NKG2C, CDS, GITR, BAFFR, NKp80, IPO-3, SLAMF8, LTBR, LAT, GADS, SLP-76, PAG/Cbp, NKp44, NKp30, NKp46, NKG2D, CD83, SLAMF1, CTLA-4, LAG-3, PD-L2, PD-L1, DAP10, TRIM, ZAP70, a ligand that specifically binds to CD83, and any combination thereof. In some embodiments, the costimulatory domain is derived from 4-1BB. In some embodiments, the CAR further comprises a hinge domain between the C-terminus of the antigen binding domain and the N-terminus of the transmembrane domain. In some embodiments, the hinge domain is derived from CD8α or CD28. In some embodiments, the antigen binding domain comprises the aforementioned engineered TIGIT extracellular region polypeptide. In some embodiments, the antigen binding domain is the aforementioned engineered TIGIT extracellular region polypeptide. As used herein, the "costimulatory domain" is typically derived from a costimulatory receptor of immune cells, providing a second signal or secondary intracellular signal for activating immune cells (e.g., T cells).

As used herein, the engineered "TCR," i.e., engineered T cell receptor, comprises: (a) an antigen binding domain (as used in the present application, the antigen binding domain comprises a domain that binds to an antigen, a ligand domain that binds to a receptor, or a receptor domain that binds to a ligand), which comprises an antigen binding fragment (e.g., sdAb, scFv, Fab, DARPin) that specifically recognizes one or more target antigens (e.g., tumor antigens) or target epitopes (e.g., tumor epitopes); (b) an optional first linker; (c) an optional extracellular antigen binding domain of a first TCR subunit (e.g., Cα, Cβ, Cδ, Cγ, CD3ε) or a part thereof; (d) a transmembrane domain of a second TCR subunit (e.g., TCRα, TCRβ); and (e) an intracellular signal transduction domain comprising a third TCR subunit (e.g., TCRα, TCRβ), wherein the first, second, and third TCR subunits are each independently any one selected from the group consisting of: TCRα, TCRβ, TCRγ, TCRδ, CD3ε, CD3γ, CD3δ, and CD3ζ. In some embodiments, the first, second, and third TCR subunits are the same (e.g., all CD3ε, all TCRα or all TCRβ). In some embodiments, the first, second, and third TCR subunits are different. In some embodiments, the engineered TCR further comprises a hinge domain located between C-terminus of the antigen binding domain and N-terminus of the transmembrane domain. In some embodiments, the hinge domain is derived from CD8α. In some embodiments, the antigen binding domain comprises the aforementioned engineered TIGIT extracellular region polypeptide. In some embodiments, the antigen binding domain is the aforementioned engineered TIGIT extracellular region polypeptide.

As used herein, "TAC," i.e., T cell antigen coupler, comprises (i) an antigen binding domain, (ii) a TCR binding domain (e.g., scFv), and (iii) a co-receptor domain (e.g., hinge, transmembrane and/or cytosolic region). See, e.g., Helsen et al. Nat Commun. 2018;9(1):3049. In some embodiments, the antigen binding domain comprises the aforementioned engineered TIGIT extracellular region polypeptide. In some embodiments, the antigen binding domain is the aforementioned engineered TIGIT extracellular region polypeptide. In some embodiments, the TAC comprises: (a) an antigen binding domain (as used in the present application, the antigen binding domain comprises a domain that binds to an antigen, a ligand domain that binds to a receptor, or a receptor domain that binds to a ligand), which comprises an antigen binding fragment (e.g., sdAb, scFv, Fab, DARPin) that specifically recognizes one or more target antigens (e.g., tumor antigens) or target epitopes (e.g., tumor epitopes); (b) an optional first linker; (c) an extracellular TCR binding domain (e.g., sdAb, scFv, Fab, DARPin) that specifically recognizes an extracellular antigen binding domain of a TCR subunit (e.g., CD3ε); (d) an optional second linker; (e) an optional extracellular antigen binding domain of a first TCR co-receptor (e.g., CD4, CD8) or a part thereof; (f) a transmembrane domain, comprising a transmembrane domain of a second TCR co-receptor (e.g., CD4, CD8); and (g) an optional intracellular signal transduction domain, comprising an intracellular signal transduction domain of a third TCR co-receptor (e.g., CD4, CD8), wherein the TCR subunit is any one or more selected from the group consisting of: CD3ε, CD3δ, CD3γ, TCRα, TCRβ, TCRγ, and TCRδ, and wherein the first, second, and third TCR co-receptors are each independently any one selected from the group consisting of: CD4, CD8, and CD28. In some embodiments, the first, second, and third TCR co-receptors are the same. In some embodiments, the first, second, and third TCR co-receptors are different. In some embodiments, the TAC further comprises a hinge domain (e.g., derived from CD8α) located between C-terminus of the antigen binding domain and N-terminus of the transmembrane domain. In some embodiments, the antigen binding domain comprises the aforementioned engineered TIGIT extracellular region polypeptide. In some embodiments, the antigen binding domain is the aforementioned engineered TIGIT extracellular region polypeptide.

As used herein, the term "antigen binding domain" encompasses the concepts of "ligand binding domain" and "receptor binding domain". It is typically located in the extracellular portion of a cell (especially immune cell) receptor and can specifically bind to a certain protein. The scope of the certain protein is not limited in any way. Therefore, in some embodiments, the certain protein is a certain receptor, and the "antigen binding domain" is a part of the ligand of the certain receptor that specifically recognizes the certain receptor; in some embodiments, the protein is a certain ligand, and the "antigen binding domain" is a part of the receptor of the certain ligand that specifically recognizes the ligand; in some embodiments, the protein is an antibody or an antigen binding domain of the antibody, such as a single-chain antibody (scFv), Fab, F(ab')2, Fab', Fv, Fd, dAb or diabody.

### Chimeric Antigen Receptor (CAR)

The present application further provides a CD155-binding chimeric antigen receptor (CAR), which comprises one or more antigen binding domains, a transmembrane domain, and an intracellular signal transduction domain, wherein the one or more antigen binding domains comprise the aforementioned TIGIT extracellular region polypeptide.

In some embodiments, the one or more antigens, in addition to comprising CD155 that binds to the aforementioned TIGIT extracellular region polypeptide, further comprise one or more tumor antigens and/or immune checkpoint proteins.

Herein, in some embodiments, the one or more tumor antigens are selected from: PSCA, CEA, CD123, TSHR, CD171, CS-1, C-type lectin-like molecule-1, ganglioside GD3, Tn antigen, CD19, CD20, CD22, CD30, CD70, CD123, CD138, CD33, CD44, CD44v7/8, CD38, CD44v6, B7H3 (CD276), B7H6, CD117, IL-13Rα, IL-11Rα, PSMA, NY-ESO-1, HIV-1 Gag, MART-1, gp100, tyrosinase, mesothelin, EpCAM, PRSS21, vascular endothelial growth factor receptor, Lewis(Y) antigen, CD24, PDGFR-β, SSEA-4, MUC1, MUC6, EGFR, EGFR2, ERBB3, ERBB4, EGFRvIII, NCAM, CAIX, LMP2, EphA2, fucosyl GM1, sLe, ganglioside GM3 (aNeu5Ac(2-3)bDGalp(1-4)bDGlcp(1-1)Cer), TGS5, HMWMAA, OAcGD2, folate receptor, CD248, TEM7R, Claudin 6, Claudin 18.2, Claudin 18.1, ASGPR1, CDH16, 5T4, 8H9, αvβ6 integrin, BCMA, CA9, kappa light chain, CSPG4, EGP2, EGP40, FAP, FAR, FBP, embryonic AchR, HLA-A1, HLA-A2, MAGEA1, MAGE3, KDR, MCSP, NKG2D ligand, PSC1, ROR1, Sp17, SURVIVIN, TAG72, TEM1, fibronectin, tenascin, carcinoembryonic variant of tumor necrosis region, GPRC5D, CXORF61, CD97, CD179a, ALK, polysialic acid, (PLAC1, GloboH, NY-BR-1, UPK2, HAVCR1, ADRB3, PANX3, GPR20, LY6K, OR51E2, TARP, WT1, ETV6-AML, SPA17, XAGE1, Tie2, MAD-CT-1, MAD-CT-2, Fos-related antigen 1, p53 mutant, hTERT, sarcoma translocation breakpoint, ML-IAP, ERG, NA17, PAX3, androgen receptor, cyclin B1, MYCN, RhoC, CYP1B1, BORIS, SART3, (PAX5, OYTES1, (LCK, AKAP-4, SSX2, CD79a, CD79b, CD72, LAIR1, FCAR, LILRA2, CD300LF, CLEC12A, BST2, EMR2, LY75, GPC3, FCRL5, IGLL1, and CD155.

Herein, in some embodiments, the one or more immune checkpoint proteins are selected from:
2B4, 4-1BB, 4-1BB ligand, B7-1, B7-2, B7H2, B7H3, B7H4, B7H6, BTLA, CD155, CD160, CD19, CD200, CD27, CD27 ligand, CD28, CD40, CD40 ligand, CD47, CD48, CTLA-4, DNAM-1, Galectin-9, GITR, GITR ligand, HVEM, ICOS, ICOS ligand, IDO1, KIR, 3DL3, LAG-3, OX40, OX40 ligand, PD-L1, PD-1, PD-L2, LAG3, PGK, SIRPα, TIM-3, TIGIT, and VSIG8.

In some embodiments, the CAR comprises multiple antigen binding domains, wherein the multiple antigen binding domains are linked in tandem with each other. In some embodiments, the CAR comprises multiple antigen binding domains, and at least two of the multiple antigen binding domains are linked to the transmembrane domain of the CAR in parallel with each other. In some embodiments, the transmembrane domain comprises a transmembrane domain of CD4, CD8α, CD28, or CD3ζ.

In some embodiments, the one or more antigen binding domains in the CAR are directly linked to the transmembrane domain, and the one or more antigen binding domains comprise the aforementioned TIGIT extracellular region polypeptide that binds to CD155.

In some embodiments, the CAR further comprises a hinge region between the one or more antigen binding domains and the transmembrane domain. In some embodiments, the CAR comprises multiple antigen binding domains, and at least two of the multiple antigen binding domains are each linked to the same hinge region. In some embodiments, the CAR comprises multiple antigen binding domains, wherein the multiple antigen binding domains are linked to at least two hinge regions. In some embodiments, the CAR comprises multiple tandem antigen binding domains, and the multiple tandem antigen binding domains are linked directly or indirectly to one hinge region. In some embodiments, the hinge region is selected from hinge regions of IgG, IgD, CD7, CD8α, or CD28. In some embodiments, the intracellular signal transduction domain comprises a primary signal transduction domain, wherein the primary signal transduction domain is a signal transduction domain of CD3ζ, CD3γ, CD3δ, CD3ε, FcεRIγ, FcεR1β, CD79α, CD79β, FcγRIIa, DAP10, or DAP12 molecule. In some embodiments, the signal transduction domain further comprises a costimulatory domain, wherein the costimulatory domain is derived from a signal transduction domain of one or more of the following molecules: CD27, CD28, 4-1BB, OX40, CD30, CD40, CD2, LFA-1, LIGHT, NKG2C, B7-H3, PD-1, ICOS, CDS, ICAM-1, GITR, BAFFR, LIGHTR, SLAMF7, CD7, NKp80 (KLRF1), CD160, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1, SLAMF4, CD84, CD96, CEACAM1, CRTAM, CD229, CD160, PSGL1, CD100, CD69, SLAMF6, SLAMF1, SLAMF8, CD162, LTBR, LAT, GADS, SLP-76, PAG/Cbp, NKp44, NKp30, NKp46, NKG2D, and a ligand that specifically binds to CD83. In some embodiments, the signal transduction domain is a signal transduction domain of CD3ζ molecule, and the costimulatory domain is derived from a signal transduction domain of 4-1BB, CD134, ICOS, or CD28. In some embodiments, the signal transduction domain is a signal transduction domain of CD3ζ molecule, and the costimulatory domain is derived from a signal transduction domain of 4-1BB and CD28.

In addition, the present application further provides a protein combination comprising any one or more of the above fusion proteins, engineered receptors, or CARs, such as a combination of the above fusion protein and a CAR targeting any antigen. In some embodiments, the fusion protein comprises an extracellular region and an intracellular region, wherein the extracellular region comprises the aforementioned TIGIT extracellular region polypeptide, and the intracellular region comprises a costimulatory domain. In some embodiments, the fusion protein comprises an extracellular region and an intracellular region, wherein the extracellular region comprises the aforementioned TIGIT extracellular region polypeptide, and the intracellular region comprises a costimulatory domain, wherein the costimulatory domain is derived from a signal transduction domain of one or more costimulatory receptor molecules selected from the group consisting of: CARD11, CD2, CD4, CD7, CD19, CD27, CD28, CD30, CD40, CD160, ICAM-1, OX40, 4-1BB, SELPLG, LIGHT, HVEM, B7-H3, ICOS, PD-1, SLAMF7, LFA-1, NKG2C, CDS, GITR, BAFFR, NKp80, IPO-3, SLAMF8, LTBR, LAT, GADS, SLP-76, PAG/Cbp, NKp44, NKp30, NKp46, NKG2D, CD83, SLAMF1, CTLA-4, LAG-3, PD-L2, PD-L1, DAP10, TRIM, ZAP70, a ligand that specifically binds to CD83, and any combination thereof. In some embodiments, the fusion protein comprises an extracellular region and an intracellular region, wherein the extracellular region comprises the aforementioned TIGIT extracellular region polypeptide, and the intracellular region comprises the signal transduction domain of CD28. In some embodiments, the fusion protein comprises an extracellular region, an intracellular region, and a transmembrane domain linking the extracellular region and the intracellular region, wherein the extracellular region comprises the aforementioned TIGIT extracellular region polypeptide, the intracellular region comprises a costimulatory domain, and the transmembrane domain comprises the transmembrane domain of one or more molecules selected from: TIGIT, ICOS, CD4, CD8α, CD28, and CD3ζ. In some embodiments, the fusion protein comprises an extracellular region, an intracellular region, and a transmembrane domain linking the extracellular region and the intracellular region, wherein the extracellular region comprises the aforementioned TIGIT extracellular region polypeptide, the intracellular region comprises a signal transduction domain of CD28, and the transmembrane domain comprises a transmembrane domain of CD28.

In some embodiments, the protein combination comprises any one structure of the following:
1) PSCA-8h-8TM-BBZ and TIGIT-28TM-28Z(1)
2) CEA-8h-8TM-BBZ and TIGIT-28TM-28Z(1)
3) CD123-8h-8TM-2B4Z and TIGIT-28TM-28Z(1)
4) PSCA-G4h-28TM-28-BBZ(3) and TIGIT-28TM-28Z(1)
5) PSCA-G4h-28TM-28-BBZ(3) and TIGIT-28TM-28Z(1)
6) PSCA-7h-28TM-28Z and TIGIT-28TM-28Z(1)
7) PSCA-8h-8TM-BBZ and TIGIT-28TM-28
8) PSCA-G4h-28TM-28-BBZ(3) and TIGIT-28TM-28
9) CEA-8h-8TM-BBZ and TIGIT-28TM-28
10) CD123-8h-8TM-2B4Z and TIGIT-28TM-28
11) PSCA-G4h-28TM-28-BBZ(3) and TIGIT-28TM-28;
12) PSCA-7h-28TM-28Z(1) and TIGIT-28TM-28;
13) CEA-8h-8TM-BBZ and TIGIT-28TM-28Z; or
14) PSCA-8h-8TM-BBZ and TIGIT-28TM-28Z;
wherein CEA is an antibody that binds to CEA or an antigen-binding fragment thereof (e.g., scFv); CD123 is an antibody that binds to CD123 or an antigen-binding fragment thereof (e.g., scFv); PSCA is an antibody that binds to PSCA or an antigen-binding fragment thereof (e.g., scFv); TIGIT is the aforementioned TIGIT extracellular region polypeptide; 28TM is a transmembrane domain of CD28; 28 is an intracellular signal transduction domain of CD28; BB is an intracellular signal transduction domain of 4-1BB; Z, Z(1), or Z(3) is an intracellular region of CD3ζ; 2B4 is an intracellular signal transduction domain of 2B4; 8h is a hinge region of CD8; 8TM is a transmembrane domain of CD8; G4h is a hinge region of IgG4; 7h is a hinge region of CD7; "-" represents a peptide bond or a linking peptide.

### Engineered Nucleic Acid Molecule

The present application further provides an engineered nucleic acid molecule encoding the aforementioned engineered receptor, fusion protein, or chimeric antigen receptor, wherein the nucleic acid molecule comprises a coding sequence for a protein of interest, and the coding sequence for a protein of interest can be expressed as the engineered receptor, fusion protein, or chimeric antigen receptor. The term "engineered nucleic acid molecule" is used to distinguish from a "natural nucleic acid molecule". A "natural nucleic acid molecule" refers to a nucleic acid molecule that exists in its natural form in nature. An "engineered nucleic acid molecule" imposes a limitation on the source or preparation method for the nucleic acid molecule, without imposing any limitation on its function or structure. Therefore, an engineered nucleic acid molecule can be used to refer to any nucleic acid molecule obtainable by any or multiple bioengineering means, which may have a polynucleotide sequence identical to that of a natural nucleic acid molecule, have identical modifications to that of a natural nucleic acid molecule, or even form a structure completely consistent with that of a natural nucleic acid molecule. However, the engineered nucleic acid molecule is distinguished from its corresponding natural nucleic acid molecule, or a natural nucleic acid molecule having the same polynucleotide sequence, at least in that the engineered nucleic acid molecule is not directly purified or extracted in its natural form from animals or plants naturally occurring in nature.

In some embodiments, the engineered nucleic acid molecule is an engineered DNA molecule. In some embodiments, the DNA molecule can be replicated and/or be expressed in cells. In some embodiments, the DNA molecule can be replicated and/or be expressed in eukaryotic cells. In some embodiments, the DNA molecule can be replicated and/or be expressed in prokaryotic cells. In some embodiments, the DNA molecule can be expressed in eukaryotic cells and can replicate in prokaryotic cells. Therefore, in addition to comprising the coding sequence for the protein of interest, the DNA molecule further comprises genetic operation or regulatory elements for replication and/or expression in prokaryotic and/or eukaryotic cells.

The necessary structural elements for enabling replication or efficient replication of the engineered DNA molecule in cells are known in the art, including, for example, an origin of replication (ORI). In some embodiments, the engineered DNA molecule further comprises a marker gene or a fragment thereof and/or a reporter gene or a fragment thereof, and unique restriction endonuclease sites allowing insertion of DNA elements, preferably restriction endonuclease sites in the form of a multiple cloning site (MCS). The marker gene facilitates the identification of cells containing a plasmid that comprises the marker gene, and may be selected from, for example, antibiotic resistance genes. Each restriction endonuclease site in the MCS can be specifically recognized by a different restriction endonuclease.

In some embodiments, the DNA molecule is a DNA plasmid. As used herein, the term "DNA plasmid" refers to a plasmid composed of double-stranded DNA molecules. In some embodiments, the "plasmid" is a circular DNA molecule. In some embodiments, the "plasmid" may also encompass linear DNA molecules. Specifically, the term "plasmid" further encompasses molecules obtained by linearizing a circular plasmid, for example, by cleaving the circular plasmid with a restriction endonuclease to convert the circular plasmid molecule into a linear molecule, as well as linear molecules capable of replicating in prokaryotes. A plasmid is capable of replication, i.e., amplification in a cell independent of genomic genetic information stored in the nucleoid or nucleoid-like structure of prokaryotic cells, and can be used for cloning, i.e., for amplifying genetic information in bacterial cells. Preferably, the DNA plasmid according to the present application is a medium-copy or high-copy plasmid, more preferably a high-copy plasmid. Examples of such high-copy plasmids are vectors based on pUC, pTZ plasmids or any other plasmids that contain an ORI supporting high copy number of the plasmid (such as pMB1, pCoIE1) and the like.

In some embodiments, the engineered DNA molecule is a DNA molecule constituting a prokaryotic nucleoid or nucleoid-like structure or a fragment thereof, or a DNA molecule constituting a eukaryotic genome or a fragment thereof, i.e., the coding sequence for the protein of interest or its complementary sequence is capable of replicating along with the prokaryotic genome.

In some embodiments, the engineered DNA molecule can be transcribed into mRNA. In some embodiments, the engineered DNA molecule further comprises coding sequences for elements that can be used post-transcriptionally to initiate or regulate the expression of the protein, polypeptide or fragment thereof, the elements including but not limited to 5'UTR, 3'UTR, poly(A) tail (or polyadenylation signal) and the like. In some embodiments, the engineered DNA molecule comprises a coding sequence for at least one untranslated region (UTR). In some embodiments, the engineered DNA molecule comprises at least a coding sequence for 5'UTR and the coding sequence for the protein of interest. In some embodiments, the engineered DNA molecule, from 5' to 3', comprises sequentially at least a coding sequence for 5'UTR, the coding sequence for the protein of interest, a coding sequence for 3'UTR, a polyadenylation signal (or DNA sequence corresponding to the poly(A) tail sequence), and both ends of the coding sequence for the protein of interest may further comprise a start codon (at the 5' end) and a stop codon (at the 3' end), respectively, which are the first three nucleotides and the last three nucleotides of the mRNA molecule that are translatable. The 5'UTR generally comprises at least one ribosome binding site (RBS), such as the Shine-Dalgarno sequence in prokaryotes, or at least one translation initiation site, such as the Kozak sequence in eukaryotes. RBS promotes efficient and accurate translation of an mRNA molecule by recruiting ribosomes during initiation of translation. The activity of a given RBS can be optimized by varying its length and sequence or translation initiation site, as well as the distance to the start codon. Alternatively or optionally, the 5'UTR comprises internal ribosome entry sites or IRES. The 3'UTR may comprise one or more regulatory sequences, such as binding sites for amino acid sequences that enhance mRNA molecule stability, binding sites for regulatory RNA molecules (such as miRNA molecules), and/or signal sequences involved in the intracellular transport of mRNA molecules.

Based on the aforementioned embodiments, in some embodiments, the gene fragment of interest further comprises one or more additional regulatory sequences, such as a binding site for an amino acid sequence that enhances the mRNA molecule stability, a binding site for an amino acid sequence that enhances translation of the mRNA molecule, a regulatory element (such as riboswitch), and/or a nucleotide sequence that exerts a positive effect on translation initiation. Furthermore, within the 5'UTR, there are preferably no functional upstream open reading frames, out-of-frame upstream translation initiation sites, out-of-frame upstream start codons, and/or nucleotide sequences that generate a secondary structure that reduces or prevents translation. The presence of such nucleotide sequences in the 5'UTR may exert a negative effect on translation.

The coding sequence for the protein of interest comprises codons that can be translated into an amino acid sequence. Among all the codons comprised in the coding sequence for the protein of interest, all may be naturally occurring codons encoding amino acids, or some or all of them may be artificially synthesized codons. In some embodiments, some or all of the codons are codon-optimized. In some embodiments, some or all of the codons encode non-natural amino acids.

In some embodiments, the engineered DNA molecule further comprises, at the 5'-end side of the gene fragment of interest, structural elements necessary for initiating or regulating the transcription of the RNA, and such structural elements are known in the art. In some embodiments, the structural elements comprise at least a promoter. Promoters and sequences thereof are known in the art, including weak promoters, medium-strength promoters, strong promoters, mini promoters or core promoters and the like. In some specific embodiments, the promoter is a strong promoter. In some embodiments, the promoter can initiate transcription of the coding sequence for the protein of interest in prokaryotic cells. In some embodiments, the promoter can initiate transcription of the coding sequence for the protein of interest in eukaryotic cells. The "promoter" comprises at least one transcription recognition site and a downstream transcription factor binding site. The recognition and binding sites can interact with amino acid sequences that mediate or regulate transcription. The binding site is closer to the aforementioned gene fragment of interest than the recognition site. The binding site can be, for example, a Pribnow box in prokaryotes or a TATA box in eukaryotes. For example, in some embodiments, when a Pribnow box is used, the transcription recognition site can be located at about 35 bp upstream of the transcription start site, and the transcription factor binding site can be located at about 10 bp upstream of the transcription start site. In some embodiments, the promoter comprises at least one additional regulatory element, such as an AT-rich upstream element located about 40 and/or 60 nucleotides upstream of the transcription start site, and/or an additional regulatory element that enhances promoter activity located between the recognition site and the binding site. In some embodiments, the promoter is a strong promoter, i.e., the promoter comprises a sequence that promotes transcription of the aforementioned coding sequence for the protein of interest. Strong promoters are known to those skilled in the art, for example, OXB18, OXB19, and OXB20 promoters derived from the RecA promoter of *Escherichia coli,* or can be identified or synthesized by routine laboratory procedures. In some embodiments, the promoter is a T7 promoter. In some embodiments, additional regulatory elements, such as an enhancer comprised in a DNA plasmid that can promote transcription of the aforementioned coding sequence for the protein of interest, are further comprised upstream of the promoter.

In some embodiments, the eukaryotic cell is a yeast cell. In some embodiments, the DNA molecule is a yeast display vector.

In addition, the present application further provides an engineered RNA molecule encoding the aforementioned engineered receptor, fusion protein, engineered receptor or chimeric antigen receptor. In some embodiments, the engineered RNA molecule is obtained by transcription from the aforementioned engineered DNA molecule. In some embodiments, the engineered RNA molecule has the same sequence as an RNA molecule obtained by transcription from the aforementioned engineered DNA molecule. In some embodiments, the engineered RNA is mRNA. As used herein, "mRNA" (messenger RNA) refers to any naturally occurring, non-naturally occurring or modified RNA encoding at least one protein, polypeptide or fragment thereof. The mRNA is capable of being translated to produce the encoded protein, polypeptide, or fragment thereof *in vitro, in vivo, in situ* or *ex vivo.* Therefore, the mRNA can be mature mRNA or pre-mature mRNA, and the elements or structures that it must comprise or optionally comprise are known in the art. In some embodiments, the mRNA comprises coding sequences for multiple necessary functional components to express, regulate, or enhance the expression level of the protein, polypeptide, or fragment thereof. The functional components include, but are not limited to, 5' cap, 5'UTR, 3'UTR, and the like. Both 5'UTR and 3'UTR are typically transcribed from genomic DNA and are elements present in the pre-mature mRNA.

The term "5' cap" is located at the 5'-most end of mRNA and comprises a methylated guanylate that is linked to the 5' end of mRNA via pyrophosphate to form a 5',5'-triphosphate linkage with adjacent nucleotides. There are typically three types of 5' cap structures (m7G5'ppp5'Np, m7G5'ppp5'NmpNp, and m7G5'ppp5'NmpNmpNp), referred to as Type O, Type I, and Type II, respectively. Type O indicates that the ribose of the terminal nucleotide is unmethylated, Type I indicates that the ribose of one terminal nucleotide is methylated, and Type II indicates that the riboses of two terminal nucleotides are both methylated. In some embodiments, the 5' cap can be simultaneously added to the 5' end of a polynucleotide during an *in vitro* transcription reaction using the following chemical RNA cap analogs according to the manufacturer's protocol, producing a 5'-guanosine cap structure: 3'-O-Me-m7G(5')ppp(5')G [ARCA cap], G(5')ppp(5')A, G(5')ppp(5')G, m7G(5')ppp(5')A, m7G(5')ppp(5')G (New England BioLabs, Ipswich, MA), or m7G(5')ppp(5')(2'-OMeA)pG (CleanCap AG). For example, in some embodiments, vaccinia virus capping enzyme can be used to complete 5' capping of modified RNA after transcription to produce a Type O cap structure: m7G(5')ppp(5')G (New England BioLabs, Ipswich, MA). Both vaccinia virus capping enzyme and 2'-O-methyltransferase can be used to produce a Type I cap structure, yielding m7G(5')ppp(5')(2'-OMeA)pG. A Type II cap structure can be produced from the Type I cap structure by further 2'-O-methylation of the S'-antepenultimate nucleotide using 2'-O-methyltransferase. A Type III cap structure can be produced from the Type II cap structure by further 2'-O-methylation of the 5'-preantepenultimate nucleotide using 2'-O-methyltransferase.

In some embodiments, the mRNA further comprises a stabilizing element. Stabilizing elements can include, for example, histone stem-loops. In some embodiments, the mRNA comprises a coding region, at least one histone stem-loop, and optionally a poly(A) sequence or a polyadenylation signal. The poly(A) sequence or polyadenylation signal should generally enhance the expression level of the encoded protein. In some embodiments, the mRNA comprises a combination of a poly(A) sequence or polyadenylation signal and at least one histone stem-loop, and although the two have an alternative mechanism in nature, their synergistic effect can increase protein expression to a level exceeding that is observed with either individual element. The synergistic effect of the combination of poly(A) and at least one histone stem-loop is independent of the order of elements or the length of the poly(A) sequence. In some embodiments, the histone stem-loop is typically derived from a histone gene and comprises two adjacent partially or fully reverse complementary sequences that form a loop through intramolecular base pairing, with a spacer region (composed of a short sequence) between them. Unpaired loop region is generally incapable of base pairing with either element of the stem-loop. The stability of a stem-loop structure generally depends on the length, the number of mismatches or bulges, and the base composition of the paired region. In some embodiments, wobble base pairing (non-Watson-Crick base pairing) may occur. In some embodiments, the at least one histone stem-loop sequence comprises 15 to 45 nucleotides in length.

In some embodiments, one or more AU-rich sequences of the mRNA can be removed. These sequences, sometimes referred to as AURES, are destabilizing sequences found in the 3'UTR. AURES can be removed from the mRNA. Alternatively, AURES can be retained in the mRNA.

In some embodiments, the mRNA is formulated in a lipid nanoparticle (LNP). In some embodiments, the lipid is mixed with the mRNA to form lipid nanoparticles. In some embodiments, the RNA is formulated in lipid nanoparticles. In some embodiments, the lipid nanoparticles are first formed as empty lipid nanoparticles and combined with or encapsulate the mRNA of a vaccine immediately prior to administration (e.g., within minutes to one hour).

The lipid nanoparticles generally comprise an ionizable lipid, a non-cationic lipid, sterol, and a PEG lipid component, as well as a target nucleic acid, such as the mRNA described above. The lipid nanoparticles of the present application can be produced using components, compositions, and methods as generally known in the art, with reference to, for example, PCT/US2016/052352, PCT/US2016/068300, PCT/US2017/037551, PCT/US2015/027400, PCT/US2016/047406, PCT/US2016000129, PCT/US2016/014280, PCT/US2016/014280, PCT/US2017/038426, PCT/US2014/027077, PCT/US2014/055394, PCT/US2016/52117, PCT/US2012/069610, PCT/US2017/027492, PCT/US2016/059575, and PCT/US2016/069491, all of which are incorporated herein by reference in their entirety.

In some embodiments, the engineered nucleic acid molecule further may be a DNA-RNA hybrid molecule, and the DNA-RNA hybrid molecule carries the same genetic information as the engineered DNA molecule or the engineered RNA molecule.

### Engineered Cell

The present application further provides an engineered cell, wherein the engineered cell expresses or comprises on its cell membrane the aforementioned TIGIT extracellular region polypeptide, fusion protein, engineered receptor, chimeric antigen receptor, or engineered nucleic acid molecule.

In some embodiments, the engineered cell is an engineered immune cell. In some embodiments, the engineered cell is derived from a T cell, NK cell, macrophage, DC cell, B cell, or a precursor cell thereof. In some embodiments, the engineered cell is a CAR-T or CAR-NK cell targeting one or more antigenic epitopes of CD155. In some embodiments, the engineered cell is a TCR-T cell targeting one or more antigenic epitopes of CD155. In some embodiments, the engineered cell is a TAC-T cell targeting one or more antigenic epitopes of CD155.

In some embodiments, the engineered cell is a CAR-T or CAR-NK cell targeting multiple different antigens, and it comprises the aforementioned TIGIT extracellular region, fusion protein, engineered receptor, or chimeric antigen receptor, and one or more CARs targeting other tumor antigens. In some embodiments, the engineered cell is a CAR-T or CAR-NK cell targeting multiple different antigens, and it comprises the aforementioned TIGIT extracellular region, fusion protein, engineered receptor, or chimeric antigen receptor, and one or more CARs targeting immune checkpoint proteins. In some embodiments, the engineered cell is a CAR-T or CAR-NK cell targeting multiple different antigens, and it comprises the aforementioned TIGIT extracellular region, fusion protein, engineered receptor, or chimeric antigen receptor, one or more CARs targeting other tumor antigens, and one or more CARs targeting immune checkpoint proteins. In some embodiments, the engineered cell comprises the aforementioned engineered receptor, and the engineered cell comprises one or more CARs targeting other tumor antigens and/or one or more CARs targeting immune checkpoint proteins, wherein the aforementioned engineered receptor comprises or consists of, from N-terminus to C-terminus: an antigen binding domain-a transmembrane domain-a costimulatory domain (or a secondary signal transduction domain), or wherein the aforementioned engineered receptor comprises or consists of, from N-terminus to C-terminus: an antigen binding domain-a hinge region-a transmembrane domain-a costimulatory domain (or a secondary signal transduction domain).

In some embodiments, the engineered cell is a TCR-T cell targeting multiple different antigens, and it comprises the aforementioned TIGIT extracellular region, fusion protein, or engineered receptor, wherein the antigen binding domain of the TCR comprises the aforementioned TIGIT extracellular region polypeptide, and the TCR-T further comprises one or more TCRs targeting other tumor antigens. In some embodiments, the engineered cell is a TCR-T cell targeting multiple different antigens, and it comprises the aforementioned TIGIT extracellular region, fusion protein, or engineered receptor, wherein the antigen binding domain of the TCR comprises the aforementioned TIGIT extracellular region polypeptide, and the TCR-T further comprises one or more TCRs targeting immune checkpoint proteins. In some embodiments, the engineered cell is a TCR-T cell targeting multiple different antigens, and it comprises the aforementioned TIGIT extracellular region, fusion protein, or engineered receptor, wherein the antigen binding domain of the TCR comprises the aforementioned TIGIT extracellular region polypeptide, and the TCR-T further comprises one or more TCRs targeting other tumor antigens and one or more TCRs targeting immune checkpoint proteins. In some embodiments, the engineered cell comprises the aforementioned engineered receptor, and the engineered cell further comprises one or more TCRs targeting other tumor antigens and/or one or more TCRs targeting immune checkpoint proteins, wherein the aforementioned engineered receptor comprises or consists of, from N-terminus to C-terminus: an antigen binding domain-a transmembrane domain-a costimulatory domain (or a secondary signal transduction domain), or wherein the aforementioned engineered receptor comprises or consists of, from N-terminus to C-terminus: an antigen binding domain-a hinge region-a transmembrane domain-a costimulatory domain (or a secondary signal transduction domain).

In some embodiments, the engineered cell is a TAC-T cell targeting multiple different antigens, and it comprises the aforementioned TIGIT extracellular region, fusion protein, or engineered receptor, wherein the antigen binding domain of the TAC comprises the aforementioned TIGIT extracellular region polypeptide, and the TAC-T further comprises one or more TACs targeting other tumor antigens. In some embodiments, the engineered cell is a TAC-T cell targeting multiple different antigens, and it comprises the aforementioned TIGIT extracellular region, fusion protein, or engineered receptor, wherein the antigen binding domain of the TAC comprises the aforementioned TIGIT extracellular region polypeptide, and the TAC-T further comprises one or more TACs targeting immune checkpoint proteins. In some embodiments, the engineered cell is a TAC-T cell targeting multiple different antigens, and it comprises the aforementioned TIGIT extracellular region, fusion protein, or engineered receptor, wherein the antigen binding domain of the TAC comprises the aforementioned TIGIT extracellular region polypeptide, and the TAC-T further comprises one or more TACs targeting other tumor antigens and one or more TACs targeting immune checkpoint proteins. In some embodiments, the engineered cell comprises the aforementioned engineered receptor, and the engineered cell further comprises one or more TACs targeting other tumor antigens and/or one or more TACs targeting immune checkpoint proteins, wherein the aforementioned engineered receptor comprises or consists of, from N-terminus to C-terminus: an antigen binding domain-a transmembrane domain-a costimulatory domain (or a secondary signal transduction domain), or wherein the aforementioned engineered receptor comprises or consists of, from N-terminus to C-terminus: an antigen binding domain-a hinge region-a transmembrane domain-a costimulatory domain (or a secondary signal transduction domain).

In some embodiments, the engineered cell expresses or comprises on its cell membrane one or more engineered receptors, wherein at least one of the engineered receptors comprises the aforementioned TIGIT extracellular region polypeptide, and upon binding to CD155, the engineered receptor activates or inhibits a downstream signaling pathway of the engineered receptor. In some embodiments, the engineered receptor is one or more selected from the group consisting of CAR, TCR, TAC, and fusion protein. In some embodiments, the engineered cell expresses or comprises on its cell membrane one or more engineered receptors, wherein the engineered receptor is one or more selected from CAR, TCR, TAC, or fusion protein, at least one of the engineered receptors comprises the aforementioned TIGIT extracellular region polypeptide or fusion protein, and upon binding to CD155, the engineered receptor activates or inhibits a downstream signaling pathway of the engineered receptor. In some embodiments, the engineered cell comprises the aforementioned engineered receptor, and the engineered cell further comprises one or more TACs, CARs, or TCRs targeting other tumor antigens and/or one or more TACs, TCRs, or CARs targeting immune checkpoint proteins, wherein the aforementioned engineered receptor comprises or consists of, from N-terminus to C-terminus: an antigen binding domain-a transmembrane domain-a costimulatory domain (or a secondary signal transduction domain), or wherein the aforementioned engineered receptor comprises or consists of, from N-terminus to C-terminus: an antigen binding domain-a hinge region-a transmembrane domain-a costimulatory domain (or a secondary signal transduction domain).

In some embodiments, the engineered cell is selected from: T cell, NK cell, macrophage, DC cell, B cell, or a precursor cell thereof.

In some embodiments, the tumor antigen described herein is one or more selected from the group consisting of:
PSCA, CEA, CD123, TSHR, CD171, CS-1, C-type lectin-like molecule-1, ganglioside GD3, Tn antigen, CD19, CD20, CD22, CD30, CD70, CD123, CD138, CD33, CD44, CD44v7/8, CD38, CD44v6, B7H3 (CD276), B7H6, CD117, IL-13Rα, IL-11Rα, PSMA, NY-ESO-1, HIV-1 Gag, MART-1, gp100, tyrosinase, mesothelin, EpCAM, PRSS21, vascular endothelial growth factor receptor, Lewis(Y) antigen, CD24, PDGFR-β, SSEA-4, MUC1, MUC6, EGFR, EGFR2, ERBB3, ERBB4, EGFRvIII, NCAM, CAIX, LMP2, EphA2, fucosyl GM1, sLe, ganglioside GM3 (aNeu5Ac(2-3)bDGalp(1-4)bDGlcp(1-1)Cer), TGS5, HMWMAA, OAcGD2, folate receptor, CD248, TEM7R, Claudin 6, Claudin 18.2, Claudin 18.1, ASGPR1, CDH16, 5T4, 8H9, αvβ6 integrin, BCMA), CA9, kappa light chain, CSPG4, EGP2, EGP40, FAP, FAR, FBP, embryonic AchR, HLA-A1, HLA-A2, MAGEA1, MAGE3, KDR, MCSP, NKG2D ligand, PSC1, ROR1, Sp17, SURVIVIN, TAG72, TEM1, fibronectin, tenascin, carcinoembryonic variant of tumor necrosis region, GPRC5D, CXORF61, CD97, CD179a, ALK, polysialic acid, (PLAC1, GloboH, NY-BR-1, UPK2, HAVCR1, ADRB3, PANX3, GPR20, LY6K, OR51E2, TARP, WT1, ETV6-AML, SPA17, XAGE1, Tie2, MAD-CT-1, MAD-CT-2, Fos-related antigen 1, p53 mutant, hTERT, sarcoma translocation breakpoint, ML-IAP, ERG, NA17, PAX3, androgen receptor, cyclin B1, MYCN, RhoC, CYP1B1, BORIS, SART3, (PAX5, OYTES1, (LCK, AKAP-4, SSX2, CD79a, CD79b, CD72, LAIR1, FCAR, LILRA2, CD300LF, CLEC12A, BST2, EMR2, LY75, GPC3, FCRL5, IGLL1, and CD155.

In some embodiments, the immune checkpoint protein described herein is one or more selected from the following: 2B4, 4-1BB, 4-1BB ligand, B7-1, B7-2, B7H2, B7H3, B7H4, B7H6, BTLA, CD155, CD160, CD19, CD200, CD27, CD27 ligand, CD28, CD40, CD40 ligand, CD47, CD48, CTLA-4, DNAM-1, Galectin-9, GITR, GITR ligand, HVEM, ICOS, ICOS ligand, IDO1, KIR, 3DL3, LAG-3, OX40, OX40 ligand, PD-L1, PD-1, PD-L2, LAG3, PGK, SIRPα, TIM-3, TIGIT, and VSIG8.

### Use

The present application further provides use of the aforementioned engineered TIGIT extracellular region polypeptide, fusion protein, engineered receptor, chimeric antigen receptor, engineered nucleic acid molecule, and engineered cell in the manufacture of a medicament for treating cancer. In some embodiments, the cancer is one or more selected from the group consisting of:
bladder cancer, blood cancer, bone cancer, bone marrow cancer, brain/nervous system cancer, breast cancer, colorectal cancer, esophageal cancer, gastrointestinal cancer, head cancer, kidney cancer, liver cancer, lung cancer, nasopharyngeal cancer, neck cancer, ovarian cancer, pancreatic cancer, prostate cancer, skin cancer, gastric cancer, testicular cancer, tongue cancer, and uterine cancer.

In some embodiments, tumor cells involved in the cancer highly express CD155.

In addition, the present application further provides use of the aforementioned engineered TIGIT extracellular region polypeptide, fusion protein, engineered receptor, chimeric antigen receptor or engineered cell as a TIGIT antagonist, wherein the use prevents TIGIT on a surface of immune cells from binding to CD155, thereby reversing tumor immunosuppression caused by the binding of TIGIT to CD155. Therefore, in some embodiments, the engineered TIGIT extracellular region polypeptide, fusion protein, engineered receptor, chimeric antigen receptor or engineered cell can be administered in combination with other anti-cancer agents.

### Examples

### Example 1: Screening of TIGIT Mutant Peptides

The TIGIT extracellular region was subjected to random mutagenesis, followed by specificity screening for TIGIT mutant peptides through *in vitro* killing based on the principle of molecular recognition.

### 1) Random mutagenesis of the TIGIT extracellular region:

Random mutagenesis was performed on the TIGIT extracellular region (SEQ ID NO: 1) using Method 1, obtaining 24 mutants Mut1 to Mut24 as shown in the sequences at the end of the text.

In mutants Mut1 to Mut24, mutation sites relative to the wild-type TIGIT are marked in bold.

Chimeric antigen receptors (CARs) were constructed using TIGIT as an antigen binding domain (or an extracellular antigen binding domain). Table 1 below shows the correspondence between the CARs and the TIGIT mutants contained therein.

**Table 1: CARs and Antigen Binding Domains Therein**

| Serial No. | Mutant Peptide Name | Mutant Name | CAR No. | Viral Titer (TU/mL) |
|---|---|---|---|---|
| 1 | TIGIT(1) | Mut1 | CAR1 | 2.76E7 |
| 2 | TIGIT(2) | Mut2 | CAR2 | 1.88E7 |
| 3 | TIGIT(3) | Mut3 | CAR3 | 5.5E7 |
| 4 | TIGIT(4) | Mut4 | CAR4 | 1.19E7 |
| 5 | TIGIT(6) | Mut5 | CAR5 | 2.57E8 |
| 6 | TIGIT(C48W) | Mut6 | CAR6 | 1.51E8 |
| 7 | TIGIT(N37D) | Mut7 | CAR7 | 3.45E7 |
| 8 | TIGIT(9) | Mut8 | CAR8 | 3.3E6 |
| 9 | TIGIT(10) | Mut9 | CAR9 | 4.86E8 |
| 10 | TIGIT(12) | Mut10 | CAR10 | 8.35E7 |
| 11 | TIGIT(13) | Mut11 | CAR11 | 2E6 |
| 12 | TIGIT(17) | Mut12 | CAR12 | 5.34E7 |
| 13 | TIGIT(18) | Mut13 | CAR13 | 2.3E6 |
| 14 | TIGIT(19) | Mut14 | CAR14 | 1.59E8 |
| 15 | TIGIT(8) | Mut15 | CAR15 | 5.44E8 |
| 16 | TIGIT(11) | Mut16 | CAR16 | 1.7E6 |
| 17 | TIGIT(15) | Mut17 | CAR17 | 6.65E8 |
| 18 | TIGIT(16) | Mut18 | CAR18 | 1.9E6 |
| 19 | TIGIT(20) | Mut19 | CAR19 | 3.38E8 |
| 20 | TIGIT(22) | Mut29 | CAR20 | 7.52E8 |
| 21 | TIGIT(23) | Mut21 | CAR21 | 2.84E8 |
| 22 | TIGIT(24) | Mut22 | CAR22 | 2.3E6 |
| 23 | TIGIT(14) | Mut23 | CAR23 | 5.1E6 |
| 24 | TIGIT(21) | Mut24 | CAR24 | 9.51E7 |

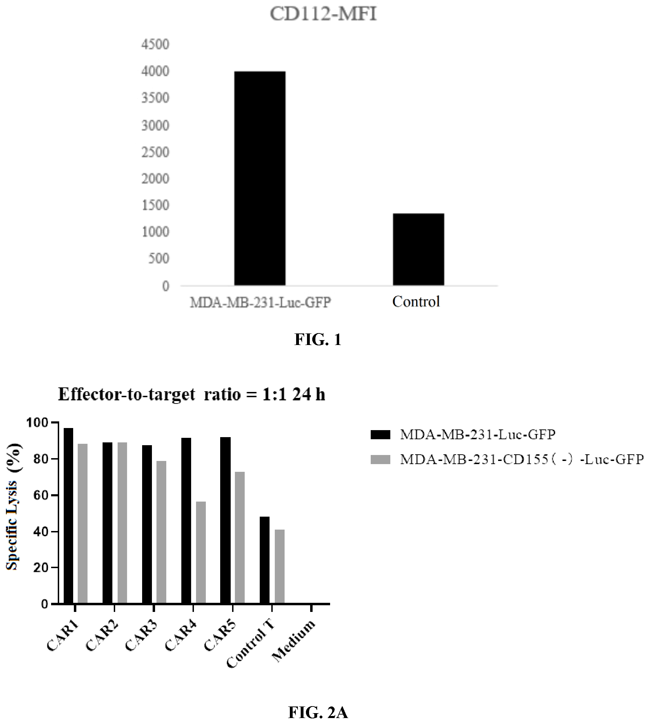

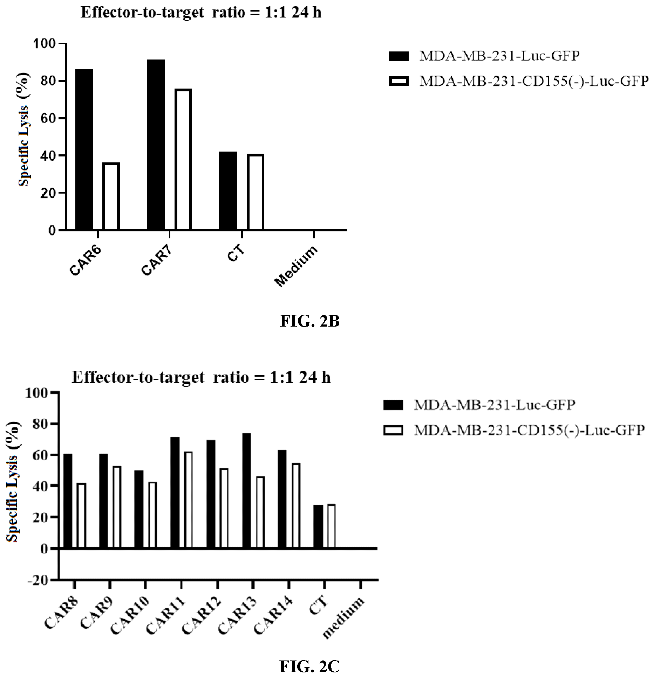

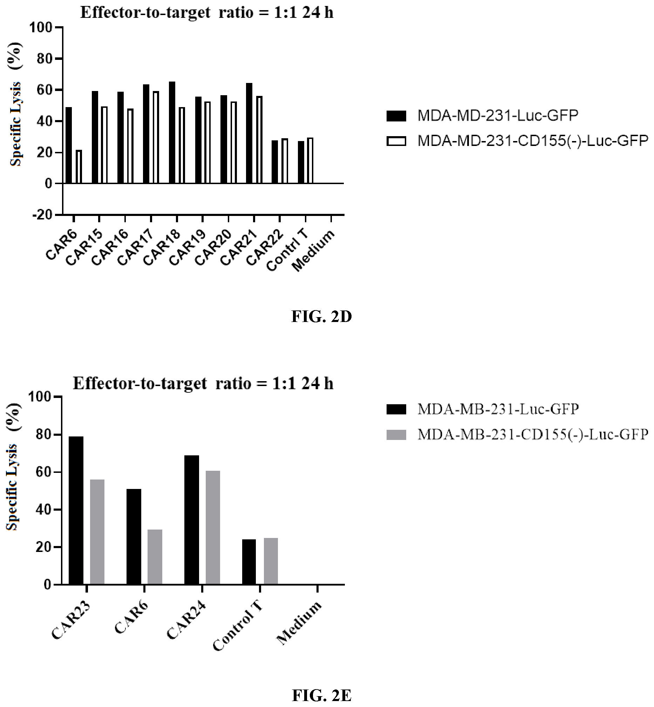

2) Screening of mutant peptides: The mutant peptides obtained in 1) were constructed as an extracellular antigen binding domain into a CAR structure of canonical CD28TM-28Z (the amino acid sequence of CD28TM is as set forth in SEQ ID NO:36, the amino acid sequence of 28 is as set forth in SEQ ID NO:37, the amino acid sequence of Z is as set forth in SEQ ID NO:41, and the amino acid sequences corresponding to Mut1 to Mut24 are as set forth in SEQ ID NO:2 to SEQ ID NO:25). Specifically, the CAR structure was: TIGIT mutant peptide-CD28TM-28Z, using common methods in the art, such as enzyme digestion and ligation, and detailed procedures can be found in Molecular Cloning: A Laboratory Manual (Third Edition, by J. Sambrook et al.), and Genetic Engineering, a textbook for higher education institutions in the 21st century, edited by Lou Shilin, Yang Shengchang, et al. A vector plasmid of interest containing a gene encoding the CAR (CAR gene) was constructed. The plasmid of interest was transfected into a 293T-derived cell line via calcium phosphate transfection for virus production. Titer was calculated by a formula as follows: Titer (TU/ml) = 1×10⁵ × positive rate × dilution factor ÷ virus volume × 1000. PBMCs or T cells obtained by Ficoll separation or apheresis, or cells thawed from cryopreserved PBMCs or T cells obtained above, were used for CAR-T cell preparation. The obtained PBMCs or T cells were activated with anti-CD3 and anti-CD28 monoclonal antibodies or activated magnetic beads coated with anti-CD3 and anti-CD28 monoclonal antibodies (CD3/CD28 Dynabeads (40203D; Gibco)), and transduced with CAR virus prepared from vectors corresponding to those in Table 2, to obtain CAR-T cells. The CAR-T cells described above were used to recognize CD155-positive and CD155-negative but CD112-positive target cells, respectively. Herein, the cell line used in MDA-MD-231-Luc-GFP was the MDA-MD-231 cell line, a human breast cancer cell line positive for both CD155 and CD112. MDA-MD-231-CD155(-)-Luc-GFP was a human breast cancer cell line negative for CD155 but positive for CD112. FIG. 1 shows CD112 expression in the MDA-MB-231-Luc-GFP group. The control (CT) group refers to the result of incubation of the MDA-MB-231 cell line with PBS, and the MDA-MB-231-Luc-GFP group refers to the result of co-incubation of MDA-MB-231-Luc-GFP with an anti-CD112 antibody (CD112 (Nectin2) Monoclonal Antibody (R2.525) PE Lot: WC3208861A). FIGS. 2A-2E screened for mutant peptides that exhibited killing against CD155-positive cells but showed significantly reduced or no killing against CD155-negative cells, i.e., mutant peptides that were capable of specifically recognizing CD155 but did not recognize or had reduced recognition ability for CD112. The effector-to-target ratio used herein was 1:1.

As shown in FIGS. 2A to 2E, the horizontal axis represents CAR-T containing different TIGIT mutant peptides, and the vertical axis represents the *in vitro* killing of CD155-positive and CD155-negative cells by the CAR-T cells. These drawings show results of specificity screening and killing of 24 CAR-T. Based on the specificity screening criteria, the Mut6 mutant peptide corresponding to CAR6, i.e., a mutant peptide with a C-to-W mutation at position 48 relative to the wild type, was determined as being capable of specifically recognizing CD155 in biological activity and exhibiting significantly reduced recognition of CD112. For the TIGIT mutant peptide sequences corresponding to CAR4, 5, 7, 8, 9, 10, 11, 12, 13, 14, 15, 16, 18, 21, 23, and 24, the ratios of binding activity to CD155 to binding activity to CD112 were also significantly higher than those of the wild type. Therefore, their corresponding TIGIT mutant peptide sequences SEQ ID NOs:5-17, SEQ ID NO:19, and SEQ ID NOs:22, 24, and 25 are also optional sequences in the present application.

The sequences used for each TIGIT mutant peptide and each element in the CAR structures used in the examples of the present application are shown in the Sequence Listing at the end of the text.

CAR structural element: The CAR structural elements described herein refers to a structural region constituting a chimeric antigen receptor, such as extracellular recognition region (such as the TIGIT sequence described in the present application, and extracellular recognition sequence recognizing PSCA, CD123, and CEA), a hinge region (also sometimes referred to as spacer region, sequence extending from a transmembrane domain to a binding region, such as 8h, 7h, and G4h described in the present application), a transmembrane region, and an intracellular signaling region (which may contain an activating signal and/or a co-stimulatory signal). Molecules constituting the above structures are structural elements.

### Example 2: Validation of Specific Recognition Site

Analysis of Mut6 sequence revealed that the amino acid at position 48 was mutated from C to W. To determine whether the mutation at position 48 is a key amino acid mutation that can improve specificity, this mutant peptide and wild-type TIGIT (WT) were constructed as an extracellular antigen binding domain into the CAR structure of canonical CD28TM-28Z. The CAR structure was: TIGIT peptide-CD28TM-28Z, forming CAR6 and CAR30, wherein CAR30 is a control CAR structure containing the wild-type TIGIT polypeptide (WT).

1) CAR6 and CAR30 were packaged into viruses and then used to infect CHO cells. First, successful preparation of CAR-CHO was confirmed by flow cytometry through labeling with an anti-TIGIT antibody. Then, CD155 and CD112 molecules were labeled, and the binding of different TIGIT mutant peptides to CD155 and CD112 was detected by flow cytometry to assess the binding specificity of different TIGIT mutants.

Data analysis method: Flow cytometry data generated in method 1) were analyzed. The analysis process involved first calculating the MFI (mean fluorescence intensity) value of the positive cell population in which the anti-TIGIT antibody bound to the TIGIT mutant, and then calculating the MFI values of the positive cell population in which the TIGIT mutant bound to the CD155 antigen and the CD112 antigen, respectively.

Relative MFI of CD155 = MFI of CD155-positive cell population / MFI of TIGIT-positive cell population

Relative MFI of CD112 = MFI of CD112-positive cell population / MFI of TIGIT-positive cell population

Relative MFI values for CD155 and CD112 between groups were each statistically analyzed against values of CAR30 by t-test. P < 0.05 indicates a significant difference.

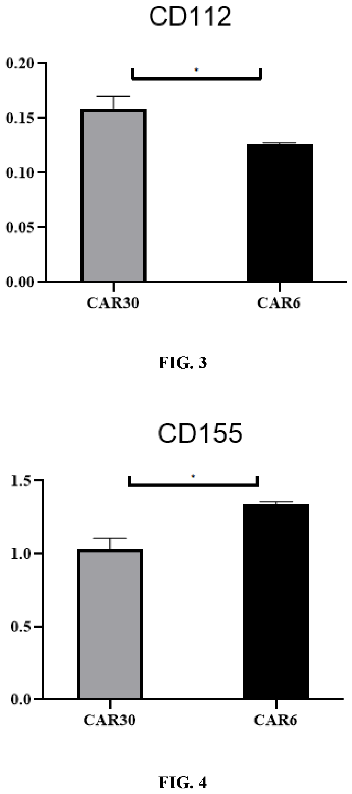

As shown in FIG. 3, the horizontal axis represents different CAR-CHO, and the vertical axis represents the relative MFI values for CD112. The results show that CAR6 exhibited weaker binding to CD112.

Herein, Table 2 shows the statistical analysis results of binding of CAR6 and CAR30 to CD112, respectively.

**Table 2**

| CAR | CD112/TIGIT | Significance analysis |
|---|---|---|
| CAR6 | 0.13 | 0.0349 |
| CAR30 | 0.16 | |

As shown in Table 2 above, the first column represents CAR molecules, the second column represents the relative MFI values for CD112, where a lower value indicates weaker binding to CD112 compared with CAR30, and the third column represents the significance analysis result by t-test.

Conclusion: According to FIG. 3 and Table 2, the results show that CAR6 exhibited significantly reduced binding ability to CD112 compared with the wild type.

FIG. 4 shows the binding of CAR6 and CAR30 to CD155, respectively. As shown in FIG. 4, the horizontal axis represents different CAR-CHO molecules, and the vertical axis represents the relative MFI values for CD155.

Table 3 shows the analysis of binding capacity of four TIGIT mutant peptides to CD155.

**Table 3**

| CAR | CD155/TIGIT | Significance analysis |
|---|---|---|
| CAR6 | 1.34 | 0.0142 |
| CAR30 | 1.03 | |

As described in Table 3, the first column represents the CAR molecules, the second column represents the relative MFI values for CD155, where a higher value indicates stronger binding to CD155 compared with CAR30, and the third column represents the significance analysis result by t-test.

Conclusion: According to FIG. 4 and Table 3, the results show that the binding ability of CAR6 to CD155 was significantly increased compared with the wild type.

Summary: Comprehensive analysis confirms that CAR6 exhibited significantly increased binding to CD155 and significantly decreased binding ability to CD112 compared with the wild type.

In conclusion, the mutant TIGIT in which the amino acid at position 48 is mutated to W (mut6) is a peptide with greater specificity.

2) Further, the functional performance of the above mutant peptides on T cells was validated by *in vitro* killing.

CAR-T cells were prepared using the protocol in Example 1. Viruses containing CAR6 and CAR30 vector genes were prepared for viral transduction. MDA-MB-231-CD155(-)-Luc-GFP was used as target cells, which do not express CD155 but express CD112. The cells containing CAR6 and CAR30 prepared above were used for killing at an effector-to-target ratio of 1:1. CAR-T cells prepared from three batches (three kinds of buffy coats) were used to detect positive rates of CAR-T. Three *in vitro* functional evaluations were performed to validate the *in vitro* killing of CD155-positive and CD155-negative but CD112-positive cells using the mutant peptide as an extracellular recognition domain. The killing results were statistically analyzed by t-test.

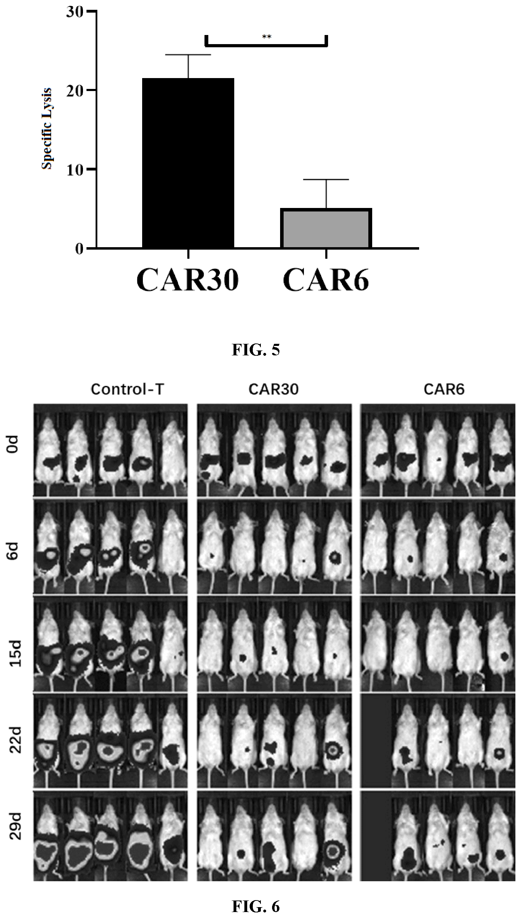

FIG. 5 shows the killing of MDA-MB-231-CD155(-)-Luc-GFP by CAR6 and CAR30. The results are statistical results of *in vitro* killing by CAR-T prepared from three batches. The horizontal axis represents different CAR molecules, and the vertical axis represents the *in vitro* specific lysis (killing percentage, %) of MDA-MB-231-CD155(-)-Luc-GFP. The results show that the killing of MDA-MB-231-CD155(-)-Luc-GFP by CAR6 was significantly decreased. * represents P < 0.05, ** represents P < 0.01.

Conclusion: In summary, the killing of MDA-MB-231-CD155(-)-Luc-GFP by CAR6 was significantly decreased. The TIGIT peptide corresponding to CAR6 is a preferred peptide in the present application, i.e., the amino acid at position 48 can be mutated to W (mut6).

3) *In vivo* Effectiveness of TIGIT Mutant Peptides as Extracellular Recognition Domain of CAR Structures

CD155 expression in target cells was detected by flow cytometry. Table 4 shows CD155 expression in HT1376-Luc-GFP and HPAC-Luc-GFP cells.

**Table 4**

| Cell | CD155 Expression Positive Rate |
|---|---|
| MDA-MB-231-Luc-GFP | 100% |
| HT-1376-Luc-GFP | 100% |
| HPAC-Luc-GFP | 100% |

Female NCG mice aged 6-8 weeks were intravenously injected with MDA-MB-231-Luc-GFP cells at 3×10⁵ cells per mouse at the tail of mice to establish a tumor-bearing model. On day 3 post-tumor engraftment, the above CAR30 and CAR6 T cells were intraperitoneally injected at 5×10⁵ CAR-T cells per mouse. Control T group was infused with the same total number of T lymphocytes on day 3. Results are shown in FIG. 6, demonstrating the *in vivo* efficacy evaluation of CAR structures with the above mutant peptide as the extracellular recognition domain in the MDA-MB-231 tumor-bearing models.

Results show that there was no significant difference in the efficacy between CAR30 and CAR6 in the MDA-MB-231-Luc-GFP tumor-bearing models, demonstrating that the TIGIT mutant peptide of the present application, when used as the extracellular recognition domain of the CAR structure, can achieve effective *in vivo* killing of target cells targeting CD155, with effectiveness superior to that of the wild-type TIGIT peptide.

### Example 3: Application Validation of Mutant Peptides as Extracellular Antigen binding domains of CAR Structures in Different CAR Structures

Different CAR structures were designed using Mut6, and native hinge of TIGIT or 7h (CD7 hinge region)/G4h/8h (CD8 hinge region), different transmembrane domain CD28TM (CD28 transmembrane domain), ICOSTM (ICOS transmembrane domain) or CD8TM (CD8 transmembrane domain), the costimulatory domain from different costimulatory receptor molecules, including CD28, ICOS, CD134 and 4-1BB, different CD3, including Z, Z(1) or Z(3), and second-generation and third-generation CAR structural designs were selected, to form the CAR structures presented in Table 6 below, wherein Z, Z(1), or Z(3) are different mutant peptides of CD3ζ and are used as intracellular signaling sequence of CAR structures in different published documents. In Table 5, except for "-" in "4-1BB", which is part of the molecule name, all other "-" indicate linkage via a peptide bond or linker (e.g., short peptide). Structures without "-" may be linked directly or linked via a linker (e.g., short peptide).

**Table 5**

| CAR No. | Structure |
|---|---|
| CAR6 | mut6-28TM-28Z(1) |
| CAR31 | mut6-ICOSTM-ICOSZ |
| CAR32 | mut6-8TM-134Z |
| CAR33 | mut6-28TM-CD28-4-1BBZ(3) |
| CAR34 | mut6-7h-28TM-28Z(1) |
| CAR35 | mut6-G4h-28TM-CD28-4-1BBZ(3) |
| CAR36 | mut6-8h-8TM-BBZ |

Exemplary amino acid sequences used for various elements in Table 5 are set forth in the Sequence Listing at the end of the text. Herein, the amino acid sequence of Mut6 is set forth in SEQ ID NO:7; the amino acid sequence of 28TM (a transmembrane sequence derived from human CD28, also denoted as CD28TM, which is a transmembrane domain) is set forth in SEQ ID NO:36; the amino acid sequence of 28 (an intracellular signaling sequence derived from human CD28, also denoted as CD28 in the present application) is set forth in SEQ ID NO:37; the amino acid sequence of Z(1) (an intracellular signaling sequence derived from human CD3ζ) is set forth in SEQ ID NO:42; the amino acid sequence of ICOSTM (a transmembrane sequence derived from human ICOS) is set forth in SEQ ID NO:44; the amino acid sequence of ICOS (an intracellular signaling sequence derived from human ICOS) is set forth in SEQ ID NO:46, the amino acid sequence of Z (an intracellular signaling sequence derived from human CD3ζ) is set forth in SEQ ID NO:41; the amino acid sequence of CD28-4-1BB (a costimulatory signal combination consisting of an intracellular signaling sequence of CD28 and an intracellular signaling sequence of 4-1BB) is set forth in SEQ ID NO:40; the amino acid sequence of Z(3) (an intracellular signaling sequence derived from human CD3ζ) is set forth in SEQ ID NO:43; the amino acid sequence of 7h (a hinge sequence derived from human CD7) is set forth in SEQ ID NO:33; the amino acid sequence of 8h (a hinge sequence derived from human CD8) is set forth in SEQ ID NO:32; the amino acid sequence of G4h (a hinge sequence derived from IgG4) is set forth in SEQ ID NO:34; the amino acid sequence of 8TM (transmembrane sequence derived from human CD8, also denoted as CD8TM) is set forth in SEQ ID NO:35; the amino acid sequence of BB (an intracellular signaling sequence derived from human 4-1BB (also known as CD137)) is set forth in SEQ ID NO:38; and the amino acid sequence of 134 (an intracellular signaling sequence derived from human CD134) is set forth in SEQ ID NO:45.

MDA-MB-231-Luc-GFP, HPAC-Luc-GFP, and HT-1376-Luc-GFP cells expressing CD155 for different indications were used as positive target cells. Herein, MDA-MB-231-Luc-GFP corresponds to human breast cancer indication, HPAC-Luc-GFP corresponds to human pancreatic cancer indication, and HT-1376-Luc-GFP corresponds to human bladder cancer indication. CAR-T cells were plated onto target cells at a ratio of 1:1, and the killing ability of different CAR-T against target cells was detected by ACEA xCELLigence RTCA MP instrument, wherein the experimental steps were performed according to the instructions of the instrument. The principle of ACEA xCELLigence RTCA MP is that data of tumor cells attached to the bottom of wells are recorded every 15 minutes with resistance index, and the proliferation or death of adherent target cells is assessed based on the resistance index. The results are analyzed using the resistance index by the formula as follows: Cell killing rate (%) = (Cell Index value of Control group - Cell Index value of Experimental group) / (Cell Index value of Control group) × 100%.

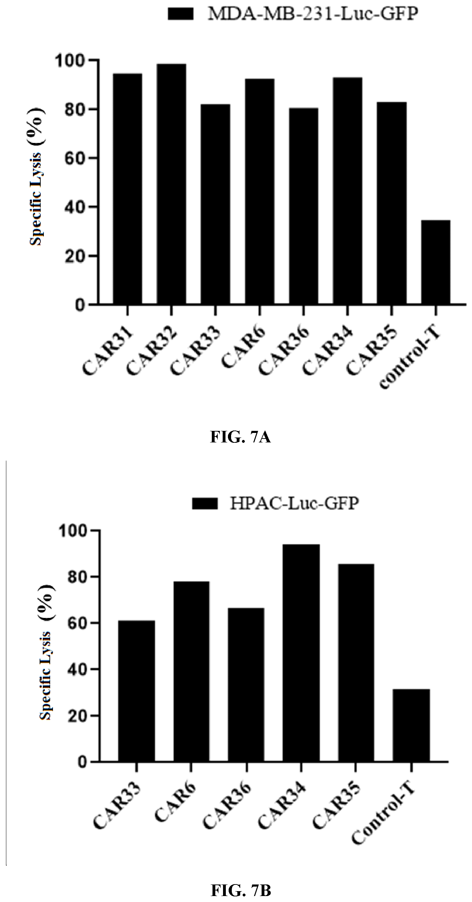

Results are shown in FIGS. 7A-7C, wherein the horizontal axis represents different CARs and the vertical axis represents the killing of different CARs against MDA-MB-231-Luc-GFP, HPAC-Luc-GFP, and HT1376-Luc-GFP, respectively. The results show that mutated TIGIT, as the extracellular recognition domains of multiple CAR structures, exhibited a significant killing effect on a variety of CD155-expressing malignant tumors, such as breast cancer, pancreatic cancer, and bladder cancer.

Furthermore, cytokine detection was performed on the above CAR-T after activation by the target cells. IFN-γ cytokine detection was carried out using ELISA with a kit from BD Biosciences. The catalog number of the detection kit is 555142, and the production lot number is 6266958. For specific steps, refer to the kit instructions. The cytokine secretion results are shown in Table 6 below, which shows IFN-γ secretion of the seven CARs with different structures during killing of the three target cells.

**Table 6**

| Cytokine Secretion | MDA-MB-231-Luc-GFP | HT-1376-Luc-GFP | HPAC-Luc-GFP |
|---|---|---|---|
| CAR31 | 23371.516 | / | / |
| CAR32 | 45592.297 | / | / |
| CAR33 | / | 2582.139 | 1239.714 |
| CAR6 | / | 16583.42 | 9826.8 |
| CAR36 | 23333.294 | / | 6779.085 |
| CAR34 | 75127.335 | / | 14634.484 |
| CAR35 | 47706.754 | / | 16733.595 |
| Control-T | 0 | 0 | 0 |

As shown in Table 6 above, in experiments for different target cells, the Control-T group exhibited no cytokine secretion, while the CAR-T cell groups all showed significant cytokine secretion.

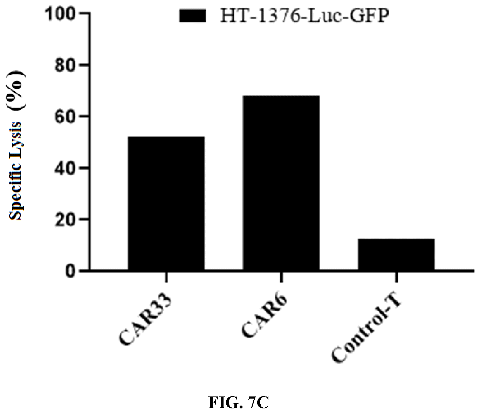

FIG. 7 and Table 6 above, through analysis of *in vitro* killing and cytokine secretion by the CAR-T cells targeting various malignant tumor indications, using seven different CAR structures with the mutated TIGIT peptide as the extracellular recognition domain, demonstrate that TIGIT, as an extracellular recognition domain, is compatible with all the different CAR structures, and exhibits significant recognition and functional killing effects against various solid tumor cells, including, but not limited to, CD155-expressing malignant tumors such as breast cancer, pancreatic cancer, and bladder cancer.

### Example 4: Application Validation of Mutant Peptides as Extracellular Antigen binding domains of CAR Structures in Dual-CAR Structures

Based on CAR6, different dual-CAR structures were designed, targeting PSCA, CD123, and CEA, respectively, in combination with the TIGIT mutant peptide. Moreover, during the design of the CAR structures, different hinges, namely, 8h, G4h, and 7h, different transmembrane domains CD8TM and CD28TM, different costimulatory signaling domains 4-1BB, 2B4, CD28, and CD28-4-1BB, and different CD3Z, namely, Z and Z(3), were evaluated in various signal combinations. The CAR structures are listed in Table 7 below.

**Table 7**

| CAR | Structure |
|---|---|
| CAR36 | PSCA(1)-8h-8TM-BBZ-P2A-mut6-28TM-28 Z(1) |
| CAR37 | CEA-8h-8TM-BBZ-P2A-mut6-28TM-28 Z(1) |
| CAR42 | CEA-8h-8TM-BBZ |
| CAR38 | CD123-8h-8TM-2B4Z-P2A- mut6-28TM-28 Z(1) |
| CAR39 | PSCA(9)-G4h-28TM-28-4-1BBZ(3)-P2A-mut6-28TM-28 Z(1) |
| CAR40 | PSCA(9)-G4h-28TM-28-4-1BBZ(3)-P2A-mut6-28TM-28 Z(1) |
| CAR41 | PSCA(9)-7h-28TM-28Z-P2A- mut6-28TM-28 Z(1) |

The above CAR-T cells were prepared using the method in Example 1. Sequence information for each element is summarized in the Sequence Listing at the end of the text. Herein, the amino acid sequence of PSCA(1) (an extracellular recognition sequence for recognizing PSCA) is set forth in SEQ ID NO:27; the amino acid sequence of 8h is set forth in SEQ ID NO:32; the amino acid sequence of 8TM is set forth in SEQ ID NO:35; the amino acid sequence of BB is set forth in SEQ ID NO:38; the amino acid sequence of Z is set forth in SEQ ID NO:41; the amino acid sequence of P2A is set forth in SEQ ID NO:31; the amino acid sequence of mut6 is set forth in SEQ ID NO:7; the amino acid sequence of 28TM is set forth in SEQ ID NO:36; the amino acid sequence of 28 is set forth in SEQ ID NO:37; the amino acid sequence of Z(1) is set forth in SEQ ID NO:42; the amino acid sequence of CEA (an antigen binding domain that binds to CEA) is set forth in SEQ ID NO:29; the amino acid sequence of CD123 (an antigen binding domain that binds to CD123) is set forth in SEQ ID NO:30; the amino acid sequence of 2B4 is set forth in SEQ ID NO:39; the amino acid sequence of PSCA(9) (an extracellular recognition sequence for recognizing PSCA) is set forth in SEQ ID NO:28; the amino acid sequence of G4h is set forth in SEQ ID NO:34; the amino acid sequence of 28-4-1BB is set forth in SEQ ID NO:40; the amino acid sequence of Z(3) is set forth in SEQ ID NO:43; and the amino acid sequence of 7h is set forth in SEQ ID NO:33.

### 1) Detection of Antigen Expression in Target Cells

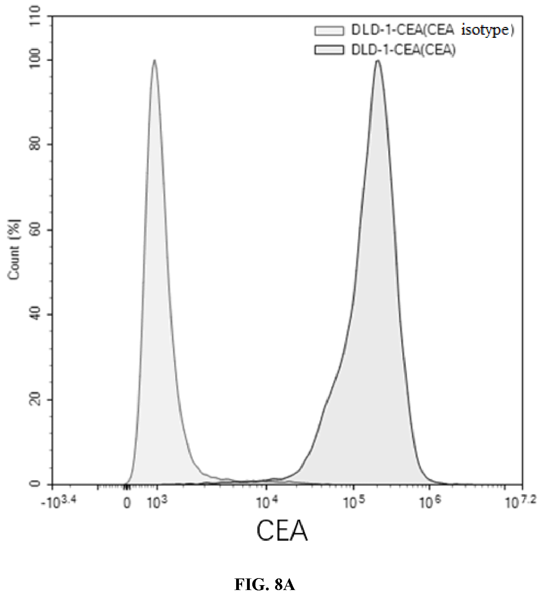

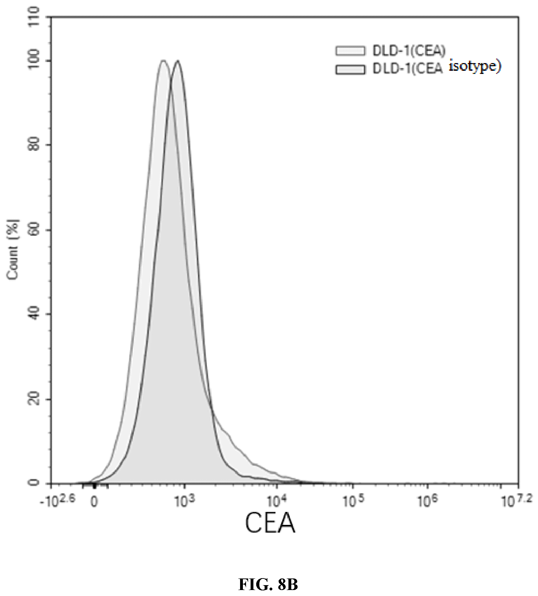

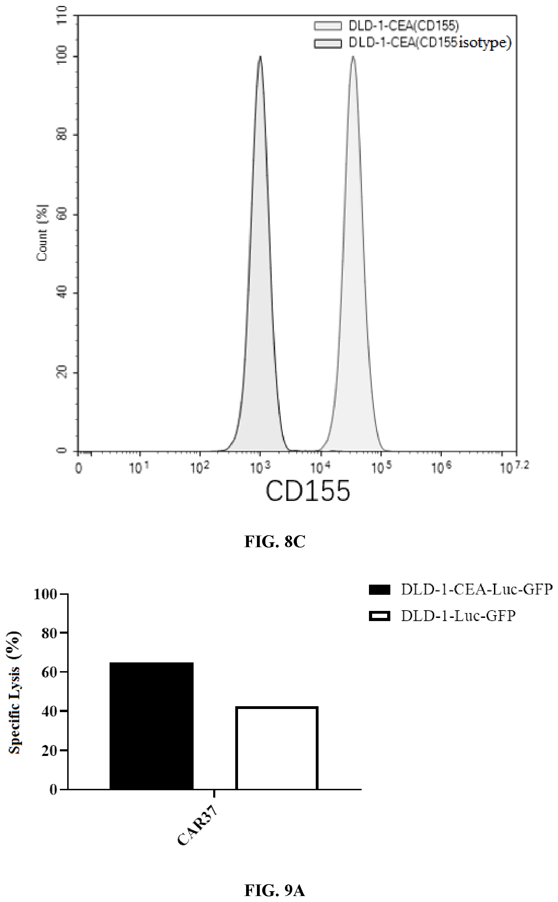

The antigen expression in target cells was detected using flow cytometry. As shown in FIG. 8, the horizontal axis represents the fluorescence intensity of antigen. The positive rate of CEA expression in DLD1-CEA-Luc-GFP was 100%, while DLD1-Luc-GFP showed minimal and weak CEA expression. The positive rate of CD155 expression in DLD1-CEA-Luc-GFP was 100%. Since DLD1-CEA-Luc-GFP and DLD1-Luc-GFP differ only in exogenously expressed CEA molecule, CD155 expression in DLD1-Luc-GFP was also 100%.

For the dual-target CAR-T comprising mutated TIGIT as an extracellular domain designed according to Table 8, dual-target effectiveness validation was performed. The specific CAR-T preparation and killing procedures were described in Example 1 and Example 3. Validation was performed on the dual-target CAR-T targeting CEA×CD155, CD123×CD155, and PSCA×CD155, respectively.

**Table 8: Antigen Expression in Different Target Cells**

| Cell | CD155 Expression | Second Antigen Expression |
|---|---|---|
| Molm13-Luc-GFP | 100% | 100% (CD123) |
| PC3-PSCA-Luc-GFP | 100% | 100% (PSCA) |
| HT-1376-Luc-GFP | 100% | 80% (PSCA) |
| HPAC-Luc-GFP | 100% | 60% (PSCA) |

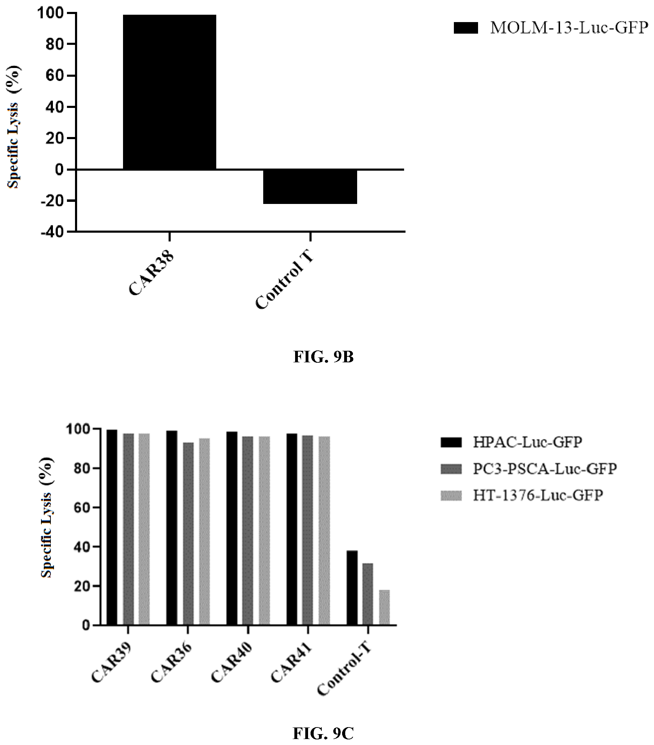

As shown in FIG. 9, CAR37 exhibited significant killing against both DLD1-CEA-Luc-GFP and DLD1-Luc-GFP, while the killing against DLD1-CEA-Luc-GFP was significantly higher than that against DLD1-Luc-GFP, mainly because that the dual-CAR recognized both CEA and CD155 in DLD1-CEA-Luc-GFP cells, resulting in higher killing than the recognition of only a single target antigen CD155 in DLD1-Luc-GFP, demonstrating that the dual-CAR can achieve better *in vitro* efficacy. CAR38 exhibited obvious killing against Molm13-Luc-GFP, demonstrating that CAR38 can exert significant *in vitro* efficacy in hematological malignancies, such as human acute myeloid leukemia tumor cell model. As shown in FIG. 9C, the horizontal axis represents different CARs and the vertical axis represents *in vitro* specific lysis in different cells, wherein the results show that CAR39, CAR36, CAR40, and CAR41 all exhibited significant killing against HPAC-Luc-GFP, PC3-PSCA-Luc-GFP, and HT1376-Luc-GFP cells, with the specific lysis all higher than 50%, demonstrating that different CARs can exert obvious *in vitro* efficacy in various solid tumor cell models.

Furthermore, cytokine detection was performed on the above CAR-T after activation by the target cells. IFN-γ cytokine detection was carried out using ELISA with a kit from BD Biosciences. The catalog number of the detection kit is 555142, and the production lot number is 6266958. For specific steps, refer to the kit instructions. The cytokine secretion results are shown in Table 9 below.

**Table 9: Summary of IFN-γ Cytokine Secretion during in vitro Cell Killing by Five CARs**

| Cytokine Secretion | MOLM-13-Luc-GFP | HPAC-Luc-GFP | PC3-PSCA-Luc-GFP | HT-1376-Luc-GFP |
|---|---|---|---|---|
| CAR40 | / | 25253.992 | 83062.804 | 35472.639 |
| CAR41 | / | 31193.523 | 90640.155 | 45293.378 |
| CAR39 | | 28104.563 | 107455.445 | 40037.06 |
| CAR36 | | 29526.806 | 83801.748 | 47134.635 |
| CAR38 | 8785.705 | | | |
| CT | 0 | 0 | 0 | 0 |

Through the analysis of *in vitro* killing and cytokine secretion of the five CAR-T cells, it is demonstrated that the mutated TIGIT peptide can serve as one of the extracellular recognition domains in dual-CAR and multi-target CAR structures, and is applicable to multiple indications of hematological malignancies and various solid tumors.

### Example 5. Validation of Combined Application of Fusion Protein Constructed with Mutated Peptide and CAR Targeting Non-CD155 Antigens

In addition to serving as an extracellular recognition domain of a single CAR or dual-CAR structures for direct tumor antigen recognition and initiation of T cell killing, the TIGIT mutant peptide in the present application can also be designed to form a TIGIT mutant peptide fusion protein (in the examples and drawings of the present application, it specifically refers to an engineered receptor that does not contain a primary signal transduction domain in the signal transduction domain, such as an engineered receptor consisting of an antigen binding domain, a transmembrane domain, and a costimulatory domain from N-terminus to C-terminus, or an engineered receptor consisting of an antigen binding domain, a hinge region, a transmembrane domain, and a costimulatory domain from N-terminus to C-terminus), and be designed in combination with a CAR structure to remodel the immune microenvironment and improve the effectiveness of the CAR-T cells.
1) The inventors designed mut6-28TM-28, and on this basis, combined it with different CARs to validate the adaptability of the TIGIT mutant peptide to different transmembrane and intracellular signaling domains. The TIGIT mutant peptide exhibited adaptability to a variety of transmembrane and intracellular signaling.
2) The inventors also performed different designs on the CAR structures, and evaluated the adaptability of different targets such as PSCA, CD123, and CEA to the TIGIT mutant peptide fusion protein. The structural designs are shown in Table 10 below.

**Table 10: CAR Structure Design**

| CAR | Structure |
|---|---|
| CAR43 | PSCA(1)-8h-8TM-BBZ-P2A-mut6-28TM-28 |
| CAR44 | PSCA(9)-G4h-28TM-CD28-4-1BBZ(3)-P2A- mut6-28TM-28 |
| CAR45 | CEA-8h-8TM-BBZ-P2A- mut6-28TM-28 |
| CAR42 | CEA-8h-8TM-BBZ |
| CAR46 | CD123-8h-8TM-2B4Z-P2A-mut6-28TM-28 |
| CAR47 | PSCA(9) -G4h-28TM-CD28-4-1BBZ(3)-P2A-mut6-28TM-28 |
| CAR48 | PSCA(9)-7h-28TM-28 Z(1)-P2 A- mut6-28TM-28 |

The sequences of various elements in the above table are set forth in the Sequence Listing at the end of the text. Herein, the amino acid sequence of PSCA(1) is set forth in SEQ ID NO:27; the amino acid sequence of 8h is set forth in SEQ ID NO:32; the amino acid sequence of 8TM is set forth in SEQ ID NO:35; the amino acid sequence of BB is set forth in SEQ ID NO:35; the amino acid sequence of Z is set forth in SEQ ID NO:41; the amino acid sequence of P2A is set forth in SEQ ID NO:31; the amino acid sequence of mut6 is set forth in SEQ ID NO:7; the amino acid sequence of 28TM is set forth in SEQ ID NO:36; the amino acid sequence of 28 is set forth in SEQ ID NO:37; the amino acid sequence of PSCA(9) is set forth in SEQ ID NO:28; the amino acid sequence of G4h is set forth in SEQ ID NO:34; the amino acid sequence of CD28-4-1BB is set forth in SEQ ID NO:40; the amino acid sequence of Z(3) is set forth in SEQ ID NO:43; the amino acid sequence of CEA (an antigen binding domain that binds to CEA) is set forth in SEQ ID NO:29; the amino acid sequence of CD123 (an antigen binding domain that binds to CD123) is set forth in SEQ ID NO:30; the amino acid sequence of 2B4 is set forth in SEQ ID NO:39; the amino acid sequence of 7h is set forth in SEQ ID NO:33; and the amino acid sequence of Z(1) is set forth in SEQ ID NO:42.

The CAR-T cells were prepared using the method in Example 1. A CEA-targeting CAR (CAR42) was selected, and CAR45 containing TIGIT mutant peptide fusion protein was designed. DLD-1-CEA-Luc-GFP and DLD1-Luc-GFP were used as target cells, with an effector-target ratio of 1:1. The *in vitro* killing ability of CAR42 and CAR45 was validated using the method in Example 3 to evaluate the effectiveness of the CAR-T cells expressing the fusion protein.

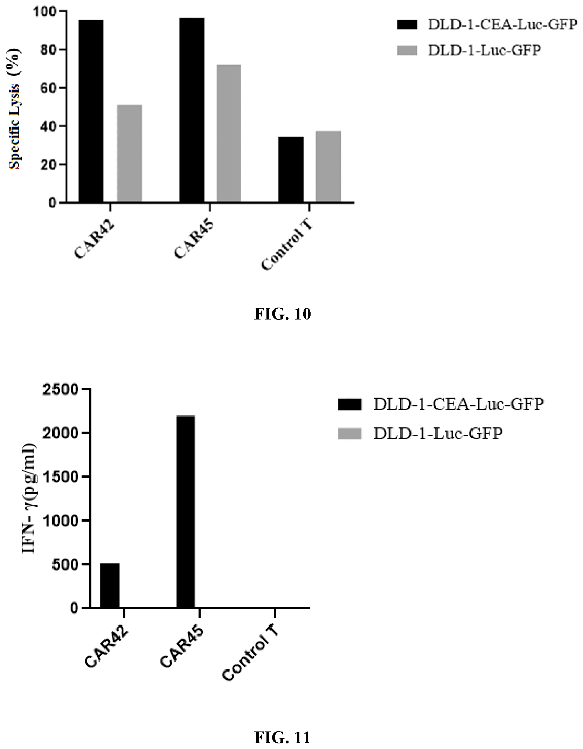

As shown in FIG. 10, the horizontal axis represents CAR42 and CAR45, and the vertical axis represents the specific lysis of the two cells against DLD-1-CEA-Luc-GFP and DLD-1-Luc-GFP. The results show that both CAR42 and CAR45 exhibited significant killing against DLD-1-CEA-Luc-GFP, and the killing of CAR45 against DLD-1-Luc-GFP was significantly higher than that of the CAR42 group, indicating that CAR-T expressing the fusion protein has superior killing efficacy compared with CAR-T that does not express the fusion protein.

As shown in FIG. 11, the horizontal axis represents CAR42 and CAR45, and the vertical axis represents their IFN-γ cytokine secretion. The results show that the cytokine secretion of CAR45 was significantly higher than that of CAR42, demonstrating that the fusion protein formed by CAR combined with the TIGIT mutant peptide can enhance the cytokine secretion during killing.

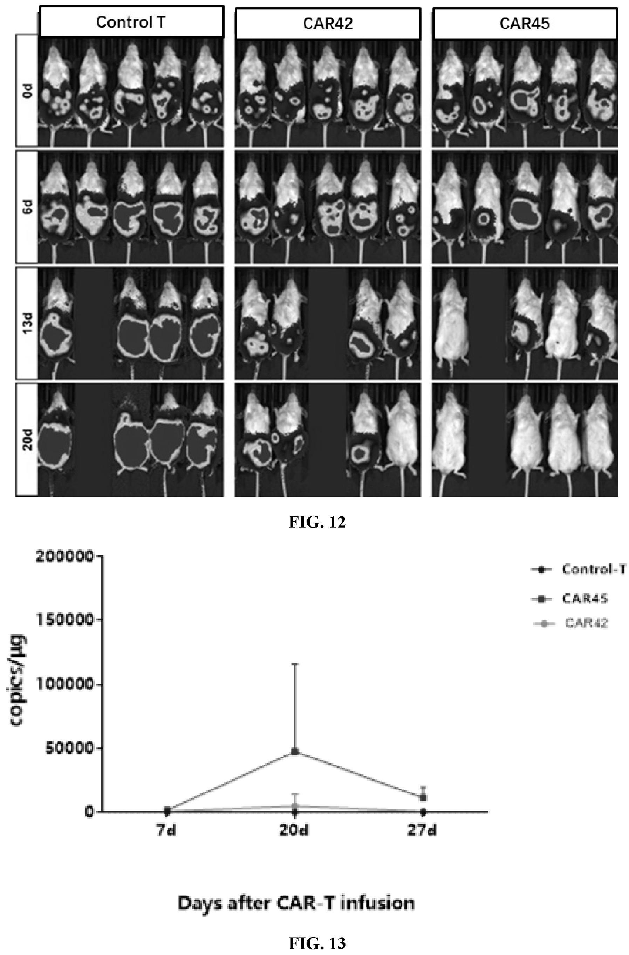

Female NCG mice aged 6-8 weeks were intraperitoneally injected with DLD-1-CEA-Luc-GFP cells at 3×10⁵ cells per mouse in the mouse tail to establish a tumor-bearing model. On day 7 post-tumor engraftment, the above CAR42 and CAR45 T cells were injected via tail vein at 5×10⁵ CAR-T cells per mouse. The Control T group was infused with the same total number of T lymphocytes on day 7. *In vivo* imaging of mice was performed every 7 days after administration to detect the tumor cell retention in the mice. The imaging fluorescence values were statistically analyzed, and the significance was analyzed using t-test. The results are shown in FIG. 12. In the intraperitoneal tumor-bearing efficacy evaluation models, terminal orbital blood sampling was performed on the mice, and the CAR-T copy number in the blood was detected by fluorescence quantitative PCR. The results show that the CAR-T copy number in the blood was significantly higher in CAR45 than in CAR42 on day 7, day 20, and day 27 after CAR-T administration, demonstrating that the fusion protein formed by CAR combined with the TIGIT mutant peptide, namely CAR45, could significantly improve *in vivo* persistence of CAR in models. FIG. 13 shows the blood copy number in the intraperitoneal tumor-bearing evaluation models.

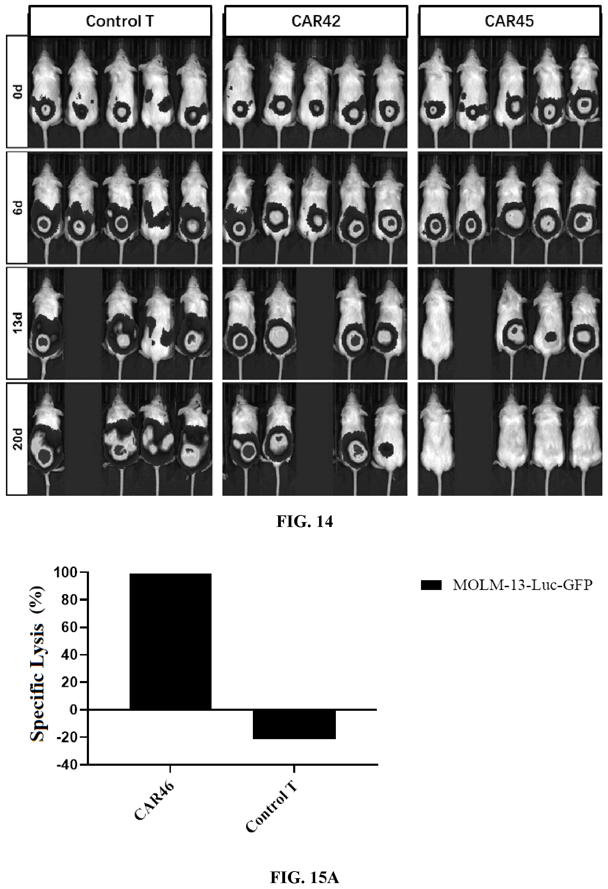

DLD-1-CEA-Luc-GFP was used for subcutaneous tumor establishment. On day 12 post-tumor engraftment, CAR-T cells were infused via tail vein. *In vivo* imaging of mice was performed every 7 days after administration to detect the tumor cell retention in the mice. The imaging fluorescence values were statistically analyzed, and the significance was analyzed using t-test, as shown in FIG. 14.

The results show that the *in vivo* efficacy of CAR45 was significantly better than that of CAR42, demonstrating that CAR45 CAR-T cells, formed from the fusion protein of CAR combined with the TIGIT mutant peptide, can significantly enhance *in vivo* effectiveness. Moreover, the effectiveness is derived from the recognition of the CD155 target by the mutant TIGIT of CAR45, further proving the feasibility of the TIGIT mutant peptide as one of the extracellular recognition domains of multi-target CAR structures.

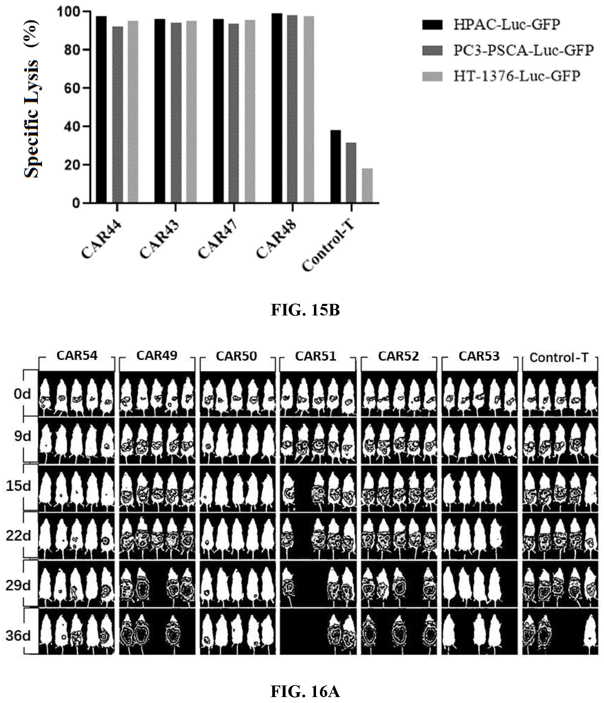

Furthermore, the inventors also validated the applicability of the TIGIT mutant peptide, as one of the extracellular recognition domains targeting CD155 for multi-target CAR products, in various hematological malignancies and solid tumors of multiple indications such as acute myeloid leukemia, pancreatic cancer, and bladder cancer. The horizontal axis represents different CARs, and the vertical axis represents the *in vitro* specific lysis in different cells. As shown in FIG. 15A, CAR46 exhibited significant killing against Molm13-Luc-GFP. As shown in FIG. 15B, the results show that CAR44, CAR43, CAR47, and CAR48 exhibited significant killing against HPAC-Luc-GFP, PC3-PSCA-Luc-GFP, and HT1376-Luc-GFP, demonstrating that dual-target or multi-target CAR-T constructed with the TIGIT mutant peptide as an extracellular recognition domain and targeting CD155 and any other malignant tumor targets can exert significant *in vitro* efficacy in various solid tumor cell models.

Cytokine detection was performed on the above CAR-T after activation by the target cells. IFN-γ cytokine detection was carried out using ELISA with a kit from BD Biosciences. The catalog number of the detection kit is 555142, and the production lot number is 6266958. For specific steps, refer to the kit instructions. The cytokine secretion results are shown in Table 11 below.

**Table 11: Summary of IFN-γ Cytokine Secretion during in vitro Cell Killing by Five CARs**

| Cytokine Secretion | MOLM-13-Luc-GFP | HPAC-Luc-GFP | PC3-PSCA-Luc-GFP | HT-1376-Luc-GFP |
|---|---|---|---|---|
| CAR44 | | 29485.412 | 78915.876 | 44167.508 |
| CAR43 | | 28068.447 | 80210.306 | 51527.378 |
| CAR47 | | 23567.502 | 65871.667 | 31787.232 |
| CAR48 | | 38508.186 | 129411.758 | 53736.732 |
| CAR46 | 13079.088 | | | |
| CT | 0 | 0 | 0 | 0 |

Through the analysis of *in vitro* killing and cytokine secretion of the five CAR-T cells, it is demonstrated that different CARs exhibited obvious recognition and functional killing effects against hematological tumor cell lines and various solid tumor cell lines, namely Molm13-Luc-GFP, HT1376-Luc-GFP, HPAC-Luc-GFP, and PC3-PSCA-Luc-GFP. The TIGIT mutant peptide, as one of extracellular recognition domains of multi-target CAR structures targeting CD155, is applicable in a variety of hematological malignancies and solid tumors.

It should be noted that although the present application takes the CAR-T cells as an example, based on the contents disclosed in the present application, those skilled in the art could obviously deduce that other immune cells, such as NK cells, macrophages, DC cells, and relevant precursor cells, can also be used to express single-target CAR, dual-target or multi-target CAR designed with mutant TIGIT, as well as fusion proteins expressing mutant TIGIT.

The inventors constructed TIGIT single CARs with different CAR structures for the preparation of CAR-NK cells and verified their expression. The results are shown in Table 12.

**Table 12**

| CAR Structure | NK Cell Source | Viral Titer | CAR Expression Positive Rate |
|---|---|---|---|
| mut6-28TM-28Z | B-756 | 4.57E+07 | 76.15% |
| mut6-8h-28TM-28Z | B-771 | 5.88E+08 | 69.74% |
| mut6-8h-8TM-BBZ | B-758 | 7.88E+08 | 70.94% |
| mut6-G4h-28TM-28Z | B-759 | 3.68E+08 | 52.53% |

### Example 6 Specificity and Functional Validation of Truncated TIGIT Comprising Mutation Sites of the Present application

Based on Mut6, truncation was performed to obtain the following truncated sequence (the sequence comprises a polypeptide fragment of positions 33-93 of the amino acid sequence set forth in the reference sequence SEQ ID NO:1):
VTQVNWEQQDQLLAI**W**NADLGWHISPSFKDRVAPGPGLGLTLQSLTVNDTGEY FCIYHTYP (SEQ ID NO:26, wherein the mutant site at position 48 relative to the reference sequence is located at position 16 in this sequence).

The protocol in Example 2 was used to validate the specificity of the truncated TIGIT mutant peptide in the above. The results show that the truncated TIGIT peptide containing the mutant site (C-to-W mutation at position 48 of the reference SEQ ID NO:1) also exhibited weak recognition toward CD112 and superior specific recognition ability for CD155.

The protocols in Example 3 and Example 5 were used to validate the application of the truncated TIGIT in the construction of CAR structures and fusion proteins, as well as in engineered cells, demonstrating that the truncated TIGIT mutant peptide, as an extracellular recognition domain or one of the domains of single-target or multi-target CAR structures targeting CD155, is applicable in a variety of hematological tumors and solid tumors. Moreover, the fusion protein constructed with the truncated TIGIT can be expressed alone or in combination with CAR in engineered cells to exert the function of remodeling the tumor microenvironment.

Based on the results of the truncated TIGIT mutant peptide and the untruncated TIGIT mutant, it can be demonstrated that any TIGIT peptide containing the mutant site we identified (C-to-W mutation at position 48 of the reference SEQ ID NO:1) and the polypeptide fragment corresponding to positions 33-93 of the reference sequence in the TIGIT extracellular portion has superior specific recognition ability for CD155.

### Example 7: Construction of TIGIT Mutant Peptides and CARs

The TIGIT extracellular region was subjected to random mutagenesis, followed by specificity screening for TIGIT mutant peptides through *in vitro* killing based on the principle of molecular recognition.

### 1) Random mutagenesis of the TIGIT extracellular region:

Random mutagenesis was performed on the TIGIT extracellular region using Method 1, obtaining five mutants Mut25 to Mut29 as shown in the sequences at the end the text (their amino acid sequences are set forth in SEQ ID NOs:47 to 51, respectively).

In mutants Mut25 to Mut29, mutation sites relative to the wild-type TIGIT are marked in bold and underlined.

Chimeric antigen receptors (CARs) were constructed using TIGIT as the antigen binding domain (or an extracellular antigen binding domain). Table 13 below shows the correspondence between the CARs and the TIGIT mutants contained therein.

**Table 13: CARs and Antigen Binding Domains Therein**

| Serial No. | Mutant Peptide Name | CAR No. | Viral Titer (TU/mL) |
|---|---|---|---|
| 1 | Mut25 | CAR49 | 2.5E7 |
| 2 | Mut26 | CAR50 | 2.2E8 |
| 3 | Mut27 | CAR51 | 1.07E8 |
| 4 | Mut28 | CAR52 | 5.1E6 |
| 5 | Mut29 | CAR53 | 1.5E8 |
| 6 | WT | CAR54 | 4.34E8 |

2) Construction of CAR-T cells: The mutant peptides obtained in 1) were constructed as an extracellular antigen binding domain into a CAR structure of canonical CD28TM-28Z. Specifically, the CAR structure is: TIGIT mutant peptide-CD28TM-28Z. A vector plasmid of interest containing a gene encoding the CAR (CAR gene) was constructed. The plasmid of interest was transfected into a 293T-derived cell line by the calcium transfection method for virus production. Titer was calculated by a formula as follows: Titer (TU/ml) = 1×10⁵ × positive rate × dilution factor ÷ virus volume × 1000. The viral titers containing the mutant peptide-encoding sequences are shown in Table 13 above.

PBMCs or T cells obtained by Ficoll separation or apheresis, or cells thawed from cryopreserved PBMCs or T cells obtained above, were used for CAR-T cell preparation. The obtained PBMCs or T cells were activated with anti-CD3 and anti-CD28 monoclonal antibodies or activated magnetic beads coated with anti-CD3 and anti-CD28 monoclonal antibodies (CD3/CD28 Dynabeads (40203D; Gibco)), and transduced with the CAR-encoding lentivirus prepared from the aforementioned plasmid of interest to obtain the CAR-T cells.

The sequences of each TIGIT mutant peptide and each element in the CAR structures in the present example are shown in the Sequence Listing at the end of the text. Among them, the amino acid sequence of CD28TM in the canonical CD28TM-28Z is set forth in SEQ ID NO:36; the amino acid sequence of 28 is set forth in SEQ ID NO:37; and the amino acid sequence of Z is set forth in SEQ ID NO:41. The sequence information of each TIGIT mutant peptide involved is as follows: the amino acid sequence of the wild-type TIGIT extracellular region is set forth in SEQ ID NO:1; the amino acid sequence of Mut25 is set forth in SEQ ID NO:47; the amino acid sequence of Mut26 is set forth in SEQ ID NO:48; the amino acid sequence of Mut27 is set forth in SEQ ID NO:49; the amino acid sequence of Mut28 is set forth in SEQ ID NO:50; and the amino acid sequence of Mut29 is set forth in SEQ ID NO:51.

### Example 8: Screening of TIGIT Mutant Peptides

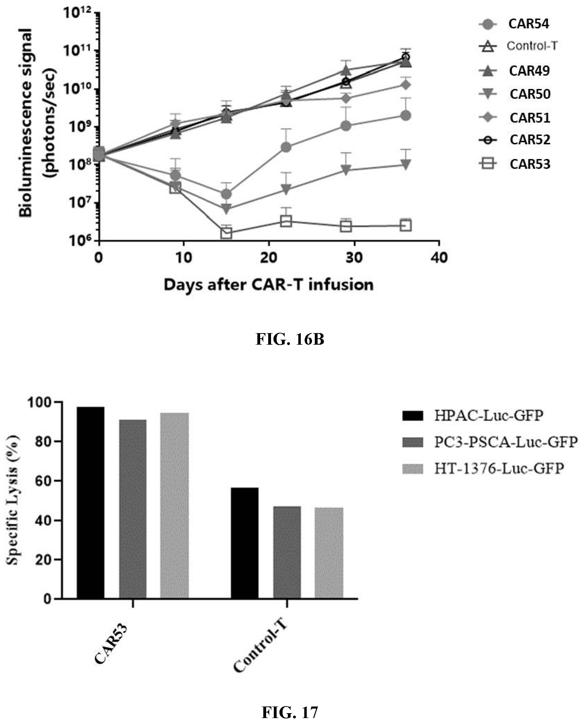

Female NCG mice aged 6-8 weeks were intraperitoneally injected with 3E5 (3*10⁵) MDA-MB-231-Luc-GFP tumor (i.e., CD155-positive human breast cancer cell line) cells and allowed to form tumors for three days to establish tumor-bearing models. On day 3 post-tumor engraftment, 5E5 (5*10⁵) CAR-T cells were infused by intraperitoneal administration. *In vivo* imaging of mice was performed every 7 days after administration to detect the tumor cell retention in the mice. The imaging fluorescence values were statistically analyzed, and the significance was analyzed using t-test. The Control T group was infused with the same total number of T lymphocytes on day 3. Results are shown in FIG. 16A and FIG. 16B. FIG. 16A shows mouse images of *in vivo* tumor killing by CAR-T cells expressing CARs that use different TIGIT mutant peptides as extracellular recognition regions, in immunodeficient mouse models of acute lymphoblastic leukemia; FIG. 16B shows the fluorescence statistical curves. The results show that CAR53 corresponding to the Mut29 mutant peptide is the optimal CAR-T, and CAR-T containing Mut26 also has better *in vivo* killing ability than the wild-type (CAR54), indicating that the mutation contained in Mut29 can significantly improve the effectiveness of the wild-type TIGIT as an extracellular recognition domain.

In addition to the amino acid sequences as set forth in SEQ ID NO:48 and SEQ ID NO:51, Mut26 and Mut29 can be further truncated to retain short functional fragments as amino acid sequences as set forth in SEQ ID NO:52 and SEQ ID NO:53. Based on the same experiments described in the present example, it can be seen that the truncated Mut26 and truncated Mut29 exhibited comparable ability to Mut26 and Mut29, and can serve as extracellular recognition domains to construct CAR structures. The CAR structure may consist of an extracellular recognition domain, a hinge structure, a transmembrane structure, and an intracellular signaling domain, or an extracellular recognition domain, a transmembrane structure, and an intracellular signaling domain. Engineered immune cells expressing CARs constructed with the truncated Mut26 and truncated Mut29, such as CAR-T and CAR-NK, exhibited effective *in vivo* killing against CD155-positive solid tumors.

### Example 9: In vitro Functional Validation of CAR-T Cells Using TIGIT Mutant Peptides as Antigen Binding Domains

Based on Mut29 mutant peptide, different CAR structures were designed, and different hinges including 7h, G4h, and 8h, different transmembrane domains including CD28TM and CD8TM, different costimulatory signaling domains including CD28, 4-1BB, CD28-4-1BB, different CD3 including Z and z, and second-generation and third-generation CAR structural designs were selected, to form different CAR structures presented in Table 14. The sequences of various CAR structural elements are listed in the Sequence Listing at the end of the text. Herein, the amino acid sequence of Mut29 is set forth in SEQ ID NO:51; the amino acid sequence of 28TM (i.e., CD28TM, derived from human CD28 transmembrane sequence) is set forth in SEQ ID NO:36; the amino acid sequence of 28 (i.e., co-stimulatory receptor molecule CD28, derived from human CD28 intracellular signaling sequence) is set forth in SEQ ID NO:37; the amino acid sequence of Z or z is set forth in SEQ ID NO:41; the amino acid sequence of WT is set forth in SEQ ID NO:1; the amino acid sequence of 8h (i.e., hinge region derived from human CD8 hinge sequence) is set forth in SEQ ID NO:32; the amino acid sequence of 8TM (i.e., CD8TM, transmembrane domain derived from human CD8 transmembrane sequence) is set forth in SEQ ID NO:35; the amino acid sequence of BB (i.e., 4-1BB, co-stimulatory receptor molecule derived from 4-1BB or CD137 intracellular signaling sequence) is set forth in SEQ ID NO:38; the amino acid sequence of 7h (i.e., hinge region derived from human CD7 molecule) is set forth in SEQ ID NO:33; the amino acid sequence of G4h (i.e., G4h hinge region) is set forth in SEQ ID NO:34; and the amino acid sequence of 28-4-1BB (i.e., CD28-4-1BB intracellular signaling) is set forth in SEQ ID NO:40.

**Table 14: Correspondence Between CAR Structures and Names**

| **CAR** | **Structure** |
|---|---|
| CAR53 | Mut29-28TM-28z |
| CAR54 | WT-28TM-28z |
| CAR55 | Mut29-8h-8TM-BBZ |
| CAR56 | Mut29-7h-28TM-28z |
| CAR57 | Mut29-G4h-28TM-28-4-1BBZ |

HPAC-Luc-GFP, PC3-PSCA-Luc-GFP, and HT1376-Luc-GFP cells expressing CD155 for different indications were used as positive target cells. Herein, HPAC-Luc-GFP corresponds to human pancreatic cancer indication, PC3-PSCA-Luc-GFP corresponds to human prostate cancer indication, and HT-1376-Luc-GFP corresponds to human bladder cancer indication. CAR-T cells were plated onto target cells at a ratio of 1:1, and the killing ability of different CAR-T against the target cells was detected by ACEA xCELLigence RTCA MP instrument, wherein the experimental steps were performed according to the instructions of the instrument. The principle of ACEA xCELLigence RTCA MP is that data of tumor cells attached to the bottom of wells are recorded every 15 minutes with resistance index, and the proliferation or death of adherent target cells is assessed based on the resistance index. The results are analyzed using the resistance index by the formula as follows: Cell killing rate (%) = (Cell Index value of Control group - Cell Index value of Experimental group) / (Cell Index value of Control group) × 100%.

Results are shown in FIG. 17, showing *in vitro* killing of three target cells by CAR53 CAR-T cells expressing CARs with the TIGIT mutant peptide as a recognition domain. The horizontal axis represents killing of different target cells by CAR53, and the vertical axis represents *in vitro* specific lysis. The results show that CAR53 exhibited a significant killing effect on HPAC-Luc-GFP, PC3-PSCA-Luc-GFP, and HT1376-Luc-GFP, demonstrating that CAR53 exerts the killing function in different target cells.

Furthermore, cytokine detection was performed on the CAR-T after activation by the target cells. IFN-γ cytokine detection was carried out using ELISA with a kit from BD Biosciences. The catalog number of the detection kit is 555142, and the production lot number is 6266958. For specific steps, refer to the kit instructions. The cytokine secretion results are shown in Table 15 below.

**Table 15: IFN-γ Cytokine Secretion during Killing of Three Target Cells by CAR53**

| | HPAC-Luc-GFP | PC3-PSCA-Luc-GFP | HT-1376-Luc-GFP |
|---|---|---|---|
| CAR53 | 94331.684 | 58256.313 | 118204.399 |
| CT | 132.826 | 352.554 | 386.588 |

It can be seen that IFN-γ cytokine secretion during the *in vitro* killing of the three target cells by CAR53 was significantly higher than that of the control group, demonstrating that CAR53 exerted effector function in different target cells.

In summary, CARs using the TIGIT mutant peptides as recognition domains exhibit excellent functions, and the constructed CAR-T cells have a killing effect on the CD155-expressing solid tumors, such as pancreatic cancer, prostate cancer, and bladder cancer.

CD155 expression in the target cells was detected by flow cytometry, with results as shown in Table 16.

**Table 16: CD155 Expression in HT1376-Luc-GFP and HPAC-Luc-GFP Cells**

| Cell | CD155 Expression Positive Rate |
|---|---|
| MDA-MB-231-Luc-GFP | 100% |
| HT-1376-Luc-GFP | 100% |
| HPAC-Luc-GFP | 100% |
| PC3-Luc-GFP | 100% |

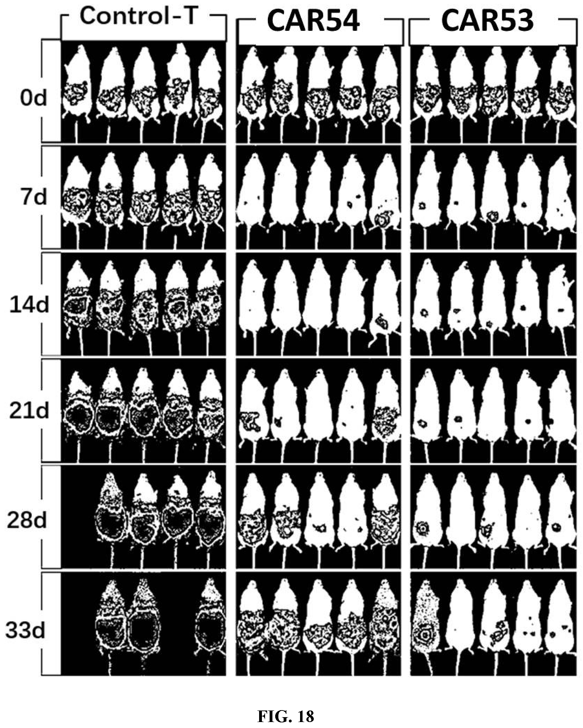

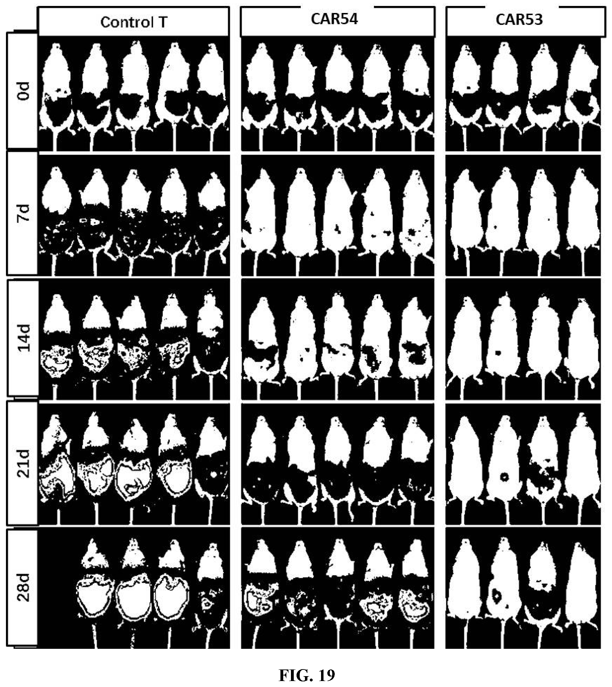

Female NCG mice aged 6-8 weeks were intraperitoneally injected with 3E5 tumor cells of HPAC-Luc-GFP and HT1376-Luc-GFP (HPAC and HT1376 cell lines were purchased from Beijing Beina Chuanglian Biotechnology Research Institute) and allowed to form tumors for three days to establish tumor-bearing models. On day 3 post-tumor engraftment, 5E5 CAR-T cells were infused by intraperitoneal administration. *In vivo* imaging of mice was performed every 7 days after administration to detect the tumor cell retention in the mice. The imaging fluorescence values were statistically analyzed, and the significance was analyzed using t-test. The Control T group was infused with the same total number of T lymphocytes on day 7. Results are shown in FIG. 18 and FIG. 19, illustrating the validation of effectiveness of CAR-T using the mutant TIGIT peptide as an extracellular recognition domain against pancreatic cancer and bladder cancer, respectively. It can be seen that after intraperitoneal tumor establishment using HPAC-Luc-GFP and HT1376-Luc-GFP, respectively, CAR53 exhibited significantly superior *in vivo* efficacy to CAR54, demonstrating that CAR-T constructed with the mutant TIGIT peptide of the present application as the extracellular recognition domain exhibited better effectiveness against CD155-expressing pancreatic cancer and bladder cancer than the wild-type TIGIT peptide. The TIGIT peptide of the present application can serve as an extracellular recognition domain of CAR structures for use in immune cell therapy of CD155-expressing cells.

In addition to being applied to the 28TM-28z structure of CAR53, the mutant TIGIT peptide of the present application is also applicable to various CAR structure combinations. Applicability of the TIGIT mutant peptide to the CAR structure combinations is validated herein using 8h-8TM-BBZ, 7h-28TM-28z, and G4h-28TM-28-4-1BBZ as examples.

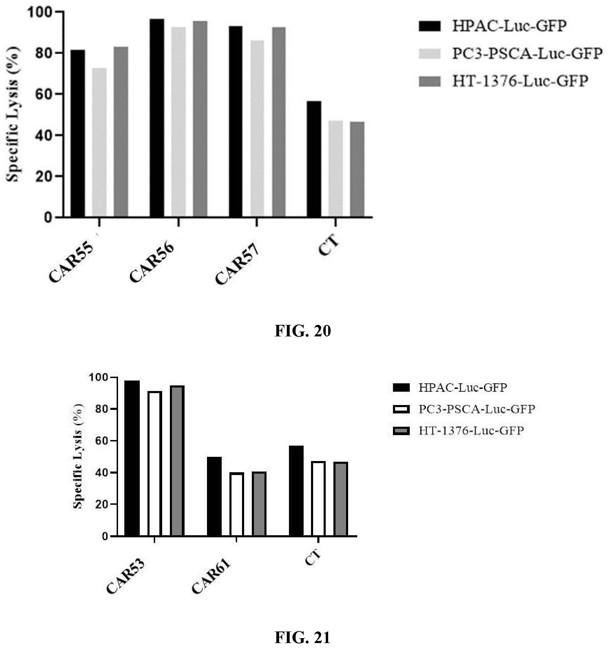

CAR-T cells of CAR55, CAR56, and CAR57 were prepared using the aforementioned methods. Using HT1376-Luc-GFP, PC3-Luc-GFP, and HPAC-Luc-GFP respectively as target cells at an effector-to-target ratio of 1:1, the killing of the target cells by CAR-T was detected after 24 hours of co-incubation with the target cells to validate the effectiveness of the mutant TIGIT peptide of the present application under different CAR structures against the CD155-expressing bladder cancer, prostate cancer, and pancreatic cancer. Results are shown in FIG. 20, illustrating killing of malignant tumors of different indications by the CAR-T cells constructed with the TIGIT mutant peptide as an extracellular recognition domain in different CAR structures. Furthermore, cytokine detection was performed on the above CAR-T (CAR55, CAR56, and CAR57) after activation by the target cells. IFN-γ cytokine detection was carried out using ELISA with a kit from BD Biosciences. The catalog number of the detection kit is 555142, and the production lot number is 6266958. For specific steps, refer to the kit instructions. The cytokine secretion results are shown in Table 17 below.

**Table 17: IFN-γ Cytokine Secretion during Killing of Three Target Cells by Three CARs with Different Structures**

| | HPAC-Luc-GFP | PC3- Luc-GFP | HT-1376-Luc-GFP |
|---|---|---|---|
| CAR55 | 11816.009 | 13474.253 | 16909.457 |
| CAR56 | 32565.975 | 39121.981 | 28343.516 |
| CAR57 | 31046.358 | 34726.952 | 32999.588 |
| CT | 132.826 | 352.554 | 386.588 |

FIG. 20 and Table 17 demonstrate that Mut29, as an optimal mutant peptide, when combined with different hinges 7h, G4h and 8h, different transmembrane domains CD28TM and CD8TM, different costimulatory signaling domains CD28, 4-1BB, and CD28-4-1BB, different CD3 Z and z, as well as second-generation and third-generation CAR structures, can exert *in vitro* killing function and effector function in different target cells, namely PC3-Luc-GFP, HT1376-Luc-GFP, and HPAC-Luc-GFP.

Based on the above, those skilled in the art could reasonably deduce that the mutant TIGIT peptide of the present application can serve as an extracellular recognition domain of CAR structures, and the therapeutic immune cells constructed or products or medicaments comprising the therapeutic immune cells can exert an effective killing effect on the CD155-expressing malignant tumor cells. In some embodiments, therapeutic immune cells refer to CAR-T cells; in some embodiments, therapeutic immune cells refer to CAR-NK cells; and in some embodiments, therapeutic immune cells refer to CAR-macrophages and the like. In some embodiments, the CD155-expressing malignant tumor cells, in addition to including breast cancer, bladder cancer, prostate cancer and pancreatic cancer, further include brain glioma, melanoma, cholangiocarcinoma, non-small cell lung cancer, colorectal cancer and the like.

Mut26, Mut29, truncated Mut26, and truncated Mut29 as described above can also be combined with various CAR structures, including but not limited to hinges 7h, G4h, and 8h, different transmembrane domains CD28TM and CD8TM, different costimulatory signaling domains CD28, 4-1BB, and CD28-4-1BB, different CD3 Z and z, as well as second-generation and third-generation CAR structures, and can exert the *in vitro* killing function and effector function in various CD155-positive tumors such as prostate cancer (PC3-Luc-GFP), bladder cancer (HT1376-Luc-GFP), pancreatic cancer (HPAC-Luc-GFP), and acute lymphoblastic leukemia.

Based on the same experiments described in the present example, it can be seen that the mutant TIGIT peptide of the present application, such as the truncated peptides set forth in SEQ ID NO:52 (truncated Mut26) and SEQ ID NO:53 (truncated Mut29), can serve as extracellular recognition domains of CAR structures, and the constructed therapeutic immune cells or products or medicaments comprising the therapeutic immune cells can exert an effective killing effect on the CD155-expressing malignant tumor cells. In some embodiments, the therapeutic immune cells refer to CAR-T cells; in some embodiments, the therapeutic immune cells refer to CAR-NK cells; in some embodiments, the therapeutic immune cells refer to CAR-macrophages and the like. In some embodiments, the CD155-expressing malignant tumor cells, in addition to including breast cancer, bladder cancer, prostate cancer and pancreatic cancer, further include brain glioma, melanoma, cholangiocarcinoma, non-small cell lung cancer, colorectal cancer and the like.

### Example 10: Application Validation of Mutant Peptides as Antigen Binding Domains in CARs or Fusion Proteins

In addition to serving as an extracellular recognition domain of single-CAR or dual-CAR structures for direct tumor antigen recognition and initiation of T cell killing, the TIGIT mutant peptides described in the present application can also be designed to form engineered receptors or fusion proteins comprising the TIGIT mutant peptides in the antigen binding domain (in the examples of the present application, fusion proteins specifically refer to engineered receptors without a primary signal transduction domain in the signal transduction domain, such as engineered receptors consisting of an antigen binding domain, a transmembrane domain, and a costimulatory domain from N-terminus to C-terminus, or engineered receptors consisting of an antigen binding domain, a hinge region, a transmembrane domain, and a costimulatory domain from N-terminus to C-terminus), and be designed in combination with CAR structures to remodel the immune microenvironment and enhance effectiveness of the CAR-T cells.
1) The inventors designed the Mut29-28TM-28 fusion protein, and on this basis, designed structures combining CARs targeting different target proteins with the fusion protein to validate the adaptability of the TIGIT mutant peptide to different transmembrane and intracellular signaling domains. The TIGIT mutant peptide exhibited adaptability to a variety of transmembrane and intracellular signaling domains.
2) The inventors also made different designs for the CAR structures and considered the adaptability of the CARs targeting different targets such as CD123 and CEA to the engineered receptors comprising the TIGIT mutant peptide in the antigen binding domain, wherein the engineered receptors are fusion proteins without a primary signal transduction domain. The specific structural designs are shown in Table 18 below. The sequences of various elements involved in the CAR structures are shown in the Sequence Listing at the end of the text, wherein the amino acid sequence of CEA (i.e., an antigen binding domain that binds to CEA) is set forth in SEQ ID NO:29; the amino acid sequence of 8h is set forth in SEQ ID NO:32; the amino acid sequence of 8TM is set forth in SEQ ID NO:35; the amino acid sequence of BB is set forth in SEQ ID NO:38; the amino acid sequence of Z or z is set forth in SEQ ID NO:41; the amino acid sequence of P2A is set forth in SEQ ID NO:31; the amino acid sequence of Mut29 is set forth in SEQ ID NO:51; the amino acid sequence of 28TM is set forth in SEQ ID NO:36; the amino acid sequence of 28 is set forth in SEQ ID NO:37; the amino acid sequence of CD123 (i.e., an antigen binding domain that binds to CD123) is set forth in SEQ ID NO:30; and the amino acid sequence of 2B4 (i.e., 2B4 intracellular signal) is set forth in SEQ ID NO:39.

**Table 18: CAR Structural Designs**

| CAR | Structure |
|---|---|
| CAR59 | CEA-8h-8TM-BBZ-P2A-mut5-28TM-28 |
| CAR60 | CD123-8h-8TM-2B4z-P2A-mut5-28TM-28 |
| CAR58 | CEA-8h-8TM-BBZ |
| CAR53 | Mut29-28TM-28z |
| CAR61 | Mut29-28TM-28 |

CAR-T cells were prepared using the protocol in Example 7. Comparison was made between the effectiveness of a stand-alone fusion protein using the mutant TIGIT extracellular domain as the antigen binding domain and that of a CAR using the mutant TIGIT extracellular domain as the antigen binding domain. The results are shown in FIG. 21, presenting the *in vitro* killing of three target cells corresponding to different indications by CAR53 and CAR61. Table 19 shows the IFN-γ cytokine secretion by CAR53 and CAR61 during *in vitro* killing experiments.

**Table 19**

| | HPAC-Luc-GFP | PC3-PSCA-Luc-GFP | HT-1376-Luc-GFP |
|---|---|---|---|
| CAR53 | 94331.684 | 58256.313 | 118204.399 |
| CAR61 | 0 | 156.922 | 256.259 |
| CT | 132.826 | 352.554 | 386.588 |

As can be found from FIG. 21 and Table 19, CAR61, prepared by expressing in T cells a fusion protein formed by the mutant peptide without a primary signal transduction domain, had a structure comprising an extracellular recognition domain containing the TIGIT mutant peptide, a transmembrane domain, and an intracellular costimulatory signaling domain, but did not comprise a primary signaling domain. CAR61 exhibited no killing effect on three CD155-expressing malignant tumor cells, whereas CAR53, a CAR-T cell constructed with the TIGIT mutant peptide, showed killing effects on all three CD155-expressing malignant tumor cells. It can thus be demonstrated that the fusion protein in the form of CAR61 constructed with the mutant TIGIT peptide alone cannot exert the killing function.

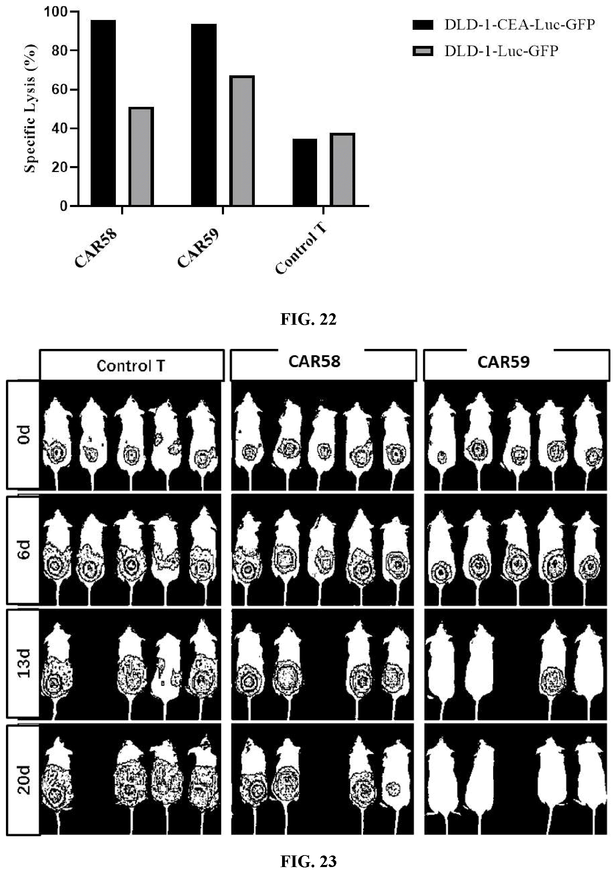

CEA-targeting CAR (CAR58) was selected, and CAR59 containing the fusion protein with the TIGIT mutant peptide was designed. DLD-1-CEA-Luc-GFP and DLD1-Luc-GFP were used as target cells, at the effector-to-target ratio of 1:1. Among them, DLD-1-CEA-Luc-GFP highly expressed CEA target molecule, while DLD1-Luc-GFP exhibited weak CEA expression. The *in vitro* killing ability of CAR58 (T cells expressing only the CEA-targeting CAR but not expressing the TIGIT-containing fusion peptide) and CAR59 (T cells co-expressing the CEA-targeting CAR and the fusion peptide shown in CAR61) was validated using the method in Example 9. This was used to evaluate the effectiveness of an engineered receptor containing the TIGIT mutant peptide in the antigen binding domain and lacking a primary signal transduction domain in the CAR-T cells. The results are shown in FIG. 22, illustrating the *in vitro* effectiveness data of the CAR-T cells expressing the engineered receptors containing the TIGIT mutant peptide in the antigen binding domain and lacking a primary signal transduction domain. In the drawing, the horizontal axis represents CAR58 and CAR59, and the vertical axis represents the specific lysis of two cells against DLD-1-CEA-Luc-GFP and DLD1-Luc-GFP. The results show that both CAR58 and CAR59 exhibited obvious killing against DLD-1-CEA-Luc-GFP, and CAR59 exhibited significantly higher killing against DLD-1-Luc-GFP than the CAR58 group. It can be seen that CAR-T co-expressing the CEA-targeting CAR and the fusion peptide shown in CAR61 exhibited a superior killing ability compared with CAR-T not expressing the fusion protein.

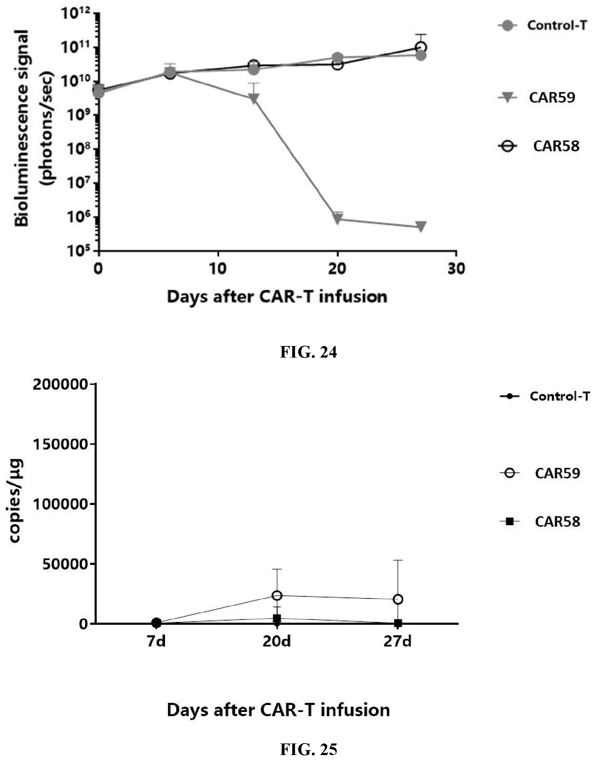

Female NCG mice aged 6-8 weeks were subcutaneously inoculated with DLD-1-CEA-Luc-GFP to establish tumors. On day 12 post-tumor engraftment, the CAR-T cells were infused via tail vein. *In vivo* imaging of mice was performed every 7 days after administration to detect the tumor cell retention in the mice. The imaging fluorescence values were statistically analyzed, and the significance was analyzed using t-test. The results are shown in FIG. 23 and FIG. 24. FIG. 23 shows the *in vivo* effectiveness of the CAR-T cells, which expressed the fusion protein comprising the TIGIT mutant peptide in the antigen binding domain, against colorectal cancer in the tumor-bearing mice with colorectal cancer indication. FIG. 24 shows the *in vivo* tumor fluorescence curves of the CAR-T cells, which expressed the fusion protein comprising the TIGIT mutant peptide in the antigen binding domain, against colorectal cancer in the tumor-bearing mice with colorectal cancer indication. The results show that the CAR59 CAR-T cells expressing the Mut29-28TM-28 fusion protein exhibited better *in vivo* effectiveness than the CAR58 CAR-T cells not expressing the fusion protein comprising the TIGIT mutant peptide in the antigen binding domain, indicating that the mut29-28TM-28 fusion protein, i.e., the engineered receptor with the TIGIT mutant peptide as the extracellular recognition domain, when combined with the CAR, can enhance the *in vivo* effectiveness of the CAR-T cells.

Terminal orbital blood sampling was performed on the experimental mice, and the copy number of CAR-T in blood was detected by fluorescence quantitative PCR. Results are shown in FIG. 25, presenting the copy number in the mice after tumor killing by the CAR-T cells, which expressed the fusion protein comprising the TIGIT mutant peptide in the antigen binding domain, in the tumor-bearing mice with colorectal cancer indication. The results show that the copy number of CAR-T in blood was significantly higher in the CAR59 than that in the CAR58 on days 7, 20, and 27 after the CAR-T administration, demonstrating that CAR13, co-expressing on the T cell membrane the CAR and the fusion protein comprising the TIGIT mutant peptide in the antigen binding domain, can significantly improve the *in vivo* persistence of CAR in models.

The above results prove that CAR59, in which CAR is combined with the fusion protein comprising the TIGIT mutant peptide in the antigen binding domain, can significantly improve the *in vivo* efficacy and enhance the *in vivo* effectiveness of the CAR-T cells. Moreover, the effectiveness and persistence derive from the co-expression of the fusion protein comprising the TIGIT mutant peptide in the antigen binding domain of the present application with the CAR.

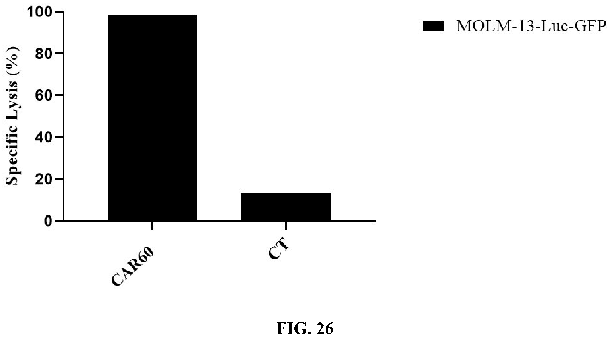

Furthermore, the inventors also validated, in various hematological tumors and solid tumors such as acute myeloid leukemia, pancreatic cancer and bladder cancer, the effectiveness of the fusion protein comprising the TIGIT mutant peptide in the antigen binding domain, when expressed in CAR-T cells targeting multiple indication targets against CD155-expressing malignant tumors. Results are shown in FIG. 26, validating the effective killing of CD123 CAR-T, expressing the fusion protein comprising the TIGIT mutant peptide in the antigen binding domain, against acute myeloid leukemia using the acute myeloid leukemia tumor cell line Molm-13.

Furthermore, through the same experiment as described in the present example, it can be seen that Mut26, Mut29, truncated Mut26, and truncated Mut29 can also be used to construct fusion proteins or engineered receptors of various structures to form engineered receptor combinations with CAR structures, which are expressed on the surface of immune cells such as T cells, NK cells, DC cells, and macrophages for the treatment of CD155-expressing solid tumors and acute lymphoblastic leukemia.

It can be seen that the fusion protein comprising the TIGIT mutant peptide in the antigen binding domain itself exerts no effector function during the killing process, ensuring its safety in application. However, when the fusion protein is co-expressed in immune cells with a CAR targeting CEA or CD123, it can significantly improve *in vivo* efficacy and improve the persistence of immune cells (such as CAR-T) in blood. Meanwhile, *in vitro* killing and cytokine secretion data demonstrate that co-expression of the fusion protein comprising the TIGIT mutant peptide in the antigen binding domain with CAR molecules targeting different target proteins on the surface of immune cells can exert obvious killing and effector functions, indicating that the fusion protein is applicable when combined with different targets.

The sequences corresponding to various elements used in the examples of the present application are set forth in the Sequence Listing below.

### Sequence Listing

| Sequence Name | Nucleic Acid Sequence/Amino Acid Sequence | SEQ ID NO: |
|---|---|---|
| WT | MMTGTIETTGNISAEKGGSIILQCHLSSTTAQVTQVNWEQQDQLL AICNADLGWHISPSFKDRVAPGPGLGLTLQSLTVNDTGEYFCIYHT YPDGTYTGRIFLEVLESSVAEHGARFQIP | 1 |
| Mut1 | MMTGTIETTGNISAEKGGSIILQCHLSSTTAQVTQVDWEQQDQLLA ISNADLGWHISPSFKDRVAPGPGLGLTLQSLTVNDTGEYFCIYHTYP DGTYTGRIFLEVLESSVAEHGARFQIP | 2 |
| Mut2 | MMTGTIETTGNISAEKGGSIILQCHLSSTTAQVTQVDWEQQDQLLA IYNADLGWHISPSFKDRVAPGPGLGLTLQSLTVNDTGEYFCIYHTYP DGTYTGRIFLEVLESSVAEHGARFQIP | 3 |
| Mut3 | MMTGTIETTGNISAEKGGSIILQCHLSSTTAQVTQVDWEQQDQLLA IYNADLGWHISPSFKDRVAPGPGLGLTLQPLTVNDTGEYFCIYHTY PDGTYTGRIFLEVLESSEAEHGARFQIP | 4 |
| Mut4 | MMTGTIETTGNISAEKGGSIIFQCHLSSTTAQVTQVDWGQQDQLL AIYNADLGWHISPSFKDRVAPGPGLGLTLQSLTVNDTGEYFCIYHT YPDGTYTGRIFLEVLESSVAEHGARFQIP | 5 |
| Mut5 | MMTGTIETTGNISAEKGGSIILQCHLSSTTAQVTQVDWEQQDQLLA IWNADLGWHISPSFKDRVAPGPGLGLTLQSLTVNDTGEYFCIYHTY PDGTYTGRIFLEVLESSVAEHGARFQIP | 6 |
| Mut6 | MMTGTIETTGNISAEKGGSIILQCHLSSTTAQVTQVNWEQQDQLLA IWNADLGWHISPSFKDRVAPGPGLGLTLQSLTVNDTGEYFCIYHTY PDGTYTGRIFLEVLESSVAEHGARFQIP | 7 |
| Mut7 | MMTGTIETTGNISAEKGGSIILQCHLSSTTAQVTQVDWEQQDQLLA ICNADLGWHISPSFKDRVAPGPGLGLTLQSLTVNDTGEYFCIYHTYP DGTYTGRIFLEVLESSVAEHGARFQIP | 8 |
| Mut8 | MMTGTIETTGNISAEKGGSTILQCHLSSTTAQVTQVDWKQQDQFL AIWNADLGWHISPSFKDRVAPGPGLGLTLQSLTVNDTGEYFCIYHT YPDGTYTGRIFLEVQESSVAEHGARFQIP | 9 |
| Mut9 | MMTGTIETTGNLSAEKGGSIILQCHLSSTTAQVTQVEWEQQDQLL AIWNADLGWHISPSFKDRVAPGPGLDLTLQSLTVNDTGEYFCIYHT YPDGTYTGRISLEVLESSVAEHGARFQIP | 10 |
| Mut10 | MMTGTIETTGNISAEKGGSTILQCHLSSTTAQVTQVDWEQQGQLL AIWNADLGWHISPSFKDRVAPGPGQGLTLQSLTVNDTGEYFCIYHT YPDGTYTGRIFLEVLESSVAEHGARFQIP | 11 |
| Mut11 | MMTGTIETTGNISAEKGGSIFLQCHLSSTTAQVSQVEWKQQDQLL ΛIWNΛDLGWHISPSFKDRVΛPGPGPGLTLQSLTVYDTGEYFCIYHT YPDGTYTGRTFLEVLESSAAEHGARFQIP | 12 |
| Mut12 | MMTGTIETSGNISAEKGGSIILQCHLSSTTAQVTQVDWVQQDQLL AIWNADLGWHISPSFRDRVAPGPGLGLTLQSLTVNDTGEYFCIYHT | 13 |
| | YPDGTYTGRTFLEVLESSVAEHGARFQIP | |
| Mut13 | MMTGTIETTGNISAEKGGSTILQCHLSSTTAQVTQVDWKQQDQLL ΛIWNΛDLGWHISPSFKDRVΛPGPGLGLTLQSLTVNDTGEYFCIYHT YPDGTYTGRIFLEVLESSVAEHGARFQIP | 14 |
| Mut14 | MMTGTIETTGNISAEKGGSIILQCHLSSTTAQVTQVDWEQQDQLLA IYNADLGWHISPSFKDRVAPGPGLGLTLQSLTVNDTGEYSCIYHTYP DGTYTGRIFLEVLESSVAEYGARFQIP | 15 |
| Mut15 | MWTGTIETTGNISAEKGGSIILQCHLSSTTAQVTQVDWEQQDQLL AIWNADLGWHFSPSFKDRVAPGPGLGLTLQSLTVNDTGEYFCIYHT YPDGTYTGRIFLEVLESSVAEHGARFQIP | 16 |
| Mut16 | MMTGTIETTGNNSVEKGGSIFLQCHLSSTTAQVTQVDWKQQDQL LAIWNADLGWHISPSFKDRVAPGPGQGLTLQSLTVNDTGEYFCIYH TYPDGTYTGRIFLEVLESSVAEHGARFQIP | 17 |
| Mut17 | MMTGTIETTGNISAEKGGSIILQCHLSSTTAQVTQVDWEQQDQLLA IWNADLGWHIPPSFKDRVAPGPGLGLTLQSLTVNDTGEYSCIYHTY PDGTYTGRIFLEVLESSVAEHGARFQIP | 18 |
| Mut18 | MTTGTIETTGNISAEKGGSIILQCHLSSTTAQVTQVDWKQQDQLLA IWNADLGWHISPSFKDRVAPGPGLGLTLQSLTVNDTGEYLCIYHTY PDGTYTGRIFLEVLESSVAEHGARFQIP | 19 |
| Mut19 | MMTGTIETTGNISAEKGGSTILQCHLSSTTAQVTQVDWEQQDQLL AIWNADLGWHISPSFKDRVAPGPGLGLTLQSLTVNDTGEYFCIYHT YPDGTYTGRIFLEVLESSVAEHGARFQIP | 20 |
| Mut20 | MMTGTIETTGNISAEKGGSIILQCHLSSTTAQVTQVDWEQQDQLLA IWNADLGWHISPSFKDRVAPGPGLGLTLQSLTENDTGEYFCIYHTY PDGTYTGRIFLEVLESSVAEHGARFQIP | 21 |
| Mut21 | MMTGTIETTGNISAEKGGSIILQCHLSSTTAQVTQVNWEQQDQLLA ISNADLGWHISPSFKDRVAPGPGLGLTLQSLTVNDTGEYFCIYHTYP DGTYTGRIFLEVLESSVAEHGARFQIP | 22 |
| Mut22 | MMTGTIETTGNISAEKGGSIILQCHLSSTTAQVTQVNWEQQDQLLA IRNADLGWHISPSFKDRVAPGPGLGLTLQSLTVNDTGEYFCIYHTYP DGTYTGRIFLEVLESSVAEHGARFQIP | 23 |
| Mut23 | MMTGTIETTGNISAEKGGSIILQCHLSSTTAQVTQVNWEQQDQLLA IGNADLGWHISPSFKDRVAPGPGLGLTLQSLTVNDTGEYFCIYHTY PDGTYTGRIFLEVLESSVAEHGARFQIP | 24 |
| Mut24 | MMTGTIETTGNISAEKGGSIILQCHLSSTTAQVTQVNWEQQDQLLA IINADLGWHISPSFKDRVAPGPGLGLTLQSLTVNDTGEYFCIYHTYP DGTYTGRIFLEVLESSVAEHGARFQIP | 25 |
| Mut6 truncation | VTQVNWEQQDQLLAIWNADLGWHISPSFKDRVAPGPGLGLTLQSL TVNDTGEYFCIYHTYP | 26 |
| PSCA(1) | DIQDIQLTQSPSSLSASVGDRVTITCSASSSVRFIHWYQQKPGKAPK RLIYDTSKLASGVPSRFSGSGSGTDFTLTISSLQPEDFATYYCQQWS SSPFTFGQGTKVEIKGSTSGSGKPGSGEGSTKGSEVQLVESGGGLV QPGGSLRLSCAASGFNIKDYYIHWVRQAPGKGLEWVAWIDPENG DTEFVPKFQGRATISADTSKNTAYLQMNSLRAEDTAVYYCKTGGF WGQGTLVTVSS | 27 |
| PSCA(9) | DIVMTQSPSSLSASVGDRVTVTCRASQGILTYLAWYQQKPGKAPN LLIYAASTLQSGVPSRFSGSGSGTDFTLTISSLQPEDFATYYCLQDY NFPRTFGQGTKLEIKRGSTSGSGKPGSGEGSTKGEVQLQQSGPEVK KPGASVKVSCKASGYTFTDYYMNWVRQMHGKGLEWMGVINPY NGGTDYNQKFKGRVTITADKSTSTAYMELSSLRSEDTAVYYCARS VYDYPFDYWGQGTLVTVSS | 28 |
| Antigen binding domain binding to CEA | DIQMTQSPSSLSASVGDRVTITCSTSSSVSYMHWYQQKPGKAPRL LIYSTSNLASGVPSRFSGSGSGTDFTFTISSLQPEDIATYYCHQWSS YPTFGQGTKVEIKGSTSGSGKPGSGEGSTKGQVQLQESGPGLVRPS QTLSLTCTVSGFTISSGYSWHWVRQPPGRGLEWIGYIQYSGITNYN PSLKSRVTMLVDTSKNQFSLRLSSVTAADTAVYYCAREDYDYHW YFDVWGQGSTVTVSSGAAA | 29 |
| Antigen binding domain binding to CD123 | ETTLTQSPGTLSLSPGERATLSCRASQSVSSNLAWYQQKPGQAPRL LIYGASTRATGIPARFSGSGSGTEFTLTISRLEPEDFAVYYCQQRSN WPPALTFGGGTKLELKRGSTSGSGKPGSGEGSTKGQVQLQQSGAE VKKPGESLRISCKGSGYSFTSYWIGWVRQMPGKGLEWMGIIYPGD SDTRYSPSFEGQVTISADRSISTAYLQWSSLKASDTAMYYCARIRF DKEQLFYYYYGMDVWGQGTTVTVSS | 30 |
| P2A | GSGATNFSLLKQAGDVEENPGP | 31 |
| 8h | TTTPAPRPPTPAPTIASQPLSLRPEACRPAAGGAVHTRGLDFACD | 32 |
| 7h | APPRASALPAPPTGSALPDPQTASALPDPPAASALP | 33 |
| G4h | ESKYGPPCPPCPAPEFLGGPSVFLFPPKPKDTLMISRTPEVTCVVVD VSQEDPEVQFNWYVDGVEVHNAKTKPREEQFNSTYRVVSVLTVL HQDWLNGKEYKCKVSNKGLPSSIEKTISKAKGQPREPQVYTLPPS QEEMTKNQVSLTCLVKGFYPSDIAVEWESNGQPENNYKTTPPVLD SDGSFFLYSRLTVDKSRWQEGNVFSCSVMHEALHNHYTQKSLSLS LGK | 34 |
| CD8TM | IYIWAPLAGTCGVLLLSLVIT | 35 |
| CD28TM | FWVLVVVGGVLACYSLLVTVAFIIFWV | 36 |
| CD28 intracellular signaling domain | RSKRSRGGHSDYMNMTPRRPGPTRKHYQPYAPPRDFAAYRS | 37 |
| 4-1BB intracellular signaling | LYCKRGRKKLLYIFKQPFMRPVQTTQEEDGCSCRFPEEEEGGCEL | 38 |
| domain | | |
| 2B4 intracellular signaling domain | WRRKRKEKQSETSPKEFLTIYEDVKDLKTRRNHEQEQTFPGGGSTI YSMIQSQSSAPTSQEPAYTLYSLIQPSRKSGSRKRNHSPSFNSTIYE VIGKSQPKAQNPARLSRKELENFDVYS | 39 |
| CD28-4-1BB intracellular signaling domain | RSKRSRGGHSDYMNMTPRRPGPTRKHYQPYAPPRDFAAYRSVKR GRKKLLYIFKQPFMRPVQTTQEEDGCSCRFPEEEEGGCEL | 40 |
| Z | RVKFSRSADAPAYKQGQNQLYNELNLGRREEYDVLDKRRGRDPE MGGKPRRKNPQEGLYNELQKDKMΛEΛYSEIGMKGERRRGKGHD GLYQGLSTATKDTYDALHMQALPPR | 41 |
| Z(1) | RVKFSRSADAPAYQQGQSQLYNELNLGRREEYDVLDKRRGRDPE MGGKPRRKNPQEGLYNELQKDKMAEAYSEIGMKGERRRGKGHD GLYQGLSTATKDTYDALHMQALPPR | 42 |
| Z(3) | RVKFSRSADAPAYQQGQNQLYNELNLGRREEYDVLDKRRGRDPE MGGKPRRKNPQEGLYNELQKDKMAEAYSEIGMKGERRRGKGHD GLYQGLSTATKDTYDALHMQALPPR | 43 |
| ICOSTM | FWLPIGCAAFVVVCILGCILI | 44 |
| CD134 intracellular signaling domain | ALYLLRRDQRLPPDAHKPPGGGSFRTPIQEEQADAHSTLAKI | 45 |
| ICOS intracellular signaling domain | CWLTKKKYSSSVHDPNGEYMFMRAVNTAKKSRLTDVTL | 46 |
| Mut25 | MMTGTIETTGNISAEKGGSIFLQCHLSSTTAQVSQVNWKQQDQLL AIWNADLGWHISPSFKDRVAPGPGPGLTLQSLTVYDTGEYFCIYH TYPDGTYTGRTFLEVLESSAAEHGARFQIP | 47 |
| Mut26 | MMTGTIETTGNISAEKGGSIILQCHLSSTTAQVTQVNWEQQDQLL AIWNADLGWHIPPSFKDRVAPGPGLGLTLQSLTVNDTGEYSCIYH TYPDGTYTGRIFLEVLESSVAEHGARFQIP | 48 |
| Mut27 | MMTGTIETSGNISAEKGGSIILQCHLSSTTAQVTQVNWVQQDQLL AIWNADLGWHISPSFRDRVAPGPGLGLTLQSLTVNDTGEYFCIYH TYPDGTYTGRTFLEVLESSVAEHGARFQIP | 49 |
| Mut28 | MMTGTIETTGNISAEKGGSTILQCHLSSTTAQVTQVNWKQQDQLL AIWNADLGWHISPSFKDRVAPGPGLGLTLQSLTVNDTGEYFCIYH TYPDGTYTGRIFLEVLESSVAEHGARFQIP | 50 |
| Mut29 | MMTGTIETTGNISAEKGGSIILQCHLSSTTAQVTQVNWEQQDQLL AIWNADLGWHISPSFKDRVAPGPGLDLTLQSLTVNDTGEYFCVYH TYPDGTYTGRIFLEVLESSVAEHGARFQIP | 51 |
| Mut26 truncation | VTQVNWEQQDQLLAIWNADLGWHIPPSFKDRVAPGPGLGLTLQS LTVNDTGEYSCIYHTYP | 52 |
| Mut29 truncation | VTQVNWEQQDQLLAIWNADLGWHISPSFKDRVAPGPGLDLTLQS LTVNDTGEYFCVYHTYP | 53 |

## Claims

1. A TIGIT extracellular region polypeptide, comprising a mutation at position 48 relative to a reference sequence, wherein the reference sequence is an amino acid sequence as set forth in SEQ ID NO:1, and wherein amino acid position numbering is based on the reference sequence.

2. The TIGIT extracellular region polypeptide according to claim 1, comprising at least amino acids corresponding to positions 33 to 93 of the reference sequence.

3. The TIGIT extracellular region polypeptide according to claim 1 or 2, wherein the mutation at the position 48 is any one selected from the group consisting of:
C48G, C48A, C48V, C48L, C48I, C48P, C48F, C48W, C48M, C48Y, C48S, C48T, C48N, C48Q, C48D, C48E, C48K, C48R, C48H, and deletion at position C48.

4. The TIGIT extracellular region polypeptide according to any one of claims 1 to 3, further comprising one or more amino acid positions selected from the group consisting of:
2M, 2W, 2T, 9T, 9S, 12I, 12N, 14A, 14V, 20I, 20T, 211, 21F, 22L, 22F, 34T, 34S, 37N, 37D, 37E, 39E, 39G, 39K, 39V, 42D, 42G, 44L, 44F, 61K, 61R, 70L, 70Q, 70P, 71G, 71D, 76S, 76P, 79V, 79E, 80N, 80Y, 86F, 86S, 86L, 1011, 101T, 102S, 102F, 106L, 106Q, 110V, 110Y, 110E, 110A, 113H, and 113Y.

5. The TIGIT extracellular region polypeptide according to claim 4, further comprising a combination of amino acid positions of any one of following 1) to 12):
1) 22F, 37D, 39G;
2) 37D;
3) 20T, 37D, 39K, 44F;
4) 22L, 37E, 71D, 102S;
5) 20T, 37D, 42G, 70Q;
6) 21F, 34S, 37E, 39K, 70P, 80Y, 101T, 110A;
7) 9S, 37D, 39V, 61R, 101T;
8) 20T, 37D, 39K;
9) 37D, 86S, 113Y;
10) 2W, 37D, 56F;
11) 12N, 14V, 21F, 37D, 39K, 70Q; or
12) 2T, 37D, 39K, 86L.

6. The TIGIT extracellular region polypeptide according to claim 1, comprising any amino acid sequence selected from the following, or a conservative substitution variant of any amino acid sequence selected from the following:
SEQ ID NOs:5-17, 19, and 22, and SEQ ID NOs:24-26.

7. The TIGIT extracellular region polypeptide according to any one of claims 1 to 6, further comprising mutations relative to the reference sequence at following positions:
1) position 57 and position 86; or
2) position 71 and position 88;
wherein the reference sequence is the amino acid sequence as set forth in SEQ ID NO:1, and wherein the amino acid position numbering is based on the reference sequence.

8. The TIGIT extracellular region polypeptide according to claim 7, comprising a following mutation combination relative to the reference sequence:
1) C48W, S57P, and F86S; or
2) C48W, G71D, and I88V.

9. The TIGIT extracellular region polypeptide according to claim 8, comprising a combination of amino acid positions of any one of 1) to 4):
1) 34T, 39E, 48W, 57P, 61K, 70L, 71G, 80N, 86S, and 88I;
2) 34T, 39E, 48W, 57S, 61K, 70L, 71D, 80N, 86F, and 88V;
3) 9T, 20I, 21I, 34T, 39E, 48W, 57P, 61K, 70L, 71G, 80N, 86S, 88I, 101I, and 110V; or
4) 9T, 20I, 21I, 34T, 39E, 48W, 57S, 61K, 70L, 71D, 80N, 86F, 88V, 1011, and 110V.

10. The TIGIT extracellular region polypeptide according to claim 7, comprising an amino acid sequence as set forth in any one of SEQ ID NOs:47-53 or a conservative substitution variant thereof.

11. A fusion protein comprising the TIGIT extracellular region polypeptide according to any one of claims 1 to 10.

12. The fusion protein according to claim 11, further comprising one or more polypeptides binding to a tumor antigen and/or an immune checkpoint protein, wherein optionally, the polypeptide binding to the tumor antigen and/or the immune checkpoint protein is an antibody, ligand or receptor of the tumor antigen and/or the immune checkpoint protein, and optionally, the antibody against the tumor antigen and/or the immune checkpoint protein is a single-chain antibody (scFv), Fab, F(ab')2, Fab', Fv, Fd, dAb or diabody.

13. An engineered receptor, comprising an antigen binding domain, wherein the antigen binding domain comprises the TIGIT extracellular region polypeptide according to any one of claims 1 to 10, or the fusion protein according to claim 11 or 12.

14. The engineered receptor according to claim 13, further comprising a signal transduction domain, wherein the signal transduction domain comprises a primary signal transduction domain and/or a costimulatory domain.

15. The engineered receptor according to claim 13 or 14, which is a chimeric antigen receptor (CAR), a T cell receptor (TCR), a T cell antigen coupler (TAC) or a fusion protein.

16. The engineered receptor according to claim 14 or 15, wherein the costimulatory domain comprises a signal transduction domain of one or more molecules selected from the group consisting of:
CD27, CD28, 4-1BB, OX40, CD30, CD40, CD2, LFA-1, LIGHT, NKG2C, B7-H3, PD-1, ICOS, CDS, ICAM-1, GITR, BAFFR, LIGHTR, SLAMF7, CD7, NKp80 (KLRF1), CD160, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAMl, SLAMF4, CD84, CD96, CEACAM1, CRTAM, CD229, CD160, PSGL1, CD100, CD69, SLAMF6, SLAMF1, SLAMF8, CD162, LTBR, LAT, GADS, SLP-76, PAG/Cbp, NKp44, NKp30, NKp46, NKG2D, and a ligand specifically binding to CD83.

17. The engineered receptor according to any one of claims 14 to 16, wherein the primary signal transduction domain comprises a signal transduction domain of one or more molecules selected from the group consisting of: CD3ζ, CD3γ, CD3δ, CD3ε, CD5, CD22, FcRy, FcRβ, FcεRIγ, FcεRIβ, FcyRIIa, CD79α, CD79β, CD66d, DAP10, and DAP12.

18. The engineered receptor according to claim 17, further comprising a transmembrane domain between the antigen binding domain and the signal transduction domain, wherein the transmembrane domain comprises a transmembrane domain of any one or more molecules selected from the group consisting of: ICOS, CD4, CD8α, CD28, CD3ζ, and TIGIT.

19. The engineered receptor according to claim 18, wherein the antigen binding domain and the transmembrane domain are linked via a hinge region, preferably the hinge region is a hinge region of TIGIT, CD7, IgG, IgD, CD8α or CD28, or a combination thereof.

20. The engineered receptor according to any one of claims 13 to 19, comprising or consisting of: the TIGIT extracellular region polypeptide, a transmembrane domain, and a costimulatory domain, wherein a hinge region is further comprised or not comprised between the TIGIT extracellular region polypeptide and the transmembrane domain, wherein
the costimulatory domain comprises or is a signal transduction domain of one or more molecules selected from the group consisting of:
CD27, CD28, 4-1BB, OX40, CD30, CD40, CD2, LFA-1, LIGHT, NKG2C, B7-H3, PD-1, ICOS, CDS, ICAM-1, GITR, BAFFR, LIGHTR, SLAMF7, CD7, NKp80 (KLRF1), CD160, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAMl, SLAMF4, CD84, CD96, CEACAM1, CRTAM, CD229, CD160, PSGL1, CD100, CD69, SLAMF6, SLAMF1, SLAMF8, CD162, LTBR, LAT, GADS, SLP-76, PAG/Cbp, NKp44, NKp30, NKp46, NKG2D, and a ligand specifically binding to CD83;
the transmembrane domain comprises or is a transmembrane domain of any one or more molecules selected from the group consisting of: ICOS, CD4, CD8α, CD28, CD3ζ, and TIGIT; and
the hinge region comprises or is a hinge region of TIGIT, CD7, IgG, IgD, CD8α or CD28, or a combination thereof.

21. The engineered receptor according to claim 20, from N-terminus to C-terminus, comprising or consisting of in sequence: the TIGIT extracellular region polypeptide, a CD28 transmembrane domain, and a CD28 signal transduction domain.

22. The engineered receptor according to any one of claims 13 to 19, from N-terminus to C-terminus, comprising or consisting of in sequence any one of following 1) to 10):
1) the TIGIT extracellular region polypeptide, a CD28 transmembrane domain, a CD28 signal transduction domain, and a CD3ζ signal transduction domain;
2) the TIGIT extracellular region polypeptide, a CD8 hinge region, a CD28 transmembrane domain, a CD28 signal transduction domain, and a CD3ζ signal transduction domain;
3) the TIGIT extracellular region polypeptide, a CD8 hinge region, a CD28 transmembrane domain, a 4-1BB signal transduction domain, and a CD3ζ signal transduction domain;
4) the TIGIT extracellular region polypeptide, a G4h hinge region, a CD28 transmembrane domain, a CD28 signal transduction domain, and a CD3ζ signal transduction domain;
5) the TIGIT extracellular region polypeptide, an ICOS transmembrane domain, an ICOS signal transduction domain, and a CD3ζ signal transduction domain;
6) the TIGIT extracellular region polypeptide, a CD8 transmembrane domain, a CD134 signal transduction domain, and a CD3ζ signal transduction domain;
7) the TIGIT extracellular region polypeptide, a CD28 transmembrane domain, a CD28 signal transduction domain, a 4-1BB signal transduction domain, and a CD3ζ signal transduction domain;
8) the TIGIT extracellular region polypeptide, a CD7 hinge region, a CD28 transmembrane domain, a CD28 signal transduction domain, and a CD3ζ signal transduction domain;
9) the TIGIT extracellular region polypeptide, a G4h hinge region, a CD28 transmembrane domain, a CD28 signal transduction domain, a 4-1BB signal transduction domain, and a CD3ζ signal transduction domain; and
10) the TIGIT extracellular region polypeptide, a CD8 hinge region, a CD8 transmembrane domain, a 4-1BB signal transduction domain, and a CD3ζ signal transduction domain.

23. An engineered nucleic acid molecule, encoding the TIGIT extracellular region polypeptide according to any one of claims 1 to 10, the fusion protein according to claim 11 or 12, or the engineered receptor according to any one of claims 13 to 22.

24. An engineered cell, comprising the TIGIT extracellular region polypeptide according to any one of claims 1 to 10, the fusion protein according to claim 11 or 12, the engineered receptor according to any one of claims 13 to 22, and/or the engineered nucleic acid molecule according to claim 23,

25. The engineered cell according to claim 24, comprising an engineered receptor binding to a target molecule CD155.

26. The engineered cell according to claim 24 or 25, comprising two or more engineered receptors binding to same target molecule or different target molecules.

27. The engineered cell according to claim 26, wherein one of the target molecules is CD155, and other target molecules are one, two or three selected from PSCA, CD123, and CEA,
wherein the engineered receptor binding to the target molecule CD155 is the engineered receptor according to any one of claims 13 to 22, and the engineered receptor binding to one, two or three target molecules selected from PSCA, CD123, and CEA is a CAR targeting PSCA, CD123 or CEA, respectively.

28. The engineered cell according to claim 27, wherein
an antigen binding domain of the engineered receptor binding to the target molecule CD155 comprises an amino acid sequence as set forth in any one of SEQ ID NOs:2-26 and 47-53 or a conservative substitution variant thereof;
the antigen binding domain of the CAR binding to the target molecule PSCA comprises an amino acid sequence as set forth in SEQ ID NO:27 or 28, or a conservative substitution variant thereof, or an amino acid sequence having at least 80%, 85%, 90%, 91%, 92%, 93%, 94%, 95%, 96%, 97%, 98% or 99% identity thereto;
the antigen binding domain of the CAR binding to the target molecule CEA comprises an amino acid sequence as set forth in SEQ ID NO:29, or a conservative substitution variant thereof, or an amino acid sequence having at least 80%, 85%, 90%, 91%, 92%, 93%, 94%, 95%, 96%, 97%, 98% or 99% identity thereto; and
the antigen binding domain of the CAR binding to the target molecule CD123 comprises an amino acid sequence as set forth in SEQ ID NO:30, or a conservative substitution variant thereof, or an amino acid sequence having at least 80%, 85%, 90%, 91%, 92%, 93%, 94%, 95%, 96%, 97%, 98% or 99% identity thereto.

29. The engineered cell according to any one of claims 24 to 28, which is derived from a T cell, NK cell, macrophage, DC cell, B cell, or a precursor cell thereof.

30. Use of the TIGIT extracellular region polypeptide according to any one of claims 1 to 10, the fusion protein according to claim 11 or 12, the engineered receptor according to any one of claims 13 to 22, the nucleic acid molecule according to claim 23, or the engineered cell according to any one of claims 24 to 29 in manufacture of a medicament for treating cancer.

31. The use according to claim 30, wherein the cancer is one or more selected from:
bladder cancer, blood cancer, bone cancer, bone marrow cancer, brain/nervous system cancer, breast cancer, colorectal cancer, esophageal cancer, gastrointestinal cancer, head cancer, kidney cancer, liver cancer, lung cancer, nasopharyngeal cancer, neck cancer, ovarian cancer, pancreatic cancer, prostate cancer, skin cancer, gastric cancer, testicular cancer, tongue cancer, and uterine cancer.

32. A method for prolonging an *in vivo* persistence period of CAR-T cells, comprising expressing on a membrane of the CAR-T cells the TIGIT extracellular region polypeptide according to any one of claims 1 to 10, the fusion protein according to claim 11 or 12, or the engineered receptor according to any one of claims 13 to 22.

33. A method for enhancing an *in vivo* expansion ability of CAR-T cells, comprising expressing on a membrane of the CAR-T cells the TIGIT extracellular region polypeptide according to any one of claims 1 to 10, the fusion protein according to claim 11 or 12, or the engineered receptor according to any one of claims 13 to 22.

34. A method for enhancing an *in vivo* killing ability of CAR-T against target cells, comprising expressing on a membrane of CAR-T cells the TIGIT extracellular region polypeptide according to any one of claims 1 to 10, the fusion protein according to claim 11 or 12, or the engineered receptor according to any one of claims 13 to 22.
